# EUROPEAN PATENT SPECIFICATION

(11) **EP 2 710 378 B1**
(45) Date of publication and mention of the grant of the patent: **15.03.2017**
(21) Application number: 12721318.9
(22) Date of filing: 18.05.2012
(51) Int. Cl.: G01N 33/569, G01N 33/564, G01N 33/50

(54) **METHOD TO PROGNOSE VIRAL INFECTION BY MEASURING T CELL RESPONSE OR AUTO-ANTIBODIES TO APOPTOTIC EPITOPES**
VERFAHREN ZUR PROGNOSE EINER VIRALEN INFEKTION DURCH MESSUNG DER T-ZELL-REAKTION ODER AUTOANTIKÖRPER GEGEN APOPTOTISCHE EPITOPE
MÉTHODE DE PRONOSTIC D'UNE INFECTION VIRALE PAR LA MESURE DE LA RÉPONSE DES LYMPHOCYTES T OU DES AUTO-ANTICORPS VIS-À-VIS D'ÉPITOPES APOPTOTIQUES

(30) Priority: 20.05.2011 IT RM20110248
(43) Date of publication of application: 26.03.2014
(73) Proprietor: Università degli Studi di Roma "La Sapienza", 00185 Roma (IT)
(72) Inventor: BARNABA, Vincenzo, I-00185 Rome (IT)
(74) Representative: Capasso, Olga
(86) International application number: PCT/EP2012/059275
(87) International publication number: WO 2012/159993

(56) References cited:
- PISANA MORONI RAWSON ET AL: "Cross-presentation of caspase-cleaved apoptotic self antigens in HIV infection", NATURE MEDICINE, vol. 13, no. 12, 18 November 2007 (2007-11-18), pages 1431-1439, XP055011139, ISSN: 1078-8956, DOI: 10.1038/nm1679 & Pisana Moroni Rawson ET AL: "Supplementary Figures 1-8, Table 1-3, data 1-2, and methods. TO: Cross-presentation of caspase-cleaved apoptotic self-antigens in HIV infection:", Nature Medicine, vol 13, 1 December 2007 (2007-12-01), XP55030041, Retrieved from the Internet: URL:http://www.nature.com/nm/journal/v13/n 12/extref/nm1679-S1.pdf [retrieved on 2012-06-15]
- MELONI F ET AL: "Dendritic cells loaded with apoptotic oligodendrocytes as a source of myelin T-cell epitopes in multiple sclerosis", CLINICAL IMMUNOLOGY, ACADEMIC PRESS, US, vol. 129, no. 2, 1 November 2008 (2008-11-01), pages 286-294, XP025546130, ISSN: 1521-6616, DOI: 10.1016/J.CLIM.2008.07.017 [retrieved on 2008-08-26]
- PROPATO A ET AL: "Apoptotic cells overexpress vinculin and induce vinculin-specific cytotoxic T-cell cross-priming", NATURE MEDICINE, NATURE PUBLISHING GROUP, NEW YORK, NY, US, vol. 7, no. 7, 1 July 2001 (2001-07-01), pages 807-813, XP002398533, ISSN: 1078-8956, DOI: 10.1038/89930
- CITRO ALESSANDRA ET AL: "From T cell apoptosis to chronic immune activation in inflammatory diseases", INTERNATIONAL ARCHIVES OF ALLERGY AND IMMUNOLOGY, KARGER AG, CH, vol. 164, no. 2, 1 August 2014 (2014-08-01), pages 140-146, XP008176438, ISSN: 1018-2438, DOI: 10.1159/000363385 [retrieved on 2014-07-02]
- S. CHEVALIEZ: "Virological tools to diagnose and monitor hepatitis C virus infection", CLINICAL MICROBIOLOGY AND INFECTION., vol. 17, no. 2, 1 February 2011 (2011-02-01), pages 116-121, XP055266593, United Kingdom, Switzerland ISSN: 1198-743X, DOI: 10.1111/j.1469-0691.2010.03418.x
- MAURICE R. HILLEMAN: "Comparative Biology and Pathogenesis of AIDS and Hepatitis B Viruses: Related but Different", AIDS RESEARCH AND HUMAN RETROVIRUSES., vol. 10, no. 11, 1 November 1994 (1994-11-01), pages 1409-1419, XP055266658, US ISSN: 0889-2229, DOI: 10.1089/aid.1994.10.1409
- A Franciscus: "Similarities and Differences between HIV and HCV", HCSP FACT SHEET VERSION 4.5 July 2015 HCSP, 1 July 2015 (2015-07-01), XP055266660, Retrieved from the Internet: URL:http://hcvadvocate.org/hepatitis/facts heets_pdf/Similarities_and_Differences_bet ween_HIV_and_HCV.pdf [retrieved on 2016-04-19]

### SEQUENCE MISTING

<110> Università Degli Studi di Roma "La Sapienza" BARNABA, Vincenzo
<120> METHOD TO PROGNOSE VIRAL INFECTION BY MEASURING T CELL RESPONSE OR AUTO-ANTIBODIES TO APOPTOTIC EPITOPES
<130> PCT 117415
<150> RM2011A000248
   <151> 2011-05-20
<160> 379
<170> PatentIn version 3.5
<210> 1
   <211> 9
   <212> PRT
   <213> Homo sapiens
<400> 1
   Ala Met Tyr Val Ala Ile Gln Ala Val
   1               5
<210> 2
   <211> 9
   <212> PRT
   <213> Homo sapiens
<400> 2
   Ala Leu Ala Pro Ser Thr Met Lys Ile
   1               5
<210> 3
   <211> 9
   <212> PRT
   <213> Homo sapiens
<400> 3
   Ala Leu Pro His Ala Ile Leu Arg Leu
   1 5
<210> 4
   <211> 9
   <212> PRT
   <213> Homo sapiens
<400> 4
   Phe Leu Gly Met Glu Ser Cys Gly Ile
   1 5
<210> 5
   <211> 9
   <212> PRT
   <213> Homo sapiens
<400> 5
   Arg Met Gln Lys Glu Ile Thr Ala Leu
   1 5
<210> 6
   <211> 10
   <212> PRT
   <213> Homo sapiens
<400> 6
   Thr Leu Lys Tyr Pro Ile Glu His Gly Ile
   1 5 10
<210> 7
   <211> 10
   <212> PRT
   <213> Homo sapiens
<400> 7
   Ala Met Tyr Val Ala Ile Gln Ala Val Leu
   1 5 10
<210> 8
   <211> 10
   <212> PRT
   <213> Homo sapiens
<400> 8
   Ser Leu Ser Thr Phe Gln Gln Met Trp Ile
   1 5 10
<210> 9
   <211> 9
   <212> PRT
   <213> Homo sapiens
<400> 9
   Gly Met Gly Gln Lys Asp Ser Tyr Val
   1 5
<210> 10
   <211> 10
   <212> PRT
   <213> Homo sapiens
<400> 10
   Glu Leu Pro Asp Gly Gln Val Ile Thr Ile
   1 5 10
<210> 11
   <211> 9
   <212> PRT
   <213> Homo sapiens
<400> 11
   Asp Leu Thr Asp Tyr Leu Met Lys Ile
   1 5
<210> 12
   <211> 9
   <212> PRT
   <213> Homo sapiens
<400> 12
   Arg Leu Asp Leu Ala Gly Arg Asp Leu
   1 5
<210> 13
   <211> 10
   <212> PRT
   <213> Homo sapiens
<400> 13
   Asp Leu Thr Asp Tyr Leu Met Lys Ile Leu
   1               5                   10
<210> 14
   <211> 9
   <212> PRT
   <213> Homo sapiens
<400> 14
   Ser Leu Ala Gly Gly Ile Ile Gly Val
   1               5
<210> 15
   <211> 9
   <212> PRT
   <213> Homo sapiens
<400> 15
   Glu Met Val Glu Leu Arg Ile Leu Leu
   1               5
<210> 16
   <211> 10
   <212> PRT
   <213> Homo sapiens
<400> 16
   Ala Leu Arg Thr Asp Tyr Asn Ala Ser Val
   1               5                   10
<210> 17
   <211> 9
   <212> PRT
   <213> Homo sapiens
<400> 17
   Ile Leu Lys Lys Ile Ile Pro Thr Leu
   1               5
<210> 18
   <211> 10
   <212> PRT
   <213> Homo sapiens
<400> 18
   Val Leu Ile Gly Gly Lys Pro Asp Arg Val
   1 5 10
<210> 19
   <211> 10
   <212> PRT
   <213> Homo sapiens
<400> 19
   Ile Leu Asp Leu Ile Ser Glu Ser Pro Ile
   1 5 10
<210> 20
   <211> 9
   <212> PRT
   <213> Homo sapiens
<400> 20
   Gln Leu Pro Leu Glu Ser Asp Ala Val
   1 5
<210> 21
   <211> 10
   <212> PRT
   <213> Homo sapiens
<400> 21
   Leu Leu Gln Ser Lys Asn Ala Gly Ala Val
   1 5 10
<210> 22
   <211> 10
   <212> PRT
   <213> Homo sapiens
<400> 22
   Glu Leu Arg Leu Leu Ile His Gln Ser Leu
   1 5 10
<210> 23
   <211> 9
   <212> PRT
   <213> Homo sapiens
<400> 23
   Pro Leu Glu Gly Ser Glu Asp Arg Ile
   1               5
<210> 24
   <211> 10
   <212> PRT
   <213> Homo sapiens
<400> 24
   Pro Leu Glu Ser Asp Ala Val Glu Cys Leu
   1               5                   10
<210> 25
   <211> 10
   <212> PRT
   <213> Homo sapiens
<400> 25
   Glu Leu Arg Glu Asn Thr Gln Thr Thr Ile
   1               5                   10
<210> 26
   <211> 10
   <212> PRT
   <213> Homo sapiens
<400> 26
   Pro Leu Glu Gly Ser Glu Asp Arg Ile Ile
   1               5                   10
<210> 27
   <211> 9
   <212> PRT
   <213> Homo sapiens
<400> 27
   Thr Ile Trp Ala Ala Asn Ala Gly Val
   1               5
<210> 28
   <211> 9
   <212> PRT
   <213> Homo sapiens
<400> 28
   Arg Leu Ala Val Tyr Ile Asp Lys Val
   1               5
<210> 29
   <211> 9
   <212> PRT
   <213> Homo sapiens
<400> 29
   Ser Leu Ser Ser Gln Leu Ser Asn Leu
   1               5
<210> 30
   <211> 9
   <212> PRT
   <213> Homo sapiens
<400> 30
   Gln Leu Leu Asp Val Lys Leu Ala Leu
   1               5
<210> 31
   <211> 10
   <212> PRT
   <213> Homo sapiens
<400> 31
   Gln Leu Ala Asp Glu Thr Leu Leu Lys Val
   1               5                   10
<210> 32
   <211> 9
   <212> PRT
   <213> Homo sapiens
<400> 32
   Lys Leu Arg Glu Tyr Glu Ala Ala Leu
   1 5
<210> 33
   <211> 9
   <212> PRT
   <213> Homo sapiens
<400> 33
   Ala Leu Asn Ser Lys Asp Ala Ala Leu
   1 5
<210> 34
   <211> 9
   <212> PRT
   <213> Homo sapiens
<400> 34
   Glu Leu Met Glu Ser Arg Met Arg Ile
   1 5
<210> 35
   <211> 9
   <212> PRT
   <213> Homo sapiens
<400> 35
   Gln Leu Asn Asp Tyr Glu Gln Leu Leu
   1 5
<210> 36
   <211> 10
   <212> PRT
   <213> Homo sapiens
<400> 36
   Lys Leu Ser Pro Ser Pro Ser Ser Arg Val
   1 5 10
<210> 37
   <211> 10
   <212> PRT
   <213> Homo sapiens
<400> 37
   Val Leu Lys Ala Gly Gln Thr Val Thr Ile
   1 5 10
<210> 38
   <211> 10
   <212> PRT
   <213> Homo sapiens
<400> 38
   Glu Leu Asn Asp Arg Leu Ala Val Tyr Ile
   1 5 10
<210> 39
   <211> 10
   <212> PRT
   <213> Homo sapiens
<400> 39
   Gln Leu Gln Val Thr Glu Arg Glu Glu Val
   1 5 10
<210> 40
   <211> 10
   <212> PRT
   <213> Homo sapiens
<400> 40
   Lys Leu Leu Glu Gly Glu Glu Glu Arg Leu
   1 5 10
<210> 41
   <211> 10
   <212> PRT
   <213> Homo sapiens
<400> 41
   Ile Leu Lys Asn Ser Gln Gly Glu Glu Val
   1 5 10
<210> 42
   <211> 9
   <212> PRT
   <213> Homo sapiens
<400> 42
   Ser Leu Glu Thr Glu Asn Ser Ala Leu
   1 5
<210> 43
   <211> 9
   <212> PRT
   <213> Homo sapiens
<400> 43
   Glu Leu Ala Asp Ala Arg Arg Ala Leu
   1 5
<210> 44
   <211> 9
   <212> PRT
   <213> Homo sapiens
<400> 44
   Ser Leu Glu Gly Asp Leu Glu Asp Leu
   1 5
<210> 45
   <211> 9
   <212> PRT
   <213> Homo sapiens
<400> 45
   Asp Leu Lys Asp Gln Ile Ala Gln Leu
   1 5
<210> 46
   <211> 9
   <212> PRT
   <213> Homo sapiens
<400> 46
   Ser Leu Ala Ala Ala Lys Lys Gln Leu
   1 5
<210> 47
   <211> 9
   <212> PRT
   <213> Homo sapiens
<400> 47
   Leu Leu Glu Gly Glu Glu Glu Arg Leu
   1 5
<210> 48
   <211> 10
   <212> PRT
   <213> Homo sapiens
<400> 48
   Pro Leu Ser Pro Thr Arg Leu Ser Arg Leu
   1 5 10
<210> 49
   <211> 10
   <212> PRT
   <213> Homo sapiens
<400> 49
   Gly Leu Lys Ala Leu Tyr Glu Thr Glu Leu
   1 5 10
<210> 50
   <211> 9
   <212> PRT
   <213> Homo sapiens
<400> 50
   Glu Leu Arg Glu Leu Asn Asp Arg Leu
   1 5
<210> 51
   <211> 9
   <212> PRT
   <213> Homo sapiens
<400> 51
   Asp Leu Asn Gly Ala Gln Ile Lys Leu
   1 5
<210> 52
   <211> 9
   <212> PRT
   <213> Homo sapiens
<400> 52
   Asp Leu Glu Asp Leu Lys Asp Gln Ile
   1 5
<210> 53
   <211> 9
   <212> PRT
   <213> Homo sapiens
<400> 53
   Asp Leu Glu Asn Arg Cys Gln Ser Leu
   1 5
<210> 54
   <211> 10
   <212> PRT
   <213> Homo sapiens
<400> 54
   Glu Met Arg Glu Gln His Asp Ala Gln Val
   1 5 10
<210> 55
   <211> 10
   <212> PRT
   <213> Homo sapiens
<400> 55
   Glu Leu Glu Gln Thr Tyr His Ala Lys Leu
   1 5 10
<210> 56
   <211> 10
   <212> PRT
   <213> Homo sapiens
<400> 56
   Asn Leu Gln Lys Glu Ser Arg Ala Cys Leu
   1 5 10
<210> 57
   <211> 10
   <212> PRT
   <213> Homo sapiens
<400> 57
   Asp Met Glu Ile Ser Ala Tyr Arg Lys Leu
   1 5 10
<210> 58
   <211> 9
   <212> PRT
   <213> Homo sapiens
<400> 58
   Tyr Leu Tyr Val Asp Lys Asn Phe Ile
   1 5
<210> 59
   <211> 9
   <212> PRT
   <213> Homo sapiens
<400> 59
   Gly Leu Ile Tyr Thr Tyr Ser Gly Leu
   1 5
<210> 60
   <211> 9
   <212> PRT
   <213> Homo sapiens
<400> 60
   Tyr Thr Tyr Ser Gly Leu Phe Cys Val
   1 5
<210> 61
   <211> 9
   <212> PRT
   <213> Homo sapiens
<400> 61
   Asn Leu Pro Ile Tyr Ser Glu Glu Ile
   1 5
<210> 62
   <211> 9
   <212> PRT
   <213> Homo sapiens
<400> 62
   Glu Met Pro Pro His Ile Tyr Ala Ile
   1 5
<210> 63
   <211> 9
   <212> PRT
   <213> Homo sapiens
<400> 63
   Lys Val Ile Gln Tyr Leu Ala Tyr Val
   1 5
<210> 64
   <211> 9
   <212> PRT
   <213> Homo sapiens
<400> 64
   Gln Leu Phe Asn His Thr Met Phe Ile
   1 5
<210> 65
   <211> 9
   <212> PRT
   <213> Homo sapiens
<400> 65
   Trp Leu Met Lys Asn Met Asp Pro Leu
   1 5
<210> 66
   <211> 9
   <212> PRT
   <213> Homo sapiens
<400> 66
   Gln Leu Ala Lys Leu Met Ala Thr Leu
   1 5
<210> 67
   <211> 10
   <212> PRT
   <213> Homo sapiens
<400> 67
   Tyr Thr Tyr Ser Gly Leu Phe Cys Val Val
   1               5                   10
<210> 68
   <211> 10
   <212> PRT
   <213> Homo sapiens
<400> 68
   Arg Met Phe Arg Trp Leu Val Leu Arg Ile
   1               5                   10
<210> 69
   <211> 10
   <212> PRT
   <213> Homo sapiens
<400> 69
   Gln Leu Phe Asn His Thr Met Phe Ile Leu
   1               5                   10
<210> 70
   <211> 9
   <212> PRT
   <213> Homo sapiens
<400> 70
   Tyr Leu Leu Glu Lys Ser Arg Ala Ile
   1               5
<210> 71
   <211> 9
   <212> PRT
   <213> Homo sapiens
<400> 71
   Arg Thr Phe His Ile Phe Tyr Tyr Leu
   1               5
<210> 72
   <211> 9
   <212> PRT
   <213> Homo sapiens
<400> 72
   Leu Leu Ser Gly Ala Gly Glu His Leu
   1               5
<210> 73
   <211> 9
   <212> PRT
   <213> Homo sapiens
<400> 73
   Phe Leu Ser Asn Gly His Val Thr Ile
   1               5
<210> 74
   <211> 9
   <212> PRT
   <213> Homo sapiens
<400> 74
   Gly Leu Leu Arg Val Ile Ser Gly Val
   1               5
<210> 75
   <211> 9
   <212> PRT
   <213> Homo sapiens
<400> 75
   Phe Ala Ile Glu Ala Leu Ala Lys Ala
   1               5
<210> 76
   <211> 9
   <212> PRT
   <213> Homo sapiens
<400> 76
   Ile Leu Asp Ile Ala Gly Phe Glu Ile
   1               5
<210> 77
   <211> 9
   <212> PRT
   <213> Homo sapiens
<400> 77
   Phe Met Asp Gly Lys Gln Ala Cys Val
   1 5
<210> 78
   <211> 9
   <212> PRT
   <213> Homo sapiens
<400> 78
   Val Leu Met Ile Lys Ala Leu Glu Leu
   1 5
<210> 79
   <211> 9
   <212> PRT
   <213> Homo sapiens
<400> 79
   Arg Leu Phe Thr Lys Val Lys Pro Leu
   1 5
<210> 80
   <211> 9
   <212> PRT
   <213> Homo sapiens
<400> 80
   Glu Leu Gln Ala Ala Leu Ala Arg Val
   1 5
<210> 81
   <211> 9
   <212> PRT
   <213> Homo sapiens
<400> 81
   Lys Leu Gln Val Glu Leu Asp Asn Val
   1 5
<210> 82
   <211> 9
   <212> PRT
   <213> Homo sapiens
<400> 82
   Ser Leu Ser Thr Lys Leu Lys Gln Val
   1 5
<210> 83
   <211> 9
   <212> PRT
   <213> Homo sapiens
<400> 83
   Lys Leu Lys Asp Val Leu Leu Gln Val
   1 5
<210> 84
   <211> 10
   <212> PRT
   <213> Homo sapiens
<400> 84
   Tyr Leu Leu Ser Gly Ala Gly Glu His Leu
   1 5 10
<210> 85
   <211> 10
   <212> PRT
   <213> Homo sapiens
<400> 85
   Thr Met Glu Ala Met Arg Ile Met Gly Ile
   1 5 10
<210> 86
   <211> 10
   <212> PRT
   <213> Homo sapiens
<400> 86
   Gly Leu Leu Arg Val Ile Ser Gly Val Leu
   1 5 10
<210> 87
   <211> 10
   <212> PRT
   <213> Homo sapiens
<400> 87
   Gln Leu Tyr Lys Glu Gln Leu Ala Lys Leu
   1 5 10
<210> 88
   <211> 10
   <212> PRT
   <213> Homo sapiens
<400> 88
   Phe Met Asp Gly Lys Gln Ala Cys Val Leu
   1 5 10
<210> 89
   <211> 10
   <212> PRT
   <213> Homo sapiens
<400> 89
   Lys Leu Arg Arg Gly Asp Leu Pro Phe Val
   1 5 10
<210> 90
   <211> 9
   <212> PRT
   <213> Homo sapiens
<400> 90
   Gln Leu Leu Gln Ala Asn Pro Ile Leu
   1 5
<210> 91
   <211> 9
   <212> PRT
   <213> Homo sapiens
<400> 91
   Met Met Ala Lys Glu Glu Glu Leu Val
   1 5
<210> 92
   <211> 9
   <212> PRT
   <213> Homo sapiens
<400> 92
   Gln Leu Met Ala Glu Lys Leu Gln Leu
   1 5
<210> 93
   <211> 9
   <212> PRT
   <213> Homo sapiens
<400> 93
   Lys Met Gln Gln Asn Ile Gln Glu Leu
   1 5
<210> 94
   <211> 9
   <212> PRT
   <213> Homo sapiens
<400> 94
   Glu Leu Ala Asp Lys Val Thr Lys Leu
   1 5
<210> 95
   <211> 9
   <212> PRT
   <213> Homo sapiens
<400> 95
   Lys Leu Thr Lys Asp Phe Ser Ala Leu
   1 5
<210> 96
   <211> 9
   <212> PRT
   <213> Homo sapiens
<400> 96
   Lys Met Glu Asp Ser Val Gly Cys Leu
   1 5
<210> 97
   <211> 9
   <212> PRT
   <213> Homo sapiens
<400> 97
   Lys Leu Gln Lys Asp Leu Glu Gly Leu
   1 5
<210> 98
   <211> 9
   <212> PRT
   <213> Homo sapiens
<400> 98
   Glu Met Lys Thr Gln Leu Glu Glu Leu
   1 5
<210> 99
   <211> 9
   <212> PRT
   <213> Homo sapiens
<400> 99
   Arg Leu Glu Ala Arg Ile Ala Gln Leu
   1 5
<210> 100
   <211> 9
   <212> PRT
   <213> Homo sapiens
<400> 100
   Lys Leu Gln Glu Met Glu Gly Thr Val
   1 5
<210> 101
   <211> 9
   <212> PRT
   <213> Homo sapiens
<400> 101
   Ala Leu Glu Ala Lys Ile Ala Gln Leu
   1 5
<210> 102
   <211> 9
   <212> PRT
   <213> Homo sapiens
<400> 102
   Ala Met Asn Arg Glu Val Ser Ser Leu
   1 5
<210> 103
   <211> 10
   <212> PRT
   <213> Homo sapiens
<400> 103
   Lys Met Asn Pro Pro Lys Phe Ser Lys Val
   1 5 10
<210> 104
   <211> 10
   <212> PRT
   <213> Homo sapiens
<400> 104
   Thr Leu Arg Asn Thr Asn Pro Asn Phe Val
   1 5 10
<210> 105
   <211> 10
   <212> PRT
   <213> Homo sapiens
<400> 105
   Val Leu Asp Gln Leu Arg Cys Asn Gly Val
   1 5 10
<210> 106
   <211> 10
   <212> PRT
   <213> Homo sapiens
<400> 106
   Asn Leu Tyr Arg Ile Gly Gln Ser Lys Val
   1 5 10
<210> 107
   <211> 10
   <212> PRT
   <213> Homo sapiens
<400> 107
   Val Leu Gln Arg Asn Cys Ala Ala Tyr Leu
   1 5 10
<210> 108
   <211> 10
   <212> PRT
   <213> Homo sapiens
<400> 108
   Arg Leu Phe Thr Lys Val Lys Pro Leu Leu
   1 5 10
<210> 109
   <211> 10
   <212> PRT
   <213> Homo sapiens
<400> 109
   Ala Met Ile Thr Asp Leu Glu Glu Arg Leu
   1 5 10
<210> 110
   <211> 10
   <212> PRT
   <213> Homo sapiens
<400> 110
   Glu Leu Ala Asn Glu Val Lys Val Leu Leu
   1 5 10
<210> 111
   <211> 9
   <212> PRT
   <213> Homo sapiens
<400> 111
   Tyr Ile Val Gly Ala Asn Ile Glu Thr
   1 5
<210> 112
   <211> 9
   <212> PRT
   <213> Homo sapiens
<400> 112
   Pro Leu Asn Asp Asn Ile Ala Thr Leu
   1 5
<210> 113
   <211> 9
   <212> PRT
   <213> Homo sapiens
<400> 113
   Gly Met Phe Arg Thr Val Gly Gln Leu
   1 5
<210> 114
   <211> 9
   <212> PRT
   <213> Homo sapiens
<400> 114
   Lys Leu Glu Glu Glu Gln Ile Ile Leu
   1 5
<210> 115
   <211> 9
   <212> PRT
   <213> Homo sapiens
<400> 115
   Ile Leu Glu Asp Gln Asn Cys Lys Leu
   1 5
<210> 116
   <211> 9
   <212> PRT
   <213> Homo sapiens
<400> 116
   Gln Leu Gln Asp Thr Gln Glu Leu Leu
   1 5
<210> 117
   <211> 9
   <212> PRT
   <213> Homo sapiens
<400> 117
   Gln Leu Glu Glu Leu Glu Asp Glu Leu
   1 5
<210> 118
   <211> 9
   <212> PRT
   <213> Homo sapiens
<400> 118
   Gln Met Lys Asp Cys Met Arg Glu Leu
   1 5
<210> 119
   <211> 9
   <212> PRT
   <213> Homo sapiens
<400> 119
   Ser Met Glu Ala Glu Met Ile Gln Leu
   1 5
<210> 120
   <211> 9
   <212> PRT
   <213> Homo sapiens
<400> 120
   Glu Met Ile Gln Leu Gln Glu Glu Leu
   1 5
<210> 121
   <211> 9
   <212> PRT
   <213> Homo sapiens
<400> 121
   Arg Leu Lys Lys Ala Asn Leu Gln Ile
   1 5
<210> 122
   <211> 10
   <212> PRT
   <213> Homo sapiens
<400> 122
   Asn Leu Lys Glu Arg Tyr Tyr Ser Gly Leu
   1 5 10
<210> 123
   <211> 10
   <212> PRT
   <213> Homo sapiens
<400> 123
   Met Met Gln Asp Arg Glu Asp Gln Ser Ile
   1 5 10
<210> 124
   <211> 10
   <212> PRT
   <213> Homo sapiens
<400> 124
   Trp Leu Val Leu Arg Ile Asn Lys Ala Leu
   1 5 10
<210> 125
   <211> 10
   <212> PRT
   <213> Homo sapiens
<400> 125
   Leu Leu His Gln Ser Ser Asp Lys Phe Val
   1 5 10
<210> 126
   <211> 10
   <212> PRT
   <213> Homo sapiens
<400> 126
   Lys Leu Arg Asn Trp Gln Trp Trp Arg Leu
   1 5 10
<210> 127
   <211> 10
   <212> PRT
   <213> Homo sapiens
<400> 127
   Glu Met Met Ala Lys Glu Glu Glu Leu Val
   1 5 10
<210> 128
   <211> 10
   <212> PRT
   <213> Homo sapiens
<400> 128
   Ala Leu Lys Thr Glu Leu Glu Asp Thr Leu
   1 5 10
<210> 129
   <211> 10
   <212> PRT
   <213> Homo sapiens
<400> 129
   Leu Leu Gln Glu Glu Asn Arg Gln Lys Leu
   1 5 10
<210> 130
   <211> 10
   <212> PRT
   <213> Homo sapiens
<400> 130
   Arg Leu Gln Gln Glu Leu Asp Asp Leu Leu
   1 5 10
<210> 131
   <211> 10
   <212> PRT
   <213> Homo sapiens
<400> 131
   Lys Leu Lys Ser Met Glu Ala Glu Met Ile
   1 5 10
<210> 132
   <211> 9
   <212> PRT
   <213> Homo sapiens
<400> 132
   Leu Leu Arg Val Ile Ser Gly Val Leu
   1 5
<210> 133
   <211> 9
   <212> PRT
   <213> Homo sapiens
<400> 133
   Asn Met Asp Pro Leu Asn Asp Asn Ile
   1 5
<210> 134
   <211> 9
   <212> PRT
   <213> Homo sapiens
<400> 134
   Glu Leu Asp Ser Asn Leu Tyr Arg Ile
   1 5
<210> 135
   <211> 9
   <212> PRT
   <213> Homo sapiens
<400> 135
   Glu Met Met Ala Lys Glu Glu Glu Leu
   1 5
<210> 136
   <211> 9
   <212> PRT
   <213> Homo sapiens
<400> 136
   Glu Met Glu Thr Leu Gln Ser Gln Leu
   1 5
<210> 137
   <211> 9
   <212> PRT
   <213> Homo sapiens
<400> 137
   Glu Leu Cys Ala Glu Ala Glu Glu Leu
   1 5
<210> 138
   <211> 9
   <212> PRT
   <213> Homo sapiens
<400> 138
   Lys Leu Ala Lys Glu Lys Lys Leu Leu
   1 5
<210> 139
   <211> 9
   <212> PRT
   <213> Homo sapiens
<400> 139
   Glu Leu Glu Lys Thr Arg Arg Lys Leu
   1 5
<210> 140
   <211> 9
   <212> PRT
   <213> Homo sapiens
<400> 140
   Lys Leu Glu Gly Asp Ser Thr Asp Leu
   1 5
<210> 141
   <211> 9
   <212> PRT
   <213> Homo sapiens
<400> 141
   Asp Leu Ser Asp Gln Ile Ala Glu Leu
   1 5
<210> 142
   <211> 9
   <212> PRT
   <213> Homo sapiens
<400> 142
   Glu Leu Gln Ala Gln Ile Ala Glu Leu
   1 5
<210> 143
   <211> 9
   <212> PRT
   <213> Homo sapiens
<400> 143
   Gln Leu Ala Lys Lys Glu Glu Glu Leu
   1 5
<210> 144
   <211> 9
   <212> PRT
   <213> Homo sapiens
<400> 144
   Glu Leu Glu Ser Gln Ile Ser Glu Leu
   1 5
<210> 145
   <211> 9
   <212> PRT
   <213> Homo sapiens
<400> 145
   Glu Leu Ala Asn Glu Val Lys Val Leu
   1 5
<210> 146
   <211> 9
   <212> PRT
   <213> Homo sapiens
<400> 146
   Glu Leu Asp Asn Val Thr Gly Leu Leu
   1 5
<210> 147
   <211> 9
   <212> PRT
   <213> Homo sapiens
<400> 147
   Asn Leu Glu Lys Gln Ile Ala Thr Leu
   1 5
<210> 148
   <211> 9
   <212> PRT
   <213> Homo sapiens
<400> 148
   Lys Leu Glu Lys Thr Lys Thr Arg Leu
   1 5
<210> 149
   <211> 9
   <212> PRT
   <213> Homo sapiens
<400> 149
   Arg Leu Gln Gln Glu Leu Asp Asp Leu
   1 5
<210> 150
   <211> 9
   <212> PRT
   <213> Homo sapiens
<400> 150
   Glu Leu Asp Asp Leu Leu Val Asp Leu
   1 5
<210> 151
   <211> 9
   <212> PRT
   <213> Homo sapiens
<400> 151
   Asp Leu Met Ser Ser Lys Asp Asp Val
   1 5
<210> 152
   <211> 10
   <212> PRT
   <213> Homo sapiens
<400> 152
   Glu Leu Thr Cys Leu Asn Glu Ala Ser Val
   1 5 10
<210> 153
   <211> 10
   <212> PRT
   <213> Homo sapiens
<400> 153
   Gln Leu Lys Asp Lys Ala Asp Phe Cys Ile
   1 5 10
<210> 154
   <211> 10
   <212> PRT
   <213> Homo sapiens
<400> 154
   Pro Leu Asn Asp Asn Ile Ala Thr Leu Leu
   1 5 10
<210> 155
   <211> 10
   <212> PRT
   <213> Homo sapiens
<400> 155
   Glu Leu Val Lys Val Arg Glu Lys Gln Leu
   1 5 10
<210> 156
   <211> 10
   <212> PRT
   <213> Homo sapiens
<400> 156
   Lys Leu Lys Lys Leu Glu Glu Glu Gln Ile
   1 5 10
<210> 157
   <211> 10
   <212> PRT
   <213> Homo sapiens
<400> 157
   Lys Leu Lys Asn Lys His Glu Ala Met Ile
   1 5 10
<210> 158
   <211> 10
   <212> PRT
   <213> Homo sapiens
<400> 158
   Gly Leu Ser Gln Arg His Glu Glu Lys Val
   1 5 10
<210> 159
   <211> 10
   <212> PRT
   <213> Homo sapiens
<400> 159
   Glu Leu Gln Ala Thr Glu Asp Ala Lys Leu
   1 5 10
<210> 160
   <211> 10
   <212> PRT
   <213> Homo sapiens
<400> 160
   Ala Leu Ala Leu Glu Glu Lys Arg Arg Leu
   1 5 10
<210> 161
   <211> 9
   <212> PRT
   <213> Homo sapiens
<400> 161
   Glu Leu Val Glu Asn Gly Lys Lys Val
   1 5
<210> 162
   <211> 9
   <212> PRT
   <213> Homo sapiens
<400> 162
   Asp Leu Gln Pro Cys Ile Asp Leu Ile
   1 5
<210> 163
   <211> 9
   <212> PRT
   <213> Homo sapiens
<400> 163
   Glu Leu Trp Lys Asp Val Asp Arg Ile
   1 5
<210> 164
   <211> 9
   <212> PRT
   <213> Homo sapiens
<400> 164
   Asp Leu Lys Ile Thr Asp Val Ile Ile
   1 5
<210> 165
   <211> 9
   <212> PRT
   <213> Homo sapiens
<400> 165
   Arg Leu Thr Ala Lys Lys Gln Glu Leu
   1 5
<210> 166
   <211> 9
   <212> PRT
   <213> Homo sapiens
<400> 166
   Glu Leu Glu Glu Ile Cys His Asp Leu
   1 5
<210> 167
   <211> 9
   <212> PRT
   <213> Homo sapiens
<400> 167
   Asp Leu Gly Glu Glu Leu Glu Ala Leu
   1 5
<210> 168
   <211> 9
   <212> PRT
   <213> Homo sapiens
<400> 168
   Glu Leu Glu Ala Leu Lys Thr Glu Leu
   1 5
<210> 169
   <211> 9
   <212> PRT
   <213> Homo sapiens
<400> 169
   Asn Leu Glu Lys Ala Lys Gln Thr Leu
   1 5
<210> 170
   <211> 9
   <212> PRT
   <213> Homo sapiens
<400> 170
   Thr Leu Glu Asn Glu Arg Gly Glu Leu
   1 5
<210> 171
   <211> 9
   <212> PRT
   <213> Homo sapiens
<400> 171
   Gln Leu Leu Ala Glu Glu Lys Thr Ile
   1               5
<210> 172
   <211> 9
   <212> PRT
   <213> Homo sapiens
<400> 172
   Glu Leu Glu Lys Ser Lys Arg Ala Leu
   1               5
<210> 173
   <211> 9
   <212> PRT
   <213> Homo sapiens
<400> 173
   Lys Leu Glu Met Asp Leu Lys Asp Leu
   1               5
<210> 174
   <211> 9
   <212> PRT
   <213> Homo sapiens
<400> 174
   Asp Leu Lys Asp Leu Glu Ala His Ile
   1               5
<210> 175
   <211> 9
   <212> PRT
   <213> Homo sapiens
<400> 175
   Gln Leu Glu Arg Gln Asn Lys Glu Leu
   1               5
<210> 176
   <211> 9
   <212> PRT
   <213> Homo sapiens
<400> 176
   Arg Leu Lys Gln Leu Lys Arg Gln Leu
   1 5
<210> 177
   <211> 10
   <212> PRT
   <213> Homo sapiens
<400> 177
   Asn Leu Pro Ile Tyr Ser Glu Glu Ile Val
   1 5 10
<210> 178
   <211> 10
   <212> PRT
   <213> Homo sapiens
<400> 178
   Asp Leu Asn Ser Phe Glu Gln Leu Cys Ile
   1 5 10
<210> 179
   <211> 10
   <212> PRT
   <213> Homo sapiens
<400> 179
   Gly Leu Asp Leu Gln Pro Cys Ile Asp Leu
   1 5 10
<210> 180
   <211> 10
   <212> PRT
   <213> Homo sapiens
<400> 180
   Glu Leu Trp Lys Asp Val Asp Arg Ile Ile
   1 5 10
<210> 181
   <211> 10
   <212> PRT
   <213> Homo sapiens
<400> 181
   His Leu Glu Glu Glu Arg Asp Leu Lys Ile
   1               5                   10
<210> 182
   <211> 10
   <212> PRT
   <213> Homo sapiens
<400> 182
   Asn Met Ala Leu Lys Lys Ile Arg Glu Leu
   1               5                   10
<210> 183
   <211> 10
   <212> PRT
   <213> Homo sapiens
<400> 183
   Glu Leu Arg Ser Lys Arg Glu Gln Glu Val
   1               5                   10
<210> 184
   <211> 10
   <212> PRT
   <213> Homo sapiens
<400> 184
   Glu Met Arg Gln Lys His Ser Gln Ala Val
   1               5                   10
<210> 185
   <211> 10
   <212> PRT
   <213> Homo sapiens
<400> 185
   Asp Met Lys Lys Lys Met Glu Asp Ser Val
   1               5                   10
<210> 186
   <211> 10
   <212> PRT
   <213> Homo sapiens
<400> 186
   Ala Met Lys Ala Gln Phe Glu Arg Asp Leu
   1               5                   10
<210> 187
   <211> 10
   <212> PRT
   <213> Homo sapiens
<400> 187
   Ser Met Ala Val Ala Ala Arg Lys Lys Leu
   1               5                   10
<210> 188
   <211> 10
   <212> PRT
   <213> Homo sapiens
<400> 188
   Val Leu Lys Glu Gly Ser Glu Tyr Arg Val
   1               5                   10
<210> 189
   <211> 10
   <212> PRT
   <213> Homo sapiens
<400> 189
   His Leu Ser Trp Glu Trp Asn Leu Ser Ile
   1               5                   10
<210> 190
   <211> 9
   <212> PRT
   <213> Homo sapiens
<400> 190
   Glu Met Asp Lys Asp Asp Glu Ser Leu
   1               5
<210> 191
   <211> 9
   <212> PRT
   <213> Homo sapiens
<400> 191
   Thr Leu Leu Gly Asp Gly Pro Val Val
   1 5
<210> 192
   <211> 9
   <212> PRT
   <213> Homo sapiens
<400> 192
   Ala Leu Lys Lys Glu Thr Ile Val Leu
   1 5
<210> 193
   <211> 9
   <212> PRT
   <213> Homo sapiens
<400> 193
   Ser Leu Ile Lys Tyr Lys Lys Thr Leu
   1 5
<210> 194
   <211> 9
   <212> PRT
   <213> Homo sapiens
<400> 194
   Asp Leu Thr Gly Asp Leu Glu Ala Leu
   1 5
<210> 195
   <211> 10
   <212> PRT
   <213> Homo sapiens
<400> 195
   Glu Met Asp Lys Asp Asp Glu Ser Leu Ile
   1 5 10
<210> 196
   <211> 10
   <212> PRT
   <213> Homo sapiens
<400> 196
   Ser Leu Ile Lys Tyr Lys Lys Thr Leu Leu
   1               5                   10
<210> 197
   <211> 10
   <212> PRT
   <213> Homo sapiens
<400> 197
   Asp Leu Glu Ala Leu Lys Lys Glu Thr Ile
   1               5                   10
<210> 198
   <211> 9
   <212> PRT
   <213> Homo sapiens
<400> 198
   Asn Leu Ala Glu Asp Ile Met Arg Leu
   1               5
<210> 199
   <211> 10
   <212> PRT
   <213> Homo sapiens
<400> 199
   Ser Leu Tyr Ala Ser Ser Pro Gly Gly Val
   1               5                   10
<210> 200
   <211> 9
   <212> PRT
   <213> Homo sapiens
<400> 200
   Arg Leu Arg Ser Ser Val Pro Gly Val
   1               5
<210> 201
   <211> 9
   <212> PRT
   <213> Homo sapiens
<400> 201
   Ile Leu Leu Ala Glu Leu Glu Gln Leu
   1 5
<210> 202
   <211> 9
   <212> PRT
   <213> Homo sapiens
<400> 202
   Ser Leu Gln Glu Glu Ile Ala Phe Leu
   1 5
<210> 203
   <211> 9
   <212> PRT
   <213> Homo sapiens
<400> 203
   Ala Leu Lys Gly Thr Asn Glu Ser Leu
   1 5
<210> 204
   <211> 10
   <212> PRT
   <213> Homo sapiens
<400> 204>
<210> 205
   <211> 10
   <212> PRT
   <213> Homo sapiens
<400> 205
   Ser Leu Pro Leu Pro Asn Phe Ser Ser Leu
   1 5 10
<210> 206
   <211> 9
   <212> PRT
   <213> Homo sapiens
<400> 206
   Leu Gln Asp Ser Val Asp Phe Ser Leu
   1 5
<210> 207
   <211> 9
   <212> PRT
   <213> Homo sapiens
<400> 207
   Ser Leu Thr Cys Glu Val Asp Ala Leu
   1 5
<210> 208
   <211> 9
   <212> PRT
   <213> Homo sapiens
<400> 208
   His Leu Arg Glu Tyr Gln Asp Leu Leu
   1 5
<210> 209
   <211> 9
   <212> PRT
   <213> Homo sapiens
<400> 209
   Asp Leu Leu Asn Val Lys Met Ala Leu
   1 5
<210> 210
   <211> 9
   <212> PRT
   <213> Homo sapiens
<400> 210
   Ser Leu Asn Leu Arg Glu Thr Asn Leu
   1 5
<210> 211
   <211> 10
   <212> PRT
   <213> Homo sapiens
<400> 211
   Phe Leu Glu Gln Gln Asn Lys Ile Leu Leu
   1               5                   10
<210> 212
   <211> 10
   <212> PRT
   <213> Homo sapiens
<400> 212
   Phe Leu Lys Lys Leu His Glu Glu Glu Ile
   1               5                   10
<210> 213
   <211> 10
   <212> PRT
   <213> Homo sapiens
<400> 213
   Lys Leu His Glu Glu Glu Ile Gln Glu Leu
   1               5                   10
<210> 214
   <211> 10
   <212> PRT
   <213> Homo sapiens
<400> 214
   Lys Leu Leu Glu Gly Glu Glu Ser Arg Ile
   1               5                   10
<210> 215
   <211> 9
   <212> PRT
   <213> Homo sapiens
<400> 215
   Phe Leu Glu Gln Gln Asn Lys Ile Leu
   1               5
<210> 216
   <211> 9
   <212> PRT
   <213> Homo sapiens
<400> 216
   Glu Leu Arg Arg Gln Val Asp Gln Leu
   1 5
<210> 217
   <211> 9
   <212> PRT
   <213> Homo sapiens
<400> 217
   Thr Leu Gln Ser Phe Arg Gln Asp Val
   1 5
<210> 218
   <211> 9
   <212> PRT
   <213> Homo sapiens
<400> 218
   Asp Leu Glu Arg Lys Val Glu Ser Leu
   1 5
<210> 219
   <211> 10
   <212> PRT
   <213> Homo sapiens
<400> 219
   Glu Leu Asn Asp Arg Phe Ala Asn Tyr Ile
   1 5 10
<210> 220
   <211> 10
   <212> PRT
   <213> Homo sapiens
<400> 220
   Asn Met Lys Glu Glu Met Ala Arg His Leu
   1 5 10
<210> 221
   <211> 10
   <212> PRT
   <213> Homo sapiens
<400> 221
   Leu Leu Asn Val Lys Met Ala Leu Asp Ile
   1 5 10
<210> 222
   <211> 10
   <212> PRT
   <213> Homo sapiens
<400> 222
   Asn Leu Arg Glu Thr Asn Leu Asp Ser Leu
   1 5 10
<210> 223
   <211> 9
   <212> PRT
   <213> Homo sapiens
<400> 223
   Asp Leu Thr Ala Ala Leu Arg Asp Val
   1 5
<210> 224
   <211> 9
   <212> PRT
   <213> Homo sapiens
<400> 224
   Leu Leu Glu Gly Glu Glu Ser Arg Ile
   1 5
<210> 225
   <211> 10
   <212> PRT
   <213> Homo sapiens
<400> 225
   Ala Leu Arg Pro Ser Thr Ser Arg Ser Leu
   1 5 10
<210> 226
   <211> 10
   <212> PRT
   <213> Homo sapiens
<400> 226
   Asp Leu Tyr Glu Glu Glu Met Arg Glu Leu
   1 5 10
<210> 227
   <211> 10
   <212> PRT
   <213> Homo sapiens
<400> 227
   Pro Leu Pro Asn Phe Ser Ser Leu Asn Leu
   1 5 10
<210> 228
   <211> 9
   <212> PRT
   <213> Homo sapiens
<400> 228
   Glu Met Arg Glu Leu Arg Arg Gln Val
   1 5
<210> 229
   <211> 9
   <212> PRT
   <213> Homo sapiens
<400> 229
   Gln Leu Thr Asn Asp Lys Ala Arg Val
   1 5
<210> 230
   <211> 10
   <212> PRT
   <213> Homo sapiens
<400> 230
   Gln Leu Lys Gly Gln Gly Lys Ser Arg Leu
   1 5 10
<210> 231
   <211> 9
   <212> PRT
   <213> Homo sapiens
<400> 231
   Arg Leu Asn Lys Val Ile Ser Glu Leu
   1 5
<210> 232
   <211> 9
   <212> PRT
   <213> Homo sapiens
<400> 232
   Phe Met Glu Cys Asn Leu Asn Glu Leu
   1 5
<210> 233
   <211> 10
   <212> PRT
   <213> Homo sapiens
<400> 233
   Asn Leu Asn Glu Leu Val Lys His Gly Leu
   1 5 10
<210> 234
   <211> 10
   <212> PRT
   <213> Homo sapiens
<400> 234
   His Met Ser Glu Phe Met Glu Cys Asn Leu
   1 5 10
<210> 235
   <211> 10
   <212> PRT
   <213> Homo sapiens
<400> 235
   Ile Leu His Val Asp Asn His Ile Gly Ile
   1 5 10
<210> 236
   <211> 9
   <212> PRT
   <213> Homo sapiens
<400> 236
   Gly Leu Lys Ser Lys Thr His Ala Val
   1               5
<210> 237
   <211> 9
   <212> PRT
   <213> Homo sapiens
<400> 237
   Ser Leu Ile Gly Ser Lys Thr Gln Ile
   1               5
<210> 238
   <211> 10
   <212> PRT
   <213> Homo sapiens
<400> 238
   Phe Met Glu Cys Asn Leu Asn Glu Leu Val
   1               5                   10
<210> 239
   <211> 9
   <212> PRT
   <213> Homo sapiens
<400> 239
   Ala Leu Lys Arg Ala Gln Ser Glu Leu
   1               5
<210> 240
   <211> 9
   <212> PRT
   <213> Homo sapiens
<400> 240
   Gly Leu Thr Ala Asp Ala Arg Leu Leu
   1               5
<210> 241
   <211> 10
   <212> PRT
   <213> Homo sapiens
<400> 241
   Pro Leu Pro Val Ser Arg Leu Val Ser Leu
   1 5 10
<210> 242
   <211> 9
   <212> PRT
   <213> Homo sapiens
<400> 242
   Asp Leu Thr Thr Lys Asn Val Ser Ile
   1 5
<210> 243
   <211> 10
   <212> PRT
   <213> Homo sapiens
<400> 243
   Glu Leu Asn Gly Lys Asn Ile Glu Asp Val
   1 5 10
<210> 244
   <211> 9
   <212> PRT
   <213> Homo sapiens
<400> 244
   Glu Leu Ala Ala His Gln Lys Lys Ile
   1 5
<210> 245
   <211> 10
   <212> PRT
   <213> Homo sapiens
<400> 245
   Glu Leu Val Lys His Gly Leu Arg Ala Leu
   1 5 10
<210> 246
   <211> 10
   <212> PRT
   <213> Homo sapiens
<400> 246
   Gly Leu Lys Ser Lys Thr His Ala Val Leu
   1 5 10
<210> 247
   <211> 9
   <212> PRT
   <213> Homo sapiens
<400> 247
   Gly Leu Arg Ala Leu Arg Glu Thr Leu
   1 5
<210> 248
   <211> 10
   <212> PRT
   <213> Homo sapiens
<400> 248
   Glu Leu Ala Ala His Gln Lys Lys Ile Leu
   1 5 10
<210> 249
   <211> 9
   <212> PRT
   <213> Homo sapiens
<400> 249
   Phe Val Phe Asp Arg Pro Leu Pro Val
   1 5
<210> 250
   <211> 9
   <212> PRT
   <213> Homo sapiens
<400> 250
   Tyr Val Ala Ser Tyr Leu Leu Ala Ala
   1 5
<210> 251
   <211> 9
   <212> PRT
   <213> Homo sapiens
<400> 251
   Ile Leu Asp Ser Val Gly Ile Glu Ala
   1               5
<210> 252
   <211> 10
   <212> PRT
   <213> Homo sapiens
<400> 252
   Tyr Val Ala Ser Tyr Leu Leu Ala Ala Leu
   1               5                   10
<210> 253
   <211> 10
   <212> PRT
   <213> Homo sapiens
<400> 253
   Tyr Leu Leu Pro Arg Arg Gly Pro Arg Leu
   1               5                   10
<210> 254
   <211> 9
   <212> PRT
   <213> Homo sapiens
<400> 254
   Asp Leu Met Gly Tyr Ile Pro Leu Val
   1               5
<210> 255
   <211> 9
   <212> PRT
   <213> Homo sapiens
<400> 255
   Phe Leu Leu Ala Leu Leu Ser Cys Leu
   1               5
<210> 256
   <211> 10
   <212> PRT
   <213> Homo sapiens
<400> 256
   Phe Leu Leu Ala Leu Leu Ser Cys Leu Thr
   1               5                   10
<210> 257
   <211> 10
   <212> PRT
   <213> Homo sapiens
<400> 257
   Ala Leu Leu Ser Cys Leu Thr Thr Pro Ala
   1               5                   10
<210> 258
   <211> 9
   <212> PRT
   <213> Homo sapiens
<400> 258
   Leu Leu Ser Cys Leu Thr Thr Pro Ala
   1               5
<210> 259
   <211> 9
   <212> PRT
   <213> Homo sapiens
<400> 259
   Met Met Met Asn Trp Ser Pro Thr Thr
   1               5
<210> 260
   <211> 10
   <212> PRT
   <213> Homo sapiens
<400> 260
   Met Met Met Asn Trp Ser Pro Thr Thr Ala
   1               5                   10
<210> 261
   <211> 10
   <212> PRT
   <213> Homo sapiens
<400> 261
   Tyr Ser Met Ala Gly Asn Trp Ala Lys Val
   1 5 10
<210> 262
   <211> 9
   <212> PRT
   <213> Homo sapiens
<400> 262
   Val Leu Ile Val Met Leu Leu Phe Ala
   1 5
<210> 263
   <211> 10
   <212> PRT
   <213> Homo sapiens
<400> 263
   Leu Ile Val Met Leu Leu Phe Ala Gly Trp
   1 5 10
<210> 264
   <211> 9
   <212> PRT
   <213> Homo sapiens
<400> 264
   Arg Leu Trp His Tyr Pro Cys Thr Val
   1 5
<210> 265
   <211> 10
   <212> PRT
   <213> Homo sapiens
<400> 265
   Leu Leu Leu Ser Thr Thr Glu Trp Gln Val
   1 5 10
<210> 266
   <211> 9
   <212> PRT
   <213> Homo sapiens
<400> 266
   Leu Leu Ser Thr Thr Glu Trp Gln Val
   1               5
<210> 267
   <211> 9
   <212> PRT
   <213> Homo sapiens
<400> 267
   Tyr Leu Tyr Gly Ile Gly Ser Ala Val
   1               5
<210> 268
   <211> 10
   <212> PRT
   <213> Homo sapiens
<400> 268
   Tyr Leu Tyr Gly Ile Gly Ser Ala Val Val
   1               5                   10
<210> 269
   <211> 10
   <212> PRT
   <213> Homo sapiens
<400> 269
   Tyr Val Leu Leu Leu Phe Leu Leu Leu Ala
   1               5                   10
<210> 270
   <211> 9
   <212> PRT
   <213> Homo sapiens
<400> 270
   Val Leu Leu Leu Phe Leu Leu Leu Ala
   1               5
<210> 271
   <211> 9
   <212> PRT
   <213> Homo sapiens
<400> 271
   Phe Leu Leu Leu Ala Asp Ala Arg Val
   1               5
<210> 272
   <211> 9
   <212> PRT
   <213> Homo sapiens
<400> 272
   Trp Met Met Leu Leu Ile Ala Gln Ala
   1               5
<210> 273
   <211> 9
   <212> PRT
   <213> Homo sapiens
<400> 273
   Met Leu Leu Ile Ala Gln Ala Glu Ala
   1               5
<210> 274
   <211> 9
   <212> PRT
   <213> Homo sapiens
<400> 274
   Phe Leu Ala Arg Leu Ile Trp Trp Leu
   1               5
<210> 275
   <211> 10
   <212> PRT
   <213> Homo sapiens
<400> 275
   Leu Leu Met Cys Ala Val His Pro Glu Leu
   1 5 10
<210> 276
   <211> 10
   <212> PRT
   <213> Homo sapiens
<400> 276
   Lys Leu Leu Ile Ala Ile Leu Gly Pro Leu
   1 5 10
<210> 277
   <211> 9
   <212> PRT
   <213> Homo sapiens
<400> 277
   Leu Leu Ile Ala Ile Leu Gly Pro Leu
   1 5
<210> 278
   <211> 9
   <212> PRT
   <213> Homo sapiens
<400> 278
   Val Leu Gln Ala Gly Ile Thr Arg Val
   1 5
<210> 279
   <211> 9
   <212> PRT
   <213> Homo sapiens
<400> 279
   Gly Leu Ile His Ala Cys Met Leu Val
   1 5
<210> 280
   <211> 9
   <212> PRT
   <213> Homo sapiens
<400> 280
   Lys Leu Gly Ala Leu Thr Gly Tyr Ile
   1               5
<210> 281
   <211> 9
   <212> PRT
   <213> Homo sapiens
<400> 281
   Tyr Ile Tyr Asn His Leu Thr Pro Leu
   1               5
<210> 282
   <211> 10
   <212> PRT
   <213> Homo sapiens
<400> 282
   Gly Leu Leu Gly Cys Ile Ile Thr Ser Leu
   1               5                   10
<210> 283
   <211> 9
   <212> PRT
   <213> Homo sapiens
<400> 283
   Leu Leu Gly Cys Ile Ile Thr Ser Leu
   1               5
<210> 284
   <211> 9
   <212> PRT
   <213> Homo sapiens
<400> 284
   Phe Leu Ala Thr Cys Val Asn Gly Val
   1               5
<210> 285
   <211> 10
   <212> PRT
   <213> Homo sapiens
<400> 285
   Lys Leu Ser Gly Leu Gly Ile Asn Ala Val
   1 5 10
<210> 286
   <211> 9
   <212> PRT
   <213> Homo sapiens
<400> 286
   Tyr Leu Asn Thr Pro Gly Leu Pro Val
   1 5
<210> 287
   <211> 9
   <212> PRT
   <213> Homo sapiens
<400> 287
   Tyr Leu Val Ala Tyr Gln Ala Thr Val
   1 5
<210> 288
   <211> 9
   <212> PRT
   <213> Homo sapiens
<400> 288
   Gln Met Trp Lys Cys Leu Ile Arg Leu
   1 5
<210> 289
   <211> 10
   <212> PRT
   <213> Homo sapiens
<400> 289
   Val Leu Val Gly Gly Val Leu Ala Ala Leu
   1 5 10
<210> 290
   <211> 9
   <212> PRT
   <213> Homo sapiens
<400> 290
   His Met Trp Asn Phe Ile Ser Gly Ile
   1 5
<210> 291
   <211> 9
   <212> PRT
   <213> Homo sapiens
<400> 291
   Phe Ile Ser Gly Ile Gln Tyr Leu Ala
   1 5
<210> 292
   <211> 9
   <212> PRT
   <213> Homo sapiens
<400> 292
   Ser Leu Met Ala Phe Thr Ala Ser Ile
   1 5
<210> 293
   <211> 10
   <212> PRT
   <213> Homo sapiens
<400> 293
   Leu Leu Phe Asn Ile Leu Gly Gly Trp Val
   1 5 10
<210> 294
   <211> 9
   <212> PRT
   <213> Homo sapiens
<400> 294
   Ile Leu Ala Gly Tyr Gly Ala Gly Val
   1 5
<210> 295
   <211> 9
   <212> PRT
   <213> Homo sapiens
<400> 295
   Lys Leu Leu Pro Gln Leu Pro Gly Val
   1 5
<210> 296
   <211> 10
   <212> PRT
   <213> Homo sapiens
<400> 296
   Arg Leu Ile Val Phe Pro Asp Leu Gly Val
   1 5 10
<210> 297
   <211> 9
   <212> PRT
   <213> Homo sapiens
<400> 297
   Ala Leu Tyr Asp Val Val Ser Thr Leu
   1 5
<210> 298
   <211> 9
   <212> PRT
   <213> Homo sapiens
<400> 298
   Lys Leu Gln Asp Cys Thr Met Leu Val
   1 5
<210> 299
   <211> 10
   <212> PRT
   <213> Homo sapiens
<400> 299
   Met Leu Val Asn Gly Asp Asp Leu Val Val
   1 5 10
<210> 300
   <211> 9
   <212> PRT
   <213> Homo sapiens
<400> 300
   Trp Leu Gly Asn Ile Ile Met Tyr Ala
   1 5
<210> 301
   <211> 10
   <212> PRT
   <213> Homo sapiens
<400> 301
   Met Ile Leu Met Thr His Phe Phe Ser Ile
   1 5 10
<210> 302
   <211> 9
   <212> PRT
   <213> Homo sapiens
<400> 302
   Ile Leu Met Thr His Phe Phe Ser Ile
   1 5
<210> 303
   <211> 10
   <212> PRT
   <213> Homo sapiens
<400> 303
   Ile Leu Met Thr His Phe Phe Ser Ile Leu
   1 5 10
<210> 304
   <211> 9
   <212> PRT
   <213> Homo sapiens
<400> 304
   Ala Leu Asp Cys Gln Ile Tyr Gly Ala
   1 5
<210> 305
   <211> 10
   <212> PRT
   <213> Homo sapiens
<400> 305
   Arg Leu His Gly Leu Ser Ala Phe Ser Leu
   1 5 10
<210> 306
   <211> 9
   <212> PRT
   <213> Homo sapiens
<400> 306
   Phe Met Leu Cys Leu Leu Leu Leu Ser
   1 5
<210> 307
   <211> 10
   <212> PRT
   <213> Homo sapiens
<400> 307
   Phe Met Leu Cys Leu Leu Leu Leu Ser Val
   1 5 10
<210> 308
   <211> 9
   <212> PRT
   <213> Homo sapiens
<400> 308
   Met Leu Cys Leu Leu Leu Leu Ser Val
   1 5
<210> 309
   <211> 10
   <212> PRT
   <213> Homo sapiens
<400> 309
   Leu Leu Leu Leu Ser Val Gly Val Gly Ile
   1 5 10
<210> 310
   <211> 10
   <212> PRT
   <213> Homo sapiens
<400> 310
   Leu Leu Ser Val Gly Val Gly Ile Tyr Leu
   1               5                   10
<210> 311
   <211> 9
   <212> PRT
   <213> Homo sapiens
<400> 311
   Phe Leu Leu Ala Leu Leu Ser Cys Ile
   1               5
<210> 312
   <211> 10
   <212> PRT
   <213> Homo sapiens
<400> 312
   Leu Leu Ala Leu Leu Ser Cys Ile Ser Val
   1               5                   10
<210> 313
   <211> 10
   <212> PRT
   <213> Homo sapiens
<400> 313
   Ala Leu Leu Ser Cys Ile Ser Val Pro Val
   1               5                   10
<210> 314
   <211> 9
   <212> PRT
   <213> Homo sapiens
<400> 314
   Leu Leu Ser Cys Ile Ser Val Pro Val
   1               5
<210> 315
   <211> 10
   <212> PRT
   <213> Homo sapiens
<400> 315
   Ala Leu Met Ile Ala Ala Gln Val Val Val
   1               5                   10
<210> 316
   <211> 9
   <212> PRT
   <213> Homo sapiens
<400> 316
   Met Leu Leu Ala Tyr Leu Val Arg Ile
   1               5
<210> 317
   <211> 10
   <212> PRT
   <213> Homo sapiens
<400> 317
   Val Met Phe Gly Leu Ala Tyr Phe Ser Met
   1               5                   10
<210> 318
   <211> 10
   <212> PRT
   <213> Homo sapiens
<400> 318
   Phe Ser Met Gln Gly Ala Trp Ala Lys Val
   1               5                   10
<210> 319
   <211> 9
   <212> PRT
   <213> Homo sapiens
<400> 319
   His Leu Phe Thr Ser Met Phe Ser Leu
   1               5
<210> 320
   <211> 9
   <212> PRT
   <213> Homo sapiens
<400> 320
   Phe Leu Ala Ala Leu Phe Tyr Thr Ser
   1               5
<210> 321
   <211> 9
   <212> PRT
   <213> Homo sapiens
<400> 321
   Tyr Leu Tyr Gly Leu Ser Pro Ala Ile
   1               5
<210> 322
   <211> 10
   <212> PRT
   <213> Homo sapiens
<400> 322
   Gly Leu Leu Tyr Phe Ile Leu Phe Phe Val
   1               5                   10
<210> 323
   <211> 9
   <212> PRT
   <213> Homo sapiens
<400> 323
   Leu Leu Tyr Phe Ile Leu Phe Phe Val
   1               5
<210> 324
   <211> 10
   <212> PRT
   <213> Homo sapiens
<400> 324
   Leu Leu Tyr Phe Ile Leu Phe Phe Val Ala
   1               5                   10
<210> 325
   <211> 10
   <212> PRT
   <213> Homo sapiens
<400> 325
   Tyr Thr Leu Leu Gly Cys Trp Ser Phe Val
   1 5 10
<210> 326
   <211> 9
   <212> PRT
   <213> Homo sapiens
<400> 326
   Thr Leu Leu Gly Cys Trp Ser Phe Val
   1 5
<210> 327
   <211> 10
   <212> PRT
   <213> Homo sapiens
<400> 327
   Thr Leu Leu Gly Cys Trp Ser Phe Val Leu
   1 5 10
<210> 328
   <211> 9
   <212> PRT
   <213> Homo sapiens
<400> 328
   Leu Leu Met Ala Leu Pro His Gln Ala
   1 5
<210> 329
   <211> 8
   <212> PRT
   <213> Homo sapiens
<400> 329
   Leu Leu Ile Ala Ile Thr Ala Phe
   1 5
<210> 330
   <211> 10
   <212> PRT
   <213> Homo sapiens
<400> 330
   Ile Leu Leu Ser Arg Cys Leu Trp Trp Thr
   1 5 10
<210> 331
   <211> 10
   <212> PRT
   <213> Homo sapiens
<400> 331
   Cys Leu Trp Trp Thr Cys Tyr Met Leu Val
   1 5 10
<210> 332
   <211> 10
   <212> PRT
   <213> Homo sapiens
<400> 332
   Tyr Met Leu Val Leu Ala Glu Ala Leu Ile
   1 5 10
<210> 333
   <211> 9
   <212> PRT
   <213> Homo sapiens
<400> 333
   Leu Ile Gln Asp Trp Ile Pro Pro Leu
   1 5
<210> 334
   <211> 9
   <212> PRT
   <213> Homo sapiens
<400> 334
   Ala Met Thr Met Phe Tyr Pro Gly Val
   1 5
<210> 335
   <211> 9
   <212> PRT
   <213> Homo sapiens
<400> 335
   Ala Leu Leu Arg Met Cys Ala Leu Val
   1               5
<210> 336
   <211> 10
   <212> PRT
   <213> Homo sapiens
<400> 336
   Arg Leu Leu Ala Pro Ile Thr Ala Tyr Ala
   1               5                   10
<210> 337
   <211> 9
   <212> PRT
   <213> Homo sapiens
<400> 337
   Leu Leu Ala Pro Ile Thr Ala Tyr Ala
   1               5
<210> 338
   <211> 9
   <212> PRT
   <213> Homo sapiens
<400> 338
   Phe Leu Gly Thr Ser Ile Ser Gly Val
   1               5
<210> 339
   <211> 10
   <212> PRT
   <213> Homo sapiens
<400> 339
   Thr Ser Ile Ser Gly Val Leu Trp Thr Val
   1               5                   10
<210> 340
   <211> 9
   <212> PRT
   <213> Homo sapiens
<400> 340
   Ser Ile Ser Gly Val Leu Trp Thr Val
   1 5
<210> 341
   <211> 9
   <212> PRT
   <213> Homo sapiens
<400> 341
   Val Leu Trp Thr Val Phe His Gly Ala
   1 5
<210> 342
   <211> 9
   <212> PRT
   <213> Homo sapiens
<400> 342
   Tyr Leu Val Thr Arg Asn Ala Asp Val
   1 5
<210> 343
   <211> 9
   <212> PRT
   <213> Homo sapiens
<400> 343
   Val Met Trp Lys Cys Leu Ile Arg Leu
   1 5
<210> 344
   <211> 10
   <212> PRT
   <213> Homo sapiens
<400> 344
   Val Leu Ala Gly Gly Val Leu Ala Ala Val
   1 5 10
<210> 345
   <211> 9
   <212> PRT
   <213> Homo sapiens
<400> 345
   Ser Met Met Ser Phe Ser Ala Ala Leu
   1               5
<210> 346
   <211> 9
   <212> PRT
   <213> Homo sapiens
<400> 346
   Val Leu Ala Gly Tyr Gly Ala Gly Ile
   1               5
<210> 347
   <211> 10
   <212> PRT
   <213> Homo sapiens
<400> 347
   Lys Ile Met Ser Gly Glu Lys Pro Ser Val
   1               5                   10
<210> 348
   <211> 9
   <212> PRT
   <213> Homo sapiens
<400> 348
   Lys Leu Phe Pro Arg Leu Pro Gly Ile
   1               5
<210> 349
   <211> 10
   <212> PRT
   <213> Homo sapiens
<400> 349
   Phe Met Arg Asp Glu Val Ser Phe Ser Val
   1               5                   10
<210> 350
   <211> 10
   <212> PRT
   <213> Homo sapiens
<400> 350
   Val Leu Met Val Asp Ser Phe Asp Pro Val
   1 5 10
<210> 351
   <211> 9
   <212> PRT
   <213> Homo sapiens
<400> 351
   Leu Met Val Asp Ser Phe Asp Pro Val
   1 5
<210> 352
   <211> 9
   <212> PRT
   <213> Homo sapiens
<400> 352
   Leu Leu Asp Ser His Tyr Glu Ser Val
   1 5
<210> 353
   <211> 10
   <212> PRT
   <213> Homo sapiens
<400> 353
   Arg Leu Ile Val Tyr Pro Asp Leu Gly Val
   1 5 10
<210> 354
   <211> 10
   <212> PRT
   <213> Homo sapiens
<400> 354
   Ser Met Gly Asn Thr Leu Thr Cys Tyr Val
   1 5 10
<210> 355
   <211> 10
   <212> PRT
   <213> Homo sapiens
<400> 355
   Met Leu Val Cys Gly Asp Asp Leu Val Val
   1 5 10
<210> 356
   <211> 10
   <212> PRT
   <213> Homo sapiens
<400> 356
   Met Val Leu Met Thr His Phe Phe Ser Val
   1 5 10
<210> 357
   <211> 9
   <212> PRT
   <213> Homo sapiens
<400> 357
   Val Leu Met Thr His Phe Phe Ser Val
   1 5
<210> 358
   <211> 10
   <212> PRT
   <213> Homo sapiens
<400> 358
   Val Leu Met Thr His Phe Phe Ser Val Leu
   1 5 10
<210> 359
   <211> 10
   <212> PRT
   <213> Homo sapiens
<400> 359
   Arg Leu His Gly Leu Glu Ala Phe Ser Leu
   1 5 10
<210> 360
   <211> 10
   <212> PRT
   <213> Homo sapiens
<400> 360
   Arg Leu Leu Asp Leu Ser Ser Trp Phe Thr
   1               5                   10
<210> 361
   <211> 10
   <212> PRT
   <213> Homo sapiens
<400> 361
   Arg Leu Leu Leu Leu Gly Leu Leu Leu Leu
   1               5                   10
<210> 362
   <211> 9
   <212> PRT
   <213> Homo sapiens
<400> 362
   Leu Leu Leu Leu Gly Leu Leu Leu Leu
   1               5
<210> 363
   <211> 10
   <212> PRT
   <213> Homo sapiens
<400> 363
   Leu Leu Leu Gly Leu Leu Leu Leu Cys Val
   1               5                   10
<210> 364
   <211> 9
   <212> PRT
   <213> Homo sapiens
<400> 364
   Leu Leu Gly Leu Leu Leu Leu Cys Val
   1               5
<210> 365
   <211> 9
   <212> PRT
   <213> Homo sapiens
<400> 365
   Gly Leu Leu Leu Leu Cys Val Gly Val
   1 5
<210> 366
   <211> 466
   <212> PRT
   <213> Homo sapiens
<400> 366
   Met Ser Thr Arg Ser Val Ser Ser Ser Ser Tyr Arg Arg Met Phe Gly
   1               5                   10                  15
   Gly Pro Gly Thr Ala Ser Arg Pro Ser Ser Ser Arg Ser Tyr Val Thr
               20                  25                  30
   Thr Ser Thr Arg Thr Tyr Ser Leu Gly Ser Ala Leu Arg Pro Ser Thr
           35                  40                  45
   Ser Arg Ser Leu Tyr Ala Ser Ser Pro Gly Gly Val Tyr Ala Thr Arg
       50                  55                  60
   Ser Ser Ala Val Arg Leu Arg Ser Ser Val Pro Gly Val Arg Leu Leu
   65                  70                  75                  80
   Gln Asp Ser Val Asp Phe Ser Leu Ala Asp Ala Ile Asn Thr Glu Phe
                   85                  90                  95
   Lys Asn Thr Arg Thr Asn Glu Lys Val Glu Leu Gln Glu Leu Asn Asp
               100                 105                 110
   Arg Phe Ala Asn Tyr Ile Asp Lys Val Arg Phe Leu Glu Gln Gln Asn
           115                 120                 125
   Lys Ile Leu Leu Ala Glu Leu Glu Gln Leu Lys Gly Gln Gly Lys Ser
       130                 135                 140
   Arg Leu Gly Asp Leu Tyr Glu Glu Glu Met Arg Glu Leu Arg Arg Gln
   145                 150                 155                 160
   Val Asp Gln Leu Thr Asn Asp Lys Ala Arg Val Glu Val Glu Arg Asp
                   165                 170                 175
   Asn Leu Ala Glu Asp Ile Met Arg Leu Arg Glu Lys Leu Gln Glu Glu
               180                 185                 190
   Met Leu Gln Arg Glu Glu Ala Glu Asn Thr Leu Gln Ser Phe Arg Gln
           195                 200                 205
   Asp Val Asp Asn Ala Ser Leu Ala Arg Leu Asp Leu Glu Arg Lys Val
       210                 215                 220
   Glu Ser Leu Gln Glu Glu Ile Ala Phe Leu Lys Lys Leu His Glu Glu
   225                 230                 235                 240
   Glu Ile Gln Glu Leu Gln Ala Gln Ile Gln Glu Gln His Val Gln Ile
                   245                 250                 255
   Asp Val Asp Val Ser Lys Pro Asp Leu Thr Ala Ala Leu Arg Asp Val
               260                 265                 270
   Arg Gln Gln Tyr Glu Ser Val Ala Ala Lys Asn Leu Gln Glu Ala Glu
           275                 280                 285
   Glu Trp Tyr Lys Ser Lys Phe Ala Asp Leu Ser Glu Ala Ala Asn Arg
       290                 295                 300
   Asn Asn Asp Ala Leu Arg Gln Ala Lys Gln Glu Ser Thr Glu Tyr Arg
   305                 310                 315                 320
   Arg Gln Val Gln Ser Leu Thr Cys Glu Val Asp Ala Leu Lys Gly Thr
                   325                 330                 335
   Asn Glu Ser Leu Glu Arg Gln Met Arg Glu Met Glu Glu Asn Phe Ala
               340                 345                 350
   Val Glu Ala Ala Asn Tyr Gln Asp Thr Ile Gly Arg Leu Gln Asp Glu
           355                 360                 365
   Ile Gln Asn Met Lys Glu Glu Met Ala Arg His Leu Arg Glu Tyr Gln
       370                 375                 380
   Asp Leu Leu Asn Val Lys Met Ala Leu Asp Ile Glu Ile Ala Thr Tyr
   385                 390                 395                 400
   Arg Lys Leu Leu Glu Gly Glu Glu Ser Arg Ile Ser Leu Pro Leu Pro
                   405                 410                 415
   Asn Phe Ser Ser Leu Asn Leu Arg Glu Thr Asn Leu Asp Ser Leu Pro
               420                 425                 430
   Leu Val Asp Thr His Ser Lys Arg Thr Leu Leu Ile Lys Thr Val Glu
           435                 440                 445
   Thr Arg Asp Gly Gln Val Ile Asn Glu Thr Ser Gln His His Asp Asp
       450                 455                 460
   Leu Glu
   465
<210> 367
   <211> 1960
   <212> PRT
   <213> Homo sapiens
<400> 367
<210> 368
   <211> 586
   <212> PRT
   <213> Homo sapiens
<400> 368
<210> 369
   <211> 201
   <212> PRT
   <213> Homo sapiens
<400> 369
   Met Thr Glu Lys Ala Pro Glu Pro His Val Glu Glu Asp Asp Asp Asp
   1 5 10 15
   Glu Leu Asp Ser Lys Leu Asn Tyr Lys Pro Pro Pro Gln Lys Ser Leu
   20 25 30
   Lys Glu Leu Gln Glu Met Asp Lys Asp Asp Glu Ser Leu Ile Lys Tyr
   35 40 45
   Lys Lys Thr Leu Leu Gly Asp Gly Pro Val Val Thr Asp Pro Lys Ala
   50 55 60
   Pro Asn Val Val Val Thr Arg Leu Thr Leu Val Cys Glu Ser Ala Pro
   65                  70                  75                  80
   Gly Pro Ile Thr Met Asp Leu Thr Gly Asp Leu Glu Ala Leu Lys Lys
                   85                  90                  95
   Glu Thr Ile Val Leu Lys Glu Gly Ser Glu Tyr Arg Val Lys Ile His
               100                 105                 110
   Phe Lys Val Asn Arg Asp Ile Val Ser Gly Leu Lys Tyr Val Gln His
           115                 120                 125
   Thr Tyr Arg Thr Gly Val Lys Val Asp Lys Ala Thr Phe Met Val Gly
       130                 135                 140
   Ser Tyr Gly Pro Arg Pro Glu Glu Tyr Glu Phe Leu Thr Pro Val Glu
   145                 150                 155                 160
   Glu Ala Pro Lys Gly Met Leu Ala Arg Gly Thr Tyr His Asn Lys Ser
                   165                 170                 175
   Phe Phe Thr Asp Asp Asp Lys Gln Asp His Leu Ser Trp Glu Trp Asn
               180                 185                 190
   Leu Ser Ile Lys Lys Glu Trp Thr Glu
           195                 200
<210> 370
   <211> 463
   <212> PRT
   <213> Homo sapiens
<400> 370
   Met Glu Thr Glu Gln Pro Glu Glu Thr Phe Pro Asn Thr Glu Thr Asn
   1               5                   10                  15
   Gly Glu Phe Gly Lys Arg Pro Ala Glu Asp Met Glu Glu Glu Gln Ala
               20                  25                  30
   Phe Lys Arg Ser Arg Asn Thr Asp Glu Met Val Glu Leu Arg Ile Leu
           35                  40                  45
   Leu Gln Ser Lys Asn Ala Gly Ala Val Ile Gly Lys Gly Gly Lys Asn
       50                  55                  60
   Ile Lys Ala Leu Arg Thr Asp Tyr Asn Ala Ser Val Ser Val Pro Asp
   65                  70                  75                  80
   Ser Ser Gly Pro Glu Arg Ile Leu Ser Ile Ser Ala Asp Ile Glu Thr
                   85                  90                  95
   Ile Gly Glu Ile Leu Lys Lys Ile Ile Pro Thr Leu Glu Glu Gly Leu
               100                 105                 110
   Gln Leu Pro Ser Pro Thr Ala Thr Ser Gln Leu Pro Leu Glu Ser Asp
           115                 120                 125
   Ala Val Glu Cys Leu Asn Tyr Gln His Tyr Lys Gly Ser Asp Phe Asp
       130                 135                 140
   Cys Glu Leu Arg Leu Leu Ile His Gln Ser Leu Ala Gly Gly Ile Ile
   145                 150                 155                 160
   Gly Val Lys Gly Ala Lys Ile Lys Glu Leu Arg Glu Asn Thr Gln Thr
                   165                 170                 175
   Thr Ile Lys Leu Phe Gln Glu Cys Cys Pro His Ser Thr Asp Arg Val
               180                 185                 190
   Val Leu Ile Gly Gly Lys Pro Asp Arg Val Val Glu Cys Ile Lys Ile
           195                 200                 205
   Ile Leu Asp Leu Ile Ser Glu Ser Pro Ile Lys Gly Arg Ala Gln Pro
       210                 215                 220
   Tyr Asp Pro Asn Phe Tyr Asp Glu Thr Tyr Asp Tyr Gly Gly Phe Thr
   225                 230                 235                 240
   Met Met Phe Asp Asp Arg Arg Gly Arg Pro Val Gly Phe Pro Met Arg
                   245                 250                 255
   Gly Arg Gly Gly Phe Asp Arg Met Pro Pro Gly Arg Gly Gly Arg Pro
               260                 265                 270
   Met Pro Pro Ser Arg Arg Asp Tyr Asp Asp Met Ser Pro Arg Arg Gly
           275                 280                 285
   Pro Pro Pro Pro Pro Pro Gly Arg Gly Gly Arg Gly Gly Ser Arg Ala
       290                 295                 300
   Arg Asn Leu Pro Leu Pro Pro Pro Pro Pro Pro Arg Gly Gly Asp Leu
   305                 310                 315                 320
   Met Ala Tyr Asp Arg Arg Gly Arg Pro Gly Asp Arg Tyr Asp Gly Met
                   325                 330                 335
   Val Gly Phe Ser Ala Asp Glu Thr Trp Asp Ser Ala Ile Asp Thr Trp
               340                 345                 350
   Ser Pro Ser Glu Trp Gln Met Ala Tyr Glu Pro Gln Gly Gly Ser Gly
           355                 360                 365
   Tyr Asp Tyr Ser Tyr Ala Gly Gly Arg Gly Ser Tyr Gly Asp Leu Gly
       370                 375                 380
   Gly Pro Ile Ile Thr Thr Gln Val Thr Ile Pro Lys Asp Leu Ala Gly
   385                 390                 395                 400
   Ser Ile Ile Gly Lys Gly Gly Gln Arg Ile Lys Gln Ile Arg His Glu
                   405                 410                 415
   Ser Gly Ala Ser Ile Lys Ile Asp Glu Pro Leu Glu Gly Ser Glu Asp
               420                 425                 430
   Arg Ile Ile Thr Ile Thr Gly Thr Gln Asp Gln Ile Gln Asn Ala Gln
           435                 440                 445
   Tyr Leu Leu Gln Asn Ser Val Lys Gln Tyr Ser Gly Lys Phe Phe
       450                 455                 460
<210> 371
   <211> 375
   <212> PRT
   <213> Homo sapiens
<400> 371
   Met Asp Asp Asp Ile Ala Ala Leu Val Val Asp Asn Gly Ser Gly Met
   1               5                   10                  15
   Cys Lys Ala Gly Phe Ala Gly Asp Asp Ala Pro Arg Ala Val Phe Pro
               20                  25                  30
   Ser Ile Val Gly Arg Pro Arg His Gln Gly Val Met Val Gly Met Gly
           35                  40                  45
   Gln Lys Asp Ser Tyr Val Gly Asp Glu Ala Gln Ser Lys Arg Gly Ile
       50                  55                  60
   Leu Thr Leu Lys Tyr Pro Ile Glu His Gly Ile Val Thr Asn Trp Asp
   65                  70                  75                  80
   Asp Met Glu Lys Ile Trp His His Thr Phe Tyr Asn Glu Leu Arg Val
                   85                  90                  95
   Ala Pro Glu Glu His Pro Val Leu Leu Thr Glu Ala Pro Leu Asn Pro
               100                 105                 110
   Lys Ala Asn Arg Glu Lys Met Thr Gln Ile Met Phe Glu Thr Phe Asn
           115                 120                 125
   Thr Pro Ala Met Tyr Val Ala Ile Gln Ala Val Leu Ser Leu Tyr Ala
       130                 135                 140
   Ser Gly Arg Thr Thr Gly Ile Val Met Asp Ser Gly Asp Gly Val Thr
   145                 150                 155                 160
   His Thr Val Pro Ile Tyr Glu Gly Tyr Ala Leu Pro His Ala Ile Leu
                   165                 170                 175
   Arg Leu Asp Leu Ala Gly Arg Asp Leu Thr Asp Tyr Leu Met Lys Ile
               180                 185                 190
   Leu Thr Glu Arg Gly Tyr Ser Phe Thr Thr Thr Ala Glu Arg Glu Ile
           195                 200                 205
   Val Arg Asp Ile Lys Glu Lys Leu Cys Tyr Val Ala Leu Asp Phe Glu
       210                 215                 220
   Gln Glu Met Ala Thr Ala Ala Ser Ser Ser Ser Leu Glu Lys Ser Tyr
   225                 230                 235                 240
   Glu Leu Pro Asp Gly Gln Val Ile Thr Ile Gly Asn Glu Arg Phe Arg
                   245                 250                 255
   Cys Pro Glu Ala Leu Phe Gln Pro Ser Phe Leu Gly Met Glu Ser Cys
               260                 265                 270
   Gly Ile His Glu Thr Thr Phe Asn Ser Ile Met Lys Cys Asp Val Asp
           275                 280                 285
   Ile Arg Lys Asp Leu Tyr Ala Asn Thr Val Leu Ser Gly Gly Thr Thr
       290                 295                 300
   Met Tyr Pro Gly Ile Ala Asp Arg Met Gln Lys Glu Ile Thr Ala Leu
   305                 310                 315                 320
   Ala Pro Ser Thr Met Lys Ile Lys Ile Ile Ala Pro Pro Glu Arg Lys
                   325                 330                 335
   Tyr Ser Val Trp Ile Gly Gly Ser Ile Leu Ala Ser Leu Ser Thr Phe
   340 345 350
   Gln Gln Met Trp Ile Ser Lys Gln Glu Tyr Asp Glu Ser Gly Pro Ser
   355 360 365
   Ile Val His Arg Lys Cys Phe
   370 375
<210> 372
   <211> 254
   <212> PRT
   <213> Homo sapiens
<400> 372
   Met Ala Ala Tyr Lys Leu Val Leu Ile Arg His Gly Glu Ser Ala Trp
   1               5                   10                  15
   Asn Leu Glu Asn Arg Phe Ser Gly Trp Tyr Asp Ala Asp Leu Ser Pro
               20                  25                  30
   Ala Gly His Glu Glu Ala Lys Arg Gly Gly Gln Ala Leu Arg Asp Ala
           35                  40                  45
   Gly Tyr Glu Phe Asp Ile Cys Phe Thr Ser Val Gln Lys Arg Ala Ile
       50                  55                  60
   Arg Thr Leu Trp Thr Val Leu Asp Ala Ile Asp Gln Met Trp Leu Pro
   65                  70                  75                  80
   Val Val Arg Thr Trp Arg Leu Asn Glu Arg His Tyr Gly Gly Leu Thr
                   85                  90                  95
   Gly Leu Asn Lys Ala Glu Thr Ala Ala Lys His Gly Glu Ala Gln Val
               100                 105                 110
   Lys Ile Trp Arg Arg Ser Tyr Asp Val Pro Pro Pro Pro Met Glu Pro
           115                 120                 125
   Asp His Pro Phe Tyr Ser Asn Ile Ser Lys Asp Arg Arg Tyr Ala Asp
       130                 135                 140
   Leu Thr Glu Asp Gln Leu Pro Ser Cys Glu Ser Leu Lys Asp Thr Ile
   145                 150                 155                 160
   Ala Arg Ala Leu Pro Phe Trp Asn Glu Glu Ile Val Pro Gln Ile Lys
                   165                 170                 175
   Glu Gly Lys Arg Val Leu Ile Ala Ala His Gly Asn Ser Leu Arg Gly
   180 185 190
   Ile Val Lys His Leu Glu Gly Leu Ser Glu Glu Ala Ile Met Glu Leu
   195 200 205
   Asn Leu Pro Thr Gly Ile Pro Ile Val Tyr Glu Leu Asp Lys Asn Leu
   210 215 220
   Lys Pro Ile Lys Pro Met Gln Phe Leu Gly Asp Glu Glu Thr Val Arg
   225 230 235 240
   Lys Ala Met Glu Ala Val Ala Ala Gln Gly Lys Ala Lys Lys
   245 250
<210> 373
   <211> 352
   <212> PRT
   <213> Homo sapiens
<400> 373
   Met Ala Leu Pro Phe Arg Lys Asp Leu Glu Lys Tyr Lys Asp Leu Asp
   1               5                   10                  15
   Glu Asp Glu Leu Leu Gly Asn Leu Ser Glu Thr Glu Leu Lys Gln Leu
               20                  25                  30
   Glu Thr Val Leu Asp Asp Leu Asp Pro Glu Asn Ala Leu Leu Pro Ala
           35                  40                  45
   Gly Phe Arg Gln Lys Asn Gln Thr Ser Lys Ser Thr Thr Gly Pro Phe
       50                  55                  60
   Asp Arg Glu His Leu Leu Ser Tyr Leu Glu Lys Glu Ala Leu Glu His
   65                  70                  75                  80
   Lys Asp Arg Glu Asp Tyr Val Pro Tyr Thr Gly Glu Lys Lys Gly Lys
                   85                  90                  95
   Ile Phe Ile Pro Lys Gln Lys Pro Val Gln Thr Phe Thr Glu Glu Lys
               100                 105                 110
   Val Ser Leu Asp Pro Glu Leu Glu Glu Ala Leu Thr Ser Ala Ser Asp
           115                 120                 125
   Thr Glu Leu Cys Asp Leu Ala Ala Ile Leu Gly Met His Asn Leu Ile
       130                 135                 140
   Thr Asn Thr Lys Phe Cys Asn Ile Met Gly Ser Ser Asn Gly Val Asp
   145 150 155 160
   Gln Glu His Phe Ser Asn Val Val Lys Gly Glu Lys Ile Leu Pro Val
   165 170 175
   Phe Asp Glu Pro Pro Asn Pro Thr Asn Val Glu Glu Ser Leu Lys Arg
   180 185 190
   Thr Lys Glu Asn Asp Ala His Leu Val Glu Val Asn Leu Asn Asn Ile
   195 200 205
   Lys Asn Ile Pro Ile Pro Thr Leu Lys Asp Phe Ala Lys Ala Leu Glu
   210 215 220
   Thr Asn Thr His Val Lys Cys Phe Ser Leu Ala Ala Thr Arg Ser Asn
   225 230 235 240
   Asp Pro Val Ala Thr Ala Phe Ala Glu Met Leu Lys Val Asn Lys Thr
   245 250 255
   Leu Lys Ser Leu Asn Val Glu Ser Asn Phe Ile Thr Gly Val Gly Ile
   260 265 270
   Leu Ala Leu Ile Asp Ala Leu Arg Asp Asn Glu Thr Leu Ala Glu Leu
   275 280 285
   Lys Ile Asp Asn Gln Arg Gln Gln Leu Gly Thr Ala Val Glu Leu Glu
   290 295 300
   Met Ala Lys Met Leu Glu Glu Asn Thr Asn Ile Leu Lys Phe Gly Tyr
   305 310 315 320
   Gln Phe Thr Gln Gln Gly Pro Arg Thr Arg Ala Ala Asn Ala Ile Thr
   325 330 335
   Lys Asn Asn Asp Leu Val Arg Lys Arg Arg Val Glu Gly Asp His Gln
   340 345 350
<210> 374
   <211> 350
   <212> PRT
   <213> Homo sapiens
<400> 374
   Met Ala Met Arg Gln Thr Pro Leu Thr Cys Ser Gly His Thr Arg Pro
   1               5                   10                  15
   Val Val Asp Leu Ala Phe Ser Gly Ile Thr Pro Tyr Gly Tyr Phe Leu
               20                  25                  30
   Ile Ser Ala Cys Lys Asp Gly Lys Pro Met Leu Arg Gln Gly Asp Thr
           35                  40                  45
   Gly Asp Trp Ile Gly Thr Phe Leu Gly His Lys Gly Ala Val Trp Gly
       50                  55                  60
   Ala Thr Leu Asn Lys Asp Ala Thr Lys Ala Ala Thr Ala Ala Ala Asp
   65                  70                  75                  80
   Phe Thr Ala Lys Val Trp Asp Ala Val Ser Gly Asp Glu Leu Met Thr
                   85                  90                  95
   Leu Ala His Lys His Ile Val Lys Thr Val Asp Phe Thr Gln Asp Ser
               100                 105                 110
   Asn Tyr Leu Leu Thr Gly Gly Gln Asp Lys Leu Leu Arg Ile Tyr Asp
           115                 120                 125
   Leu Asn Lys Pro Glu Ala Glu Pro Lys Glu Ile Ser Gly His Thr Ser
       130                 135                 140
   Gly Ile Lys Lys Ala Leu Trp Cys Ser Glu Asp Lys Gln Ile Leu Ser
   145                 150                 155                 160
   Ala Asp Asp Lys Thr Val Arg Leu Trp Asp His Ala Thr Met Thr Glu
                   165                 170                 175
   Val Lys Ser Leu Asn Phe Asn Met Ser Val Ser Ser Met Glu Tyr Ile
               180                 185                 190
   Pro Glu Gly Glu Ile Leu Val Ile Thr Tyr Gly Arg Ser Ile Ala Phe
           195                 200                 205
   His Ser Ala Val Ser Leu Asp Pro Ile Lys Ser Phe Glu Ala Pro Ala
       210                 215                 220
   Thr Ile Asn Ser Ala Ser Leu His Pro Glu Lys Glu Phe Leu Val Ala
   225                 230                 235                 240
   Gly Gly Glu Asp Phe Lys Leu Tyr Lys Tyr Asp Tyr Asn Ser Gly Glu
                   245                 250                 255
   Glu Leu Glu Ser Tyr Lys Gly His Phe Gly Pro Ile His Cys Val Arg
               260                 265                 270
   Phe Ser Pro Asp Gly Glu Leu Tyr Ala Ser Gly Ser Glu Asp Gly Thr
   275 280 285
   Leu Arg Leu Trp Gln Thr Val Val Gly Lys Thr Tyr Gly Leu Trp Lys
   290 295 300
   Cys Val Leu Pro Glu Glu Asp Ser Gly Glu Leu Ala Lys Pro Lys Ile
   305 310 315 320
   Gly Phe Pro Glu Thr Thr Glu Glu Glu Leu Glu Glu Ile Ala Ser Glu
   325 330 335
   Asn Ser Asp Cys Ile Phe Pro Ser Ala Pro Asp Val Lys Ala
   340 345 350
<210> 375
   <211> 263
   <212> PRT
   <213> Homo sapiens
<400> 375
   Met Phe Arg Asn Gln Tyr Asp Asn Asp Val Thr Val Trp Ser Pro Gln
   1 5 10 15
   Gly Arg Ile His Gln Ile Glu Tyr Ala Met Glu Ala Val Lys Gln Gly
   20 25 30
   Ser Ala Thr Val Gly Leu Lys Ser Lys Thr His Ala Val Leu Val Ala
   35 40 45
   Leu Lys Arg Ala Gln Ser Glu Leu Ala Ala His Gln Lys Lys Ile Leu
   50 55 60
   His Val Asp Asn His Ile Gly Ile Ser Ile Ala Gly Leu Thr Ala Asp
   65 70 75 80
   Ala Arg Leu Leu Cys Asn Phe Met Arg Gln Glu Cys Leu Asp Ser Arg
   85 90 95
   Phe Val Phe Asp Arg Pro Leu Pro Val Ser Arg Leu Val Ser Leu Ile
   100 105 110
   Gly Ser Lys Thr Gln Ile Pro Thr Gln Arg Tyr Gly Arg Arg Pro Tyr
   115 120 125
   Gly Val Gly Leu Leu Ile Ala Gly Tyr Asp Asp Met Gly Pro His Ile
   130 135 140
   Phe Gln Thr Cys Pro Ser Ala Asn Tyr Phe Asp Cys Arg Ala Met Ser
   145                 150                 155                 160
   Ile Gly Ala Arg Ser Gln Ser Ala Arg Thr Tyr Leu Glu Arg His Met
                   165                 170                 175
   Ser Glu Phe Met Glu Cys Asn Leu Asn Glu Leu Val Lys His Gly Leu
               180                 185                 190
   Arg Ala Leu Arg Glu Thr Leu Pro Ala Glu Gln Asp Leu Thr Thr Lys
           195                 200                 205
   Asn Val Ser Ile Gly Ile Val Gly Lys Asp Leu Glu Phe Thr Ile Tyr
       210                 215                 220
   Asp Asp Asp Asp Val Ser Pro Phe Leu Glu Gly Leu Glu Glu Arg Pro
   225                 230                 235                 240
   Gln Arg Lys Ala Gln Pro Ala Gln Pro Ala Asp Glu Pro Ala Glu Lys
                   245                 250                 255
   Ala Asp Glu Pro Met Glu His
               260
<210> 376
   <211> 115
   <212> PRT
   <213> Homo sapiens
<400> 376
   Met Arg Tyr Val Ala Ser Tyr Leu Leu Ala Ala Leu Gly Gly Asn Ser
   1               5                   10                  15
   Ser Pro Ser Ala Lys Asp Ile Lys Lys Ile Leu Asp Ser Val Gly Ile
               20                  25                  30
   Glu Ala Asp Asp Asp Arg Leu Asn Lys Val Ile Ser Glu Leu Asn Gly
           35                  40                  45
   Lys Asn Ile Glu Asp Val Ile Ala Gln Gly Ile Gly Lys Leu Ala Ser
       50                  55                  60
   Val Pro Ala Gly Gly Ala Val Ala Val Ser Ala Ala Pro Gly Ser Ala
   65                  70                  75                  80
   Ala Pro Ala Ala Gly Ser Ala Pro Ala Ala Ala Glu Glu Lys Lys Asp
                   85                  90                  95
   Glu Lys Lys Glu Glu Ser Glu Glu Ser Asp Asp Asp Met Gly Phe Gly
   100 105 110
   Leu Phe Asp
   115
<210> 377
   <211> 320
   <212> PRT
   <213> Homo sapiens
<400> 377
   Met Ala Gln Val Leu Arg Gly Thr Val Thr Asp Phe Pro Gly Phe Asp
   1 5 10 15
   Glu Arg Ala Asp Ala Glu Thr Leu Arg Lys Ala Met Lys Gly Leu Gly
   20 25 30
   Thr Asp Glu Glu Ser Ile Leu Thr Leu Leu Thr Ser Arg Ser Asn Ala
   35 40 45
   Gln Arg Gln Glu Ile Ser Ala Ala Phe Lys Thr Leu Phe Gly Arg Asp
   50 55 60
   Leu Leu Asp Asp Leu Lys Ser Glu Leu Thr Gly Lys Phe Glu Lys Leu
   65 70 75 80
   Ile Val Ala Leu Met Lys Pro Ser Arg Leu Tyr Asp Ala Tyr Glu Leu
   85 90 95
   Lys His Ala Leu Lys Gly Ala Gly Thr Asn Glu Lys Val Leu Thr Glu
   100 105 110
   Ile Ile Ala Ser Arg Thr Pro Glu Glu Leu Arg Ala Ile Lys Gln Val
   115 120 125
   Tyr Glu Glu Glu Tyr Gly Ser Ser Leu Glu Asp Asp Val Val Gly Asp
   130 135 140
   Thr Ser Gly Tyr Tyr Gln Arg Met Leu Val Val Leu Leu Gln Ala Asn
   145 150 155 160
   Arg Asp Pro Asp Ala Gly Ile Asp Glu Ala Gln Val Glu Gln Asp Ala
   165 170 175
   Gln Ala Leu Phe Gln Ala Gly Glu Leu Lys Trp Gly Thr Asp Glu Glu
   180 185 190
   Lys Phe Ile Thr Ile Phe Gly Thr Arg Ser Val Ser His Leu Arg Lys
   195 200 205
   Val Phe Asp Lys Tyr Met Thr Ile Ser Gly Phe Gln Ile Glu Glu Thr
   210 215 220
   Ile Asp Arg Glu Thr Ser Gly Asn Leu Glu Gln Leu Leu Leu Ala Val
   225 230 235 240
   Val Lys Ser Ile Arg Ser Ile Pro Ala Tyr Leu Ala Glu Thr Leu Tyr
   245 250 255
   Tyr Ala Met Lys Gly Ala Gly Thr Asp Asp His Thr Leu Ile Arg Val
   260 265 270
   Met Val Ser Arg Ser Glu Ile Asp Leu Phe Asn Ile Arg Lys Glu Phe
   275 280 285
   Arg Lys Asn Phe Ala Thr Ser Leu Tyr Ser Met Ile Lys Gly Asp Thr
   290 295 300
   Ser Gly Asp Tyr Lys Lys Ala Leu Leu Leu Leu Cys Gly Glu Asp Asp
   305 310 315 320
<210> 378
   <211> 535
   <212> PRT
   <213> Homo sapiens
<400> 378
   Met Ala Ser Leu Ser Leu Ala Pro Val Asn Ile Phe Lys Ala Gly Ala
   1 5 10 15
   Asp Glu Glu Arg Ala Glu Thr Ala Arg Leu Thr Ser Phe Ile Gly Ala
   20 25 30
   Ile Ala Ile Gly Asp Leu Val Lys Ser Thr Leu Gly Pro Lys Gly Met
   35 40 45
   Asp Lys Ile Leu Leu Ser Ser Gly Arg Asp Ala Ser Leu Met Val Thr
   50 55 60
   Asn Asp Gly Ala Thr Ile Leu Lys Asn Ile Gly Val Asp Asn Pro Ala
   65 70 75 80
   Ala Lys Val Leu Val Asp Met Ser Arg Val Gln Asp Asp Glu Val Gly
   85 90 95
   Asp Gly Thr Thr Ser Val Thr Val Leu Ala Ala Glu Leu Leu Arg Glu
               100                 105                 110
   Ala Glu Ser Leu Ile Ala Lys Lys Ile His Pro Gln Thr Ile Ile Ala
           115                 120                 125
   Gly Trp Arg Glu Ala Thr Lys Ala Ala Arg Glu Ala Leu Leu Ser Ser
       130                 135                 140
   Ala Val Asp His Gly Ser Asp Glu Val Lys Phe Arg Gln Asp Leu Met
   145                 150                 155                 160
   Asn Ile Ala Gly Thr Thr Leu Ser Ser Lys Leu Leu Thr His His Lys
                   165                 170                 175
   Asp His Phe Thr Lys Leu Ala Val Glu Ala Val Leu Arg Leu Lys Gly
               180                 185                 190
   Ser Gly Asn Leu Glu Ala Ile His Ile Ile Lys Lys Leu Gly Gly Ser
           195                 200                 205
   Leu Ala Asp Ser Tyr Leu Asp Glu Gly Phe Leu Leu Asp Lys Lys Ile
       210                 215                 220
   Gly Val Asn Gln Pro Lys Arg Ile Glu Asn Ala Lys Ile Leu Ile Ala
   225                 230                 235                 240
   Asn Thr Gly Met Asp Thr Asp Lys Ile Lys Ile Phe Gly Ser Arg Val
                   245                 250                 255
   Arg Val Asp Ser Thr Ala Lys Val Ala Glu Ile Glu His Ala Glu Lys
               260                 265                 270
   Glu Lys Met Lys Glu Lys Val Glu Arg Ile Leu Lys His Gly Ile Asn
           275                 280                 285
   Cys Phe Ile Asn Arg Gln Leu Ile Tyr Asn Tyr Pro Glu Gln Leu Phe
       290                 295                 300
   Gly Ala Ala Gly Val Met Ala Ile Glu His Ala Asp Phe Ala Gly Val
   305                 310                 315                 320
   Glu Arg Leu Ala Leu Val Thr Gly Gly Glu Ile Ala Ser Thr Phe Asp
                   325                 330                 335
   His Pro Glu Leu Val Lys Leu Gly Ser Cys Lys Leu Ile Glu Glu Val
               340                 345                 350
   Met Ile Gly Glu Asp Lys Leu Ile His Phe Ser Gly Val Ala Leu Gly
   355 360 365
   Glu Ala Cys Thr Ile Val Leu Arg Gly Ala Thr Gln Gln Ile Leu Asp
   370 375 380
   Glu Ala Glu Arg Ser Leu His Asp Ala Leu Cys Val Leu Ala Gln Thr
   385 390 395 400
   Val Lys Asp Ser Arg Thr Val Tyr Gly Gly Gly Cys Ser Glu Met Leu
   405 410 415
   Met Ala His Ala Val Thr Gln Leu Ala Asn Arg Thr Pro Gly Lys Glu
   420 425 430
   Ala Val Ala Met Glu Ser Tyr Ala Lys Ala Leu Arg Met Leu Pro Thr
   435 440 445
   Ile Ile Ala Asp Asn Ala Gly Tyr Asp Ser Ala Asp Leu Val Ala Gln
   450 455 460
   Leu Arg Ala Ala His Ser Glu Gly Asn Thr Thr Ala Gly Leu Asp Met
   465 470 475 480
   Arg Glu Gly Thr Ile Gly Asp Met Ala Ile Leu Gly Ile Thr Glu Ser
   485 490 495
   Phe Gln Val Lys Arg Gln Val Leu Leu Ser Ala Ala Glu Ala Ala Glu
   500 505 510
   Val Ile Leu Arg Val Asp Asn Ile Ile Lys Ala Ala Pro Arg Lys Arg
   515 520 525
   Val Pro Asp His His Pro Cys
   530 535
<210> 379
   <211> 461
   <212> PRT
   <213> Homo sapiens
<400> 379

## Description

### Field of the invention

The invention relates to a method to monitor viral infection progression. The method is based on measuring T cell response or auto-antibodies to apoptotic epitopes (antigenic determinants).

### Background of the invention

Successful priming of naive CD4⁺ or CD8⁺ T cells results in the generation of both effector memory T (T_{EM)} cells expressing various differentiation programs (type-1, -2, -17), according to the environment in which they are exposed (1-8), and central memory T (T_{CM}) cells that promptly proliferate and generate new waves of effector cells on demand (9-11). The transcription factor T-box-containing protein expressed in T cells (T-bet) is the master regulator of the type-1 cell differentiation program that is associated with the production of IFN-γ, which is required for the development of protective immune responses against intracellular pathogens (2). GATA-binding protein 3 (GATA-3) controls the development of the type-2 cell lineage that is characterized by the production of IL-4, -5, and -13, which is critical for immunity against helminths and other extracellular pathogens (2). Retinoid acid-related orphan receptor (ROR)-γt in mice and the human ortholog RORC in humans represent the master regulators of type-17 cell differentiation that leads to the production of IL-17, which is specifically required for protection against several types of extracellular and intracellular bacterial infections (1, 3-5). All these (type-1, -2, -17) functions can elicit either protective or harmful effects, depending on whether they are executed by pathogen-specific or autoreactive T cells or whether the pathogen- specific are involved during an acute resolving infection or a chronic infection, respectively.

The fate of the enormous number of apoptotic cells that derive from effector T cells undergoing apoptosis after performing their functions during acute or chronic infections remain to be determined (12, 13). In previous studies, the author found that the proteome of apoptotic T cells includes prominent caspase-cleaved cellular proteins and that a high proportion of distinct T cell epitopes in these fragments (apoptotic epitopes) can be cross-presented by DCs to a wide repertoire of autoreactive CD8⁺ T cells (14). Recent reports have confirmed the role of caspase cleavage in the processing and presentation of epitopes that are derived from apoptotic cells in different models (15-17). In chronic HIV infection, these autoreactive CD8⁺ T cells correlate with the proportion of apoptotic CD4⁺ T cells *in vivo* and are involved in establishing polyclonal T cell activation that in the long run results in generalized T cell dysfunction/depletion (14). In addition, apoptotic cells derived from activated T cells (in contrast to those derived from resting T cells) retain the expression of CD40 ligand (L) and can then condition CD40⁺ DCs to acquire high capacities to prime or cross-prime autoreactive T cells (18, 19). Therefore, the expression of CD40L on apoptotic T cells can bypass the tolerogenic effects of conventional apoptotic cells (i.e., those derived from epithelial cells) and influence the switch from tolerogenic immature DCs to mature DCs with high stimulatory and migratory capacities (18, 19). This mechanism is consistent with the evidence that the signals provided by CD40L⁺ apoptotic cells and not those provided by conventional apoptotic cells facilitate the emergence of autoreactive T cell responses to apoptotic self-antigens (18, 19).

**Virological tools to diagnose and monitor hepatitis C virus infection are described in the review** Chevaliez S., Clin. Microbiol. Infect. 2011; 17:116-121**.**

In the present invention, the authors used the HCV infection as a human model of acute infection that generally undergoes chronic progression to verify whether CD8⁺ T cells that are specific for apoptotic self-epitopes have a distinct effector type-1, -2, or -17 phenotype, to distinguish which of them may participate in determining the fate of a viral infection (recovery versus chronicity), and to ascertain the mechanisms whereby these responses are induced and maintained.

### Brief description of the invention

Caspase-dependent cleavage of antigens associated with apoptotic cells has a prominent role in the generation of CD8⁺ T cell responses in various infectious diseases. The author found that the emergence of a large population of autoreactive CD8⁺ T effector cells specific for apoptotic T cell-associated self-epitopes exceeds the antiviral responses in patients with acute HCV infection. Importantly, they endow mixed polyfunctional type-1, type-2 and type-17 responses and correlate with the chronic progression of infection. This evolution is related to the selection of autoreactive CD8⁺ T cells with higher TCR avidity, whereas those with lower avidity undergo prompt contraction in patients who clear infection. The development of mixed responses with divergent differentiation requirements is consistent with distinct sites or kinetics of CD8⁺ T cell priming *in vivo.* These findings demonstrate a previously undescribed strict link between the emergence of high frequencies of mixed autoreactive CD8⁺ T cells producing a broad array of cytokines (IFN-γ, IL-17, IL-4, IL-2...) and the progression toward chronic disease in a human model of acute infection.

The present invention is based on autoimmune response capacities against antigen peptides derived from apoptotic cells (apoptotic epitopes) both to be used as biomarkers in the diagnosis, prognosis and to evaluate chronic evolution of various viral infections or autoimmune diseases. The present results clearly specify that such responses predict the chronic evolution of acute infections such as Hepatitis C (HCV), Hepatitis B (HBV) and HIV.

In fact, patients with acute infections as HCV etc. that, in early phases show high levels of such autoimmune responses, progress to chronic infection. The responses are correlated with tissue damage signs and high viral load. Contrarily, patients with the same acute infections that do not present such responses undergo infection resolution. In other words, said autoimmune responses predict the infection fate. In confirmation of this, the author monitored such patients with a follow-up from acute phase to chronic phase (chronic hepatitis/cirrhosis) of HCV and verified that such autoimmune responses were sustained over time only in patients progressing to chronic infection in relation with viral persistence and high transaminase levels (liver injury indication).

Therefore, the present invention represents a unique biomarker that predicts the fate (resolution vs. chronicization) of an acute infection, since that no other known clinical biomarker is available to date.

In the present invention it is confirmed that such autoimmune responses are represented by autoreactive polyfunctional CD8 T lymphocytes, since they produce high levels of different cytokines (INF-γ, IL-17, IL-23, IL-4, IL-2...) in response to apoptotic epitopes. Again, such polyfunctional responses are predictive of chronic infection. It is interesting to note that these polyfunctional responses decrease and/or modify prematurely in relation to the resolution of infection treated with different anti-viral drugs. Therefore, such responses can be used as:
i) Early biomarkers that predict the fate of an acute infection with recoils not only in diagnosis, but also in early therapeutic outline: consequently, adequate therapies can be set up prior to progression towards chronicization;
ii) Biomarkers that can be detected to determine protective effect of conventional or innovative anti-viral therapies, with enormous advantages for national health service since it could be early predicted if certain therapy will work or not.

The previous published study (14) identifies apoptotic epitopes only in molecular and immunogenic terms. In the study, it is also demonstrated that relative autoimmune responses are present in HIV chronic infection and correlates to viraemia, but there are no data or hypothesis that such responses, when present in acute infections, are predictive of disease evolution. This last point is at the base of the definition of biomarker: a biomarker cannot be defined as such if it does not have predictability value.

The present invention defines the first predictive biomarker for chronic evolution of acute infections, development of protective effects of therapies, of prognosis and therapies of autoimmune disease and their sequences.

It is therefore an object of the invention a method to predict the outcome of a viral infection and/or to monitor the progress of a viral infection and/or to monitor the efficacy of a therapy for a viral infection in a biological sample comprising T cell obtained from a subject affected by said viral infection comprising the steps of
a) incubating the isolated biological sample with at least one peptide consisting of a sequence selected from SEQ ID No.1 to SEQ ID No. 252 under appropriate conditions to generate a T cell response;
b) measuring the T cell response;
c) comparing the measured T cell response to the measured T cell response of a suitable control sample,
wherein said viral infection is caused by a virus selected from the group of HCV, HBV, thereby discriminating patients experiencing chronic infection and those undergoing infection resolution.

Preferably the biological sample is a sample of PBMCs or blood.

Preferably the at least one peptide of step a) **consist of the** sequence selected from LLQDSVDFSL (SEQ ID No. 204), QLFNHTMFI (SEQ ID No. 64) or VLMIKALEL (SEQ ID No. 78).

Still preferably step b) comprises incubating the biological sample with a pool of peptides selected among the following pools:
- pool 1: SEQ ID Nos. 1, 2, 4, 5, 8 and 9;
- pool 2: SEQ ID Nos. 14, 16, 18, 19 and 20;
- pool 3: SEQ ID Nos. 27, 28, 29, 30, 34, 35, 36, 37 and 40;
- pool 4: SEQ ID Nos. 58, 59, 60, 62, 63, 64, 65, 66 and 67;
- pool 5: SEQ ID Nos. 68, 69, 73, 74, 75, 76, 77, 78, 81, 83, 84 and 88;
- pool 6: SEQ ID Nos. 89, 90, 91, 92, 99, 100, 104, 105, 106, 111, 117, 123, 126 and 127;
- pool 7: SEQ ID Nos. 198, 199, 200, 201, 202, 204, 206, 210, 211 and 220;
- pool 8: SEQ ID Nos. 232, 233, 234, 235, 236, 237, 238, 239, 240, 241, 242, 243 and 244 or
- pool 9: SEQ ID Nos. 245, 246, 247, 248 and 249.

In a preferred embodiment the T cell response is a CD8⁺ T cell response.

Preferably the T cell response is measured by means of an Elispot assay.

Still preferably the Elispot assay is an interferon-gamma, IL-17 or IL-4 Elispot assay.

It is a further object of the invention a method to monitor the progress of a viral infection and/or to monitor the efficacy of a therapy for a viral infection in a subject affected by said viral infection comprising the step of detecting and/or quantifying the amount of auto-antibodies directed against at least one peptide as defined above, wherein said viral infection is caused by a virus selected from the group of HCV, HBV, thereby discriminating patients experiencing chronic infection and those undergoing infection resolution.

The amount of auto-antibodies directed against at least one peptide may be compared to values measured in a suitable control sample.

In a preferred embodiment the viral infection is caused by a virus selected from the group of HCV and HBV.

In the present invention the outcome of a viral infection may be a resolution of the infection or the progression toward chronic infection.

The methods of the present invention may comprise isolating a fresh biological sample or using a frozen or cryopreserved biological sample T cells response may also be measured by methods known by the skilled person in the art such as intracellular staining methods, cytofluorimetric methods, ect.

T cells response or detection and/or quantification of auto-antibodies against the peptides may be measured at regular intervals (either weekly, or every 2^{nd}, 3^{rd} or 4^{th} week or at longer, shorter and variable intervals) during the course of the viral infection or during the course of the therapy. The analysis of the responses or auto-antibodies detection/quantification at the different time points may be compared to the first and/or to previous examinations to identify relative changes in responses.

A suitable control sample may be a sample obtained or isolated before the start of a therapy, the sample of a healthy subject, the sample of a subject whose infection progress towards resolution, a sample obtained or isolated at various time points during the course of the infection or during the course of the therapy.

The invention will be now illustrated by means of non-limiting figures.

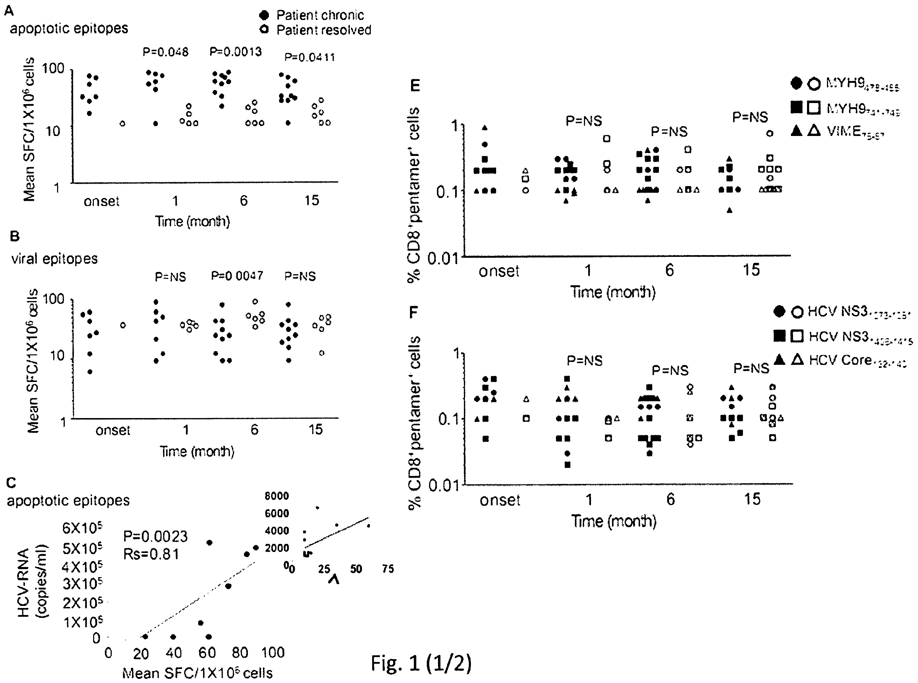

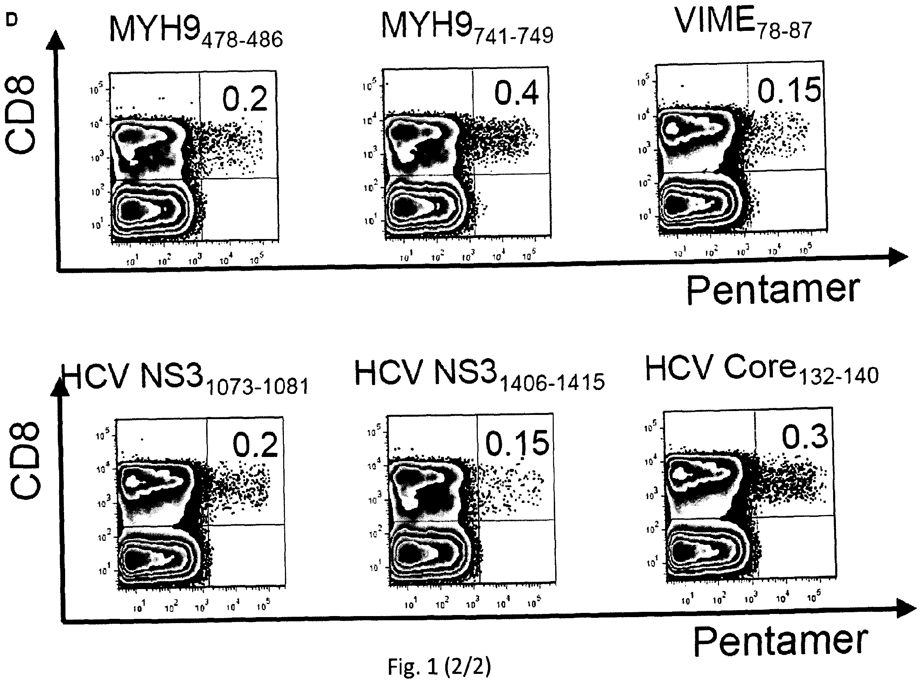

**Figure 1****: CD8⁺ T cell multispecificity to apoptotic epitopes in chronic HCV progression. (A,B)** Mean number of spot-forming cells (SFCs) by fresh CD8⁺ T effector memory (T_{EM}) cells (by ELISPOT assay) in response to pools (see **Tables 11-13 and 14-17**) of apoptotic self-epitopes (A) or viral epitopes (B) in patients with acute HCV infection experiencing chronic infection (filled circles) or undergoing infection resolution (empty circles), evaluated at different time points of the infection. Each circle represents a single patient. NS = not significant. (**C**) Representative correlation (6^{th} month after clinical onset) between the mean number of SFCs formed by fresh CD8⁺ T_{EM} cells in response to pools of apoptotic self-epitopes and the viral load (HCV-RNA copies/ml) in patients experiencing chronic infection (filled circles) or undergoing infection resolution (empty circles). The inset graph highlights the same correlation in patients whose viral load variations were not evident in the larger graph. Statistical analysis was performed using non-parametric Spearman's test. (**D**) Representative flow cytometry analyses of peripheral blood mononuclear cells (PBMCs), double-stained with a monoclonal antibody (mAb) to CD8 and pentamers expressing the indicated peptides of non muscle myosin heavy chain 9 (MYH9) or vimentin (VIME). Counterplot analyses show the percentage of CD8⁺pentamer⁺ cells. The percentage of cells is indicated in each quadrant. The pentamer values were <0.02% (mean ± s.d. = 0.0173% ± 0.0009% for all pentamers complexed to apoptotic epitopes; 0.0052% ± 0.0035% for those complexed to viral epitopes) in 20 HLA-A2⁺ healthy individuals; thus 0.02% was considered the sensitive level of the assay. (**E,F**) Percentages of CD8⁺pentamer⁺ cells from all patients studied (filled and empty symbols represent patients experiencing chronic infection or undergoing infection resolution, respectively), evaluated at different time points. Each symbo 1 represents the indicated pentamer specificity. NS = not significant.

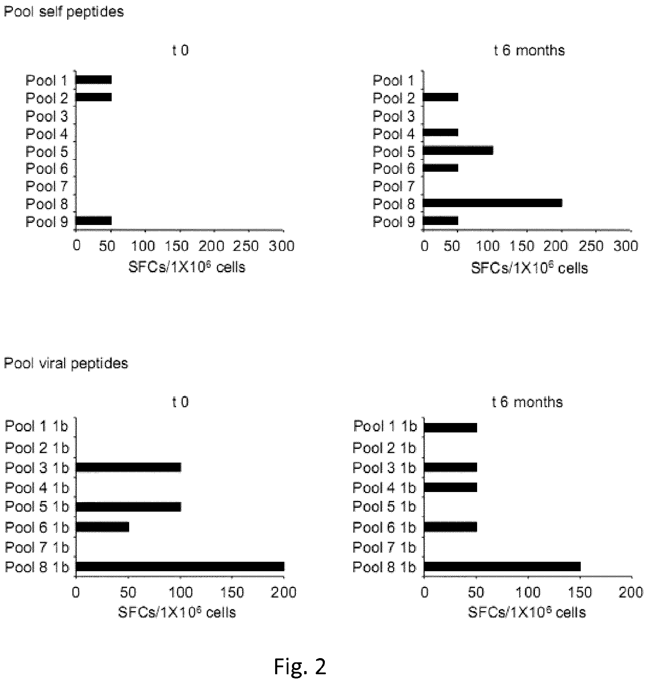

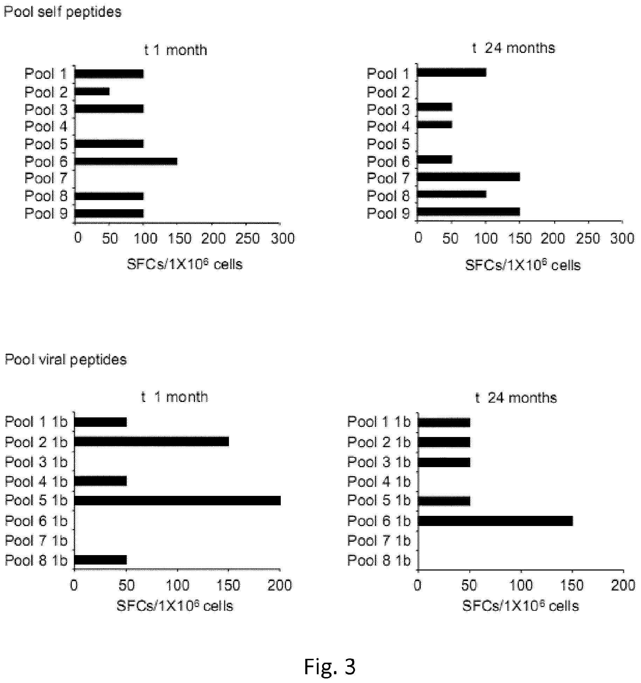

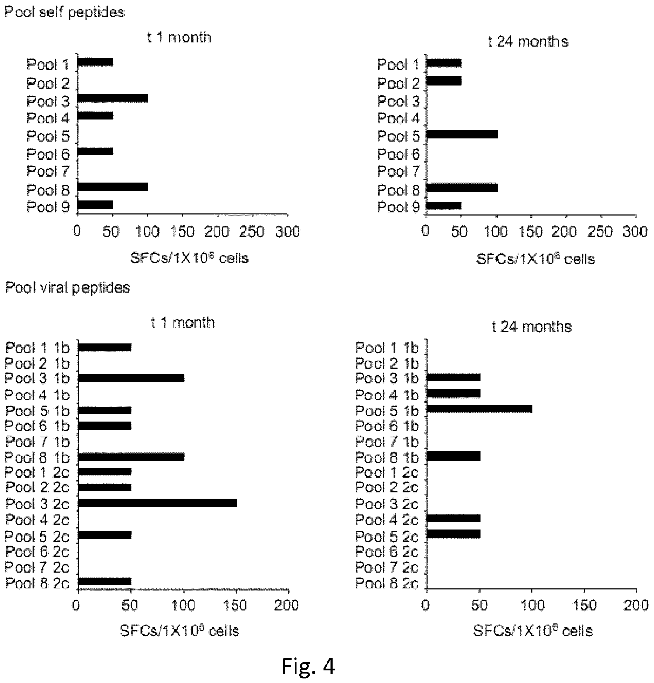

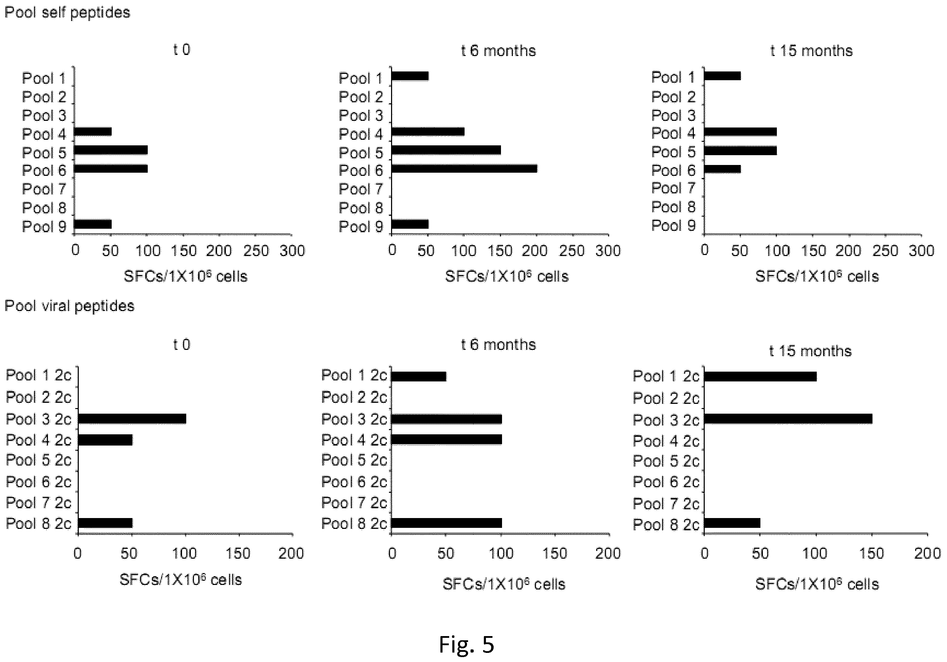

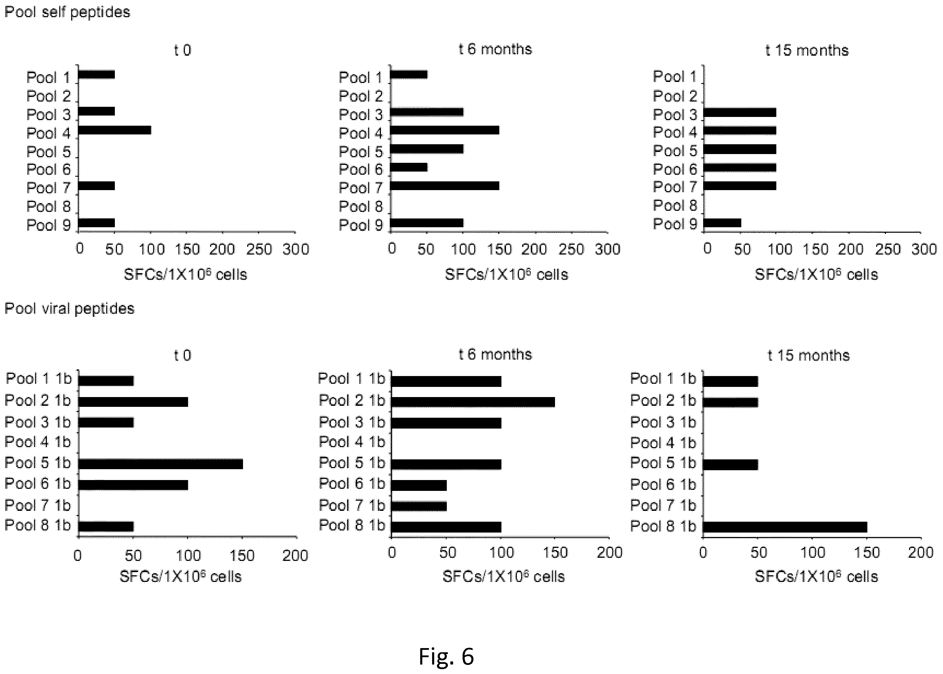

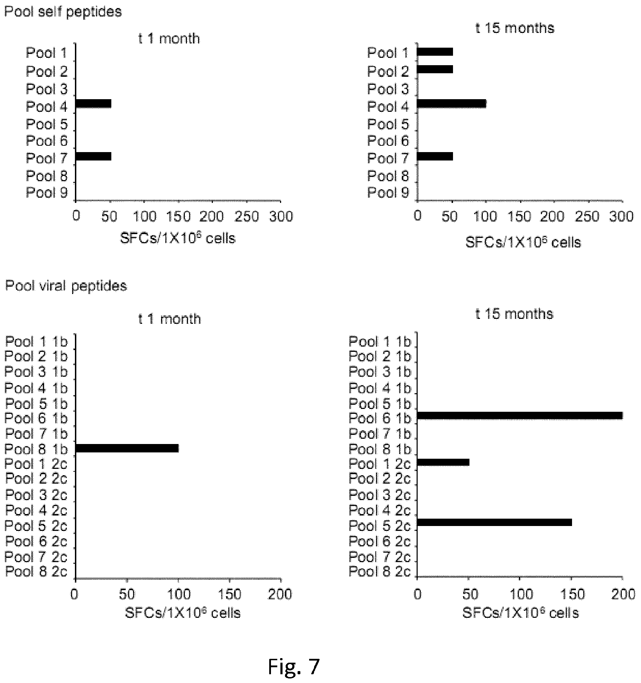

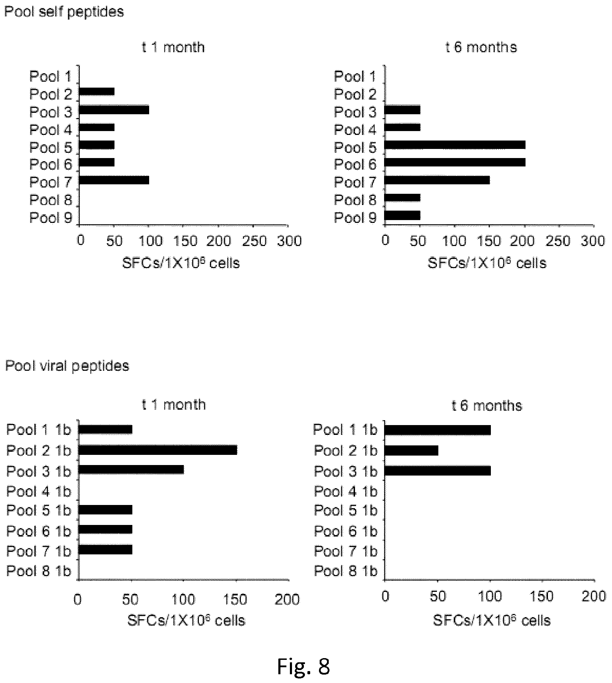

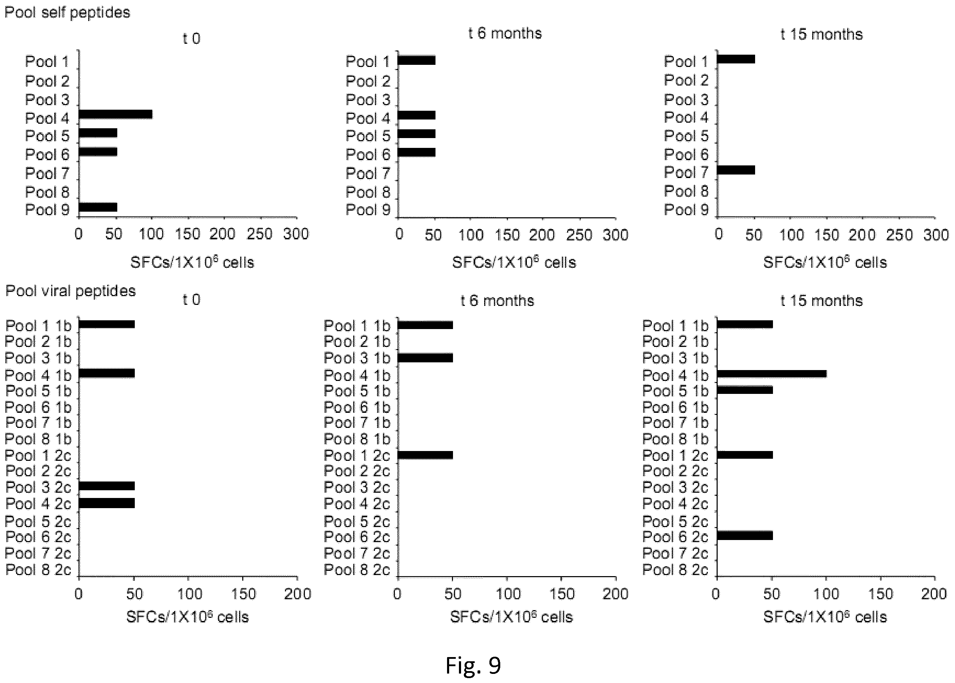

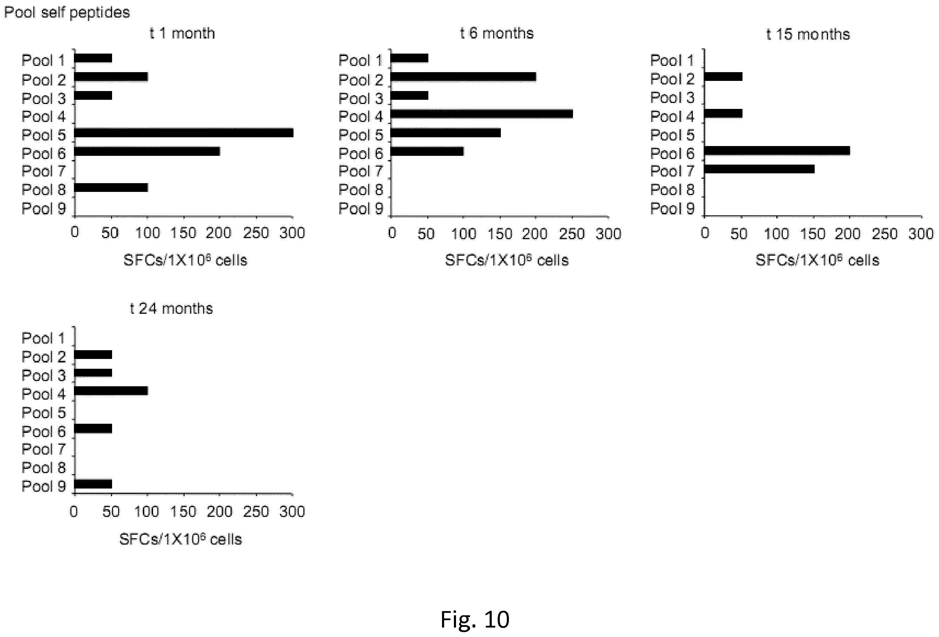

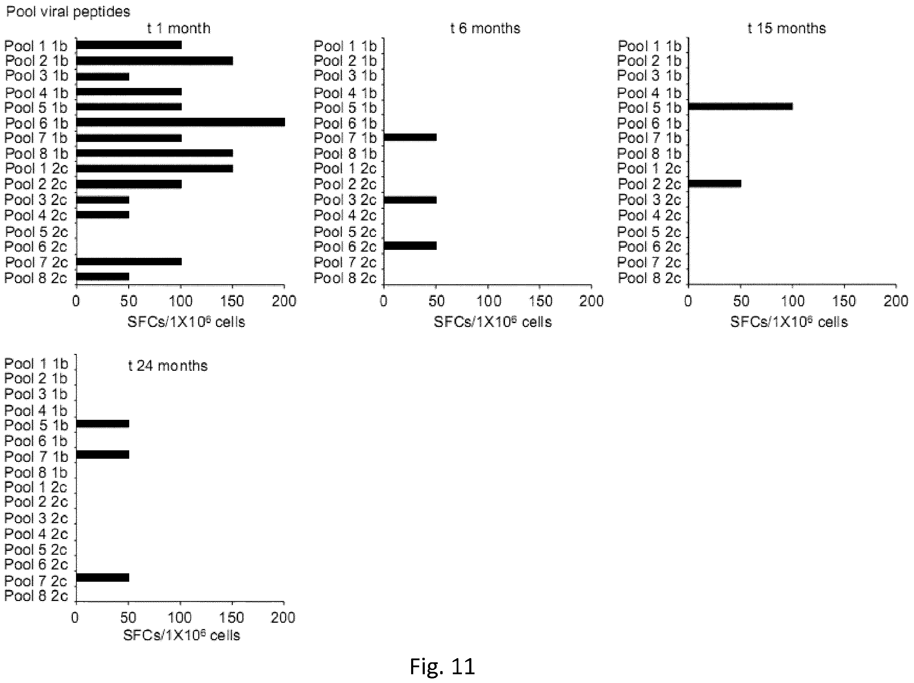

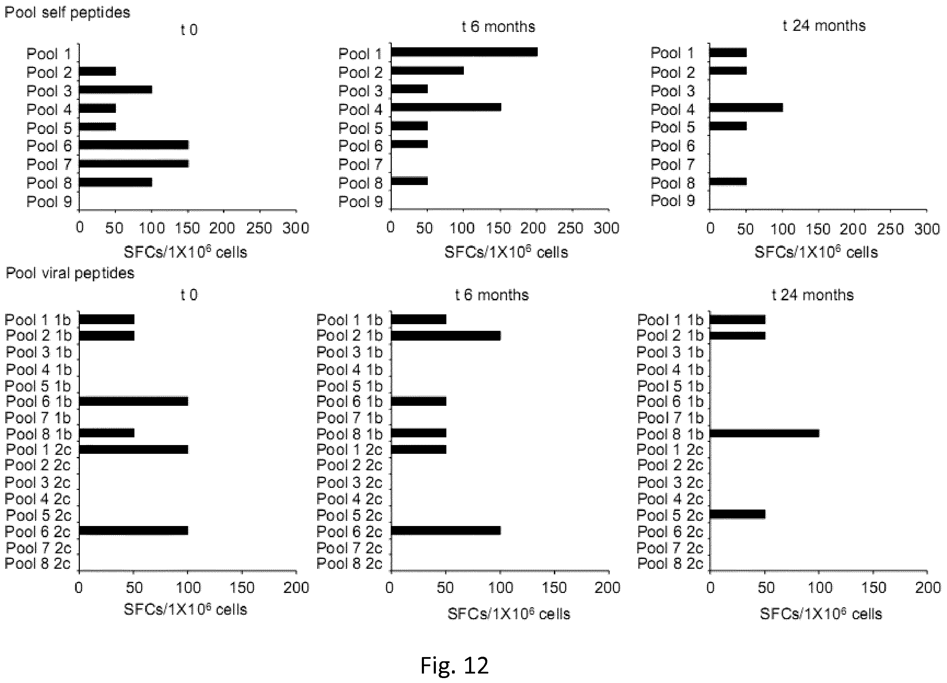

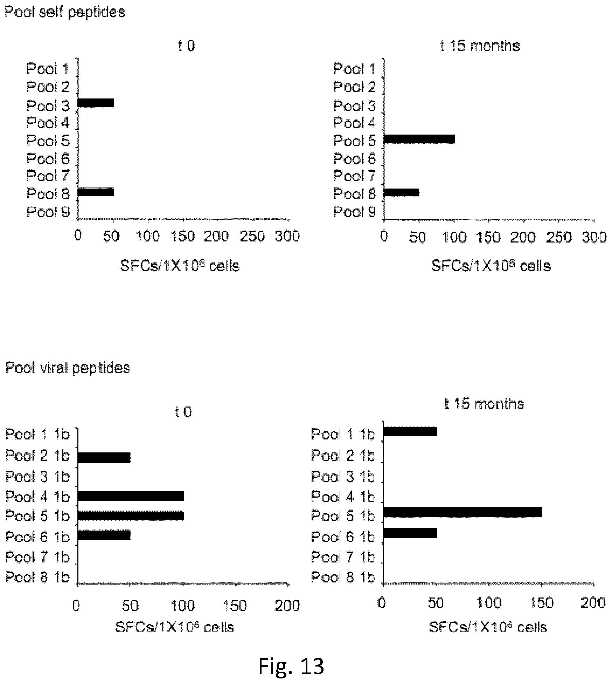

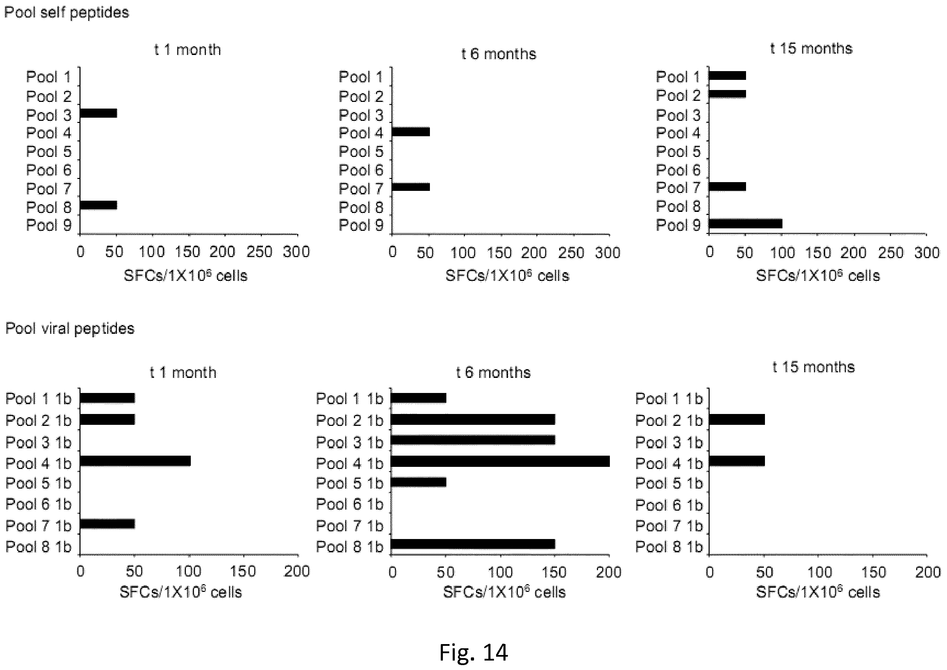

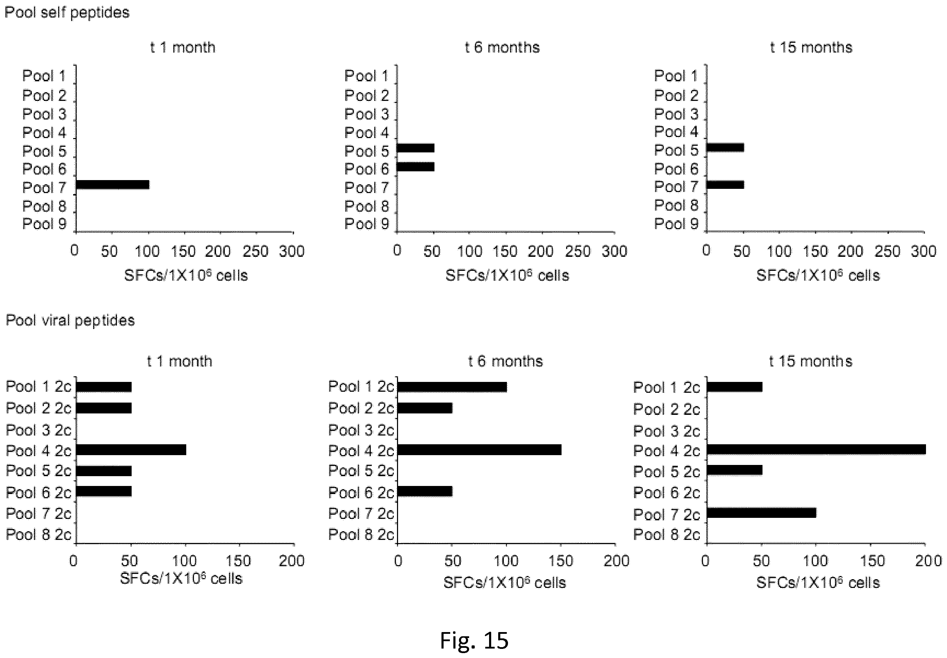

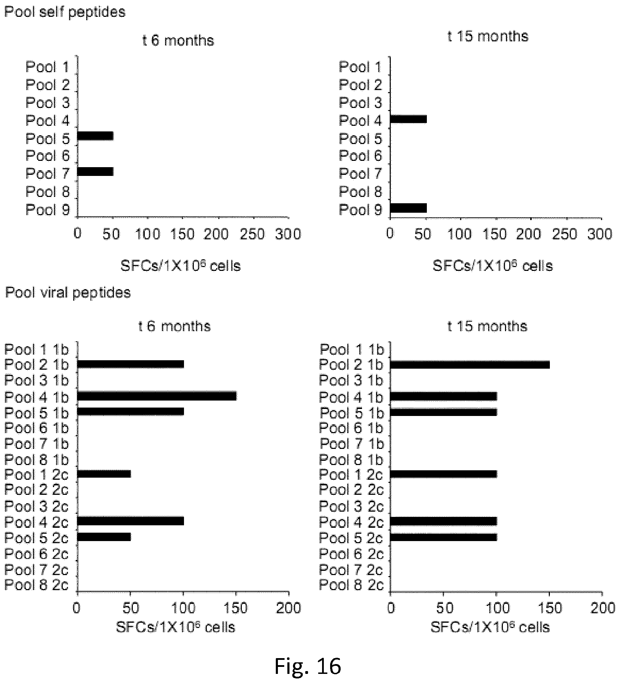

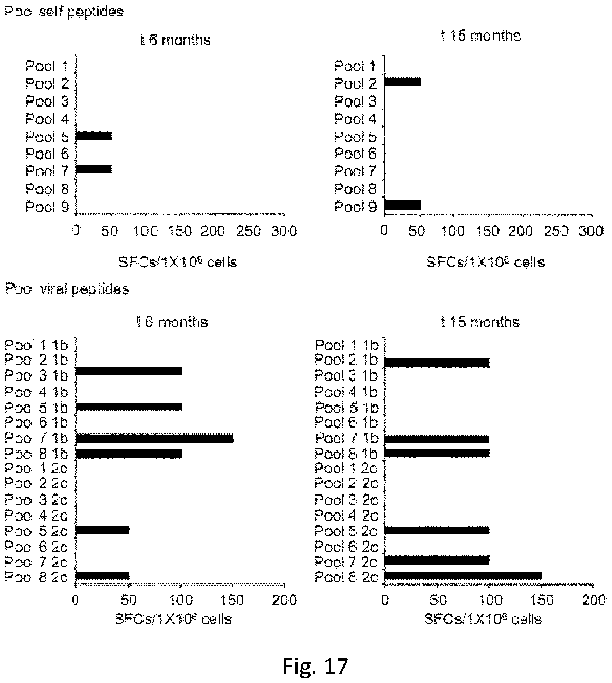

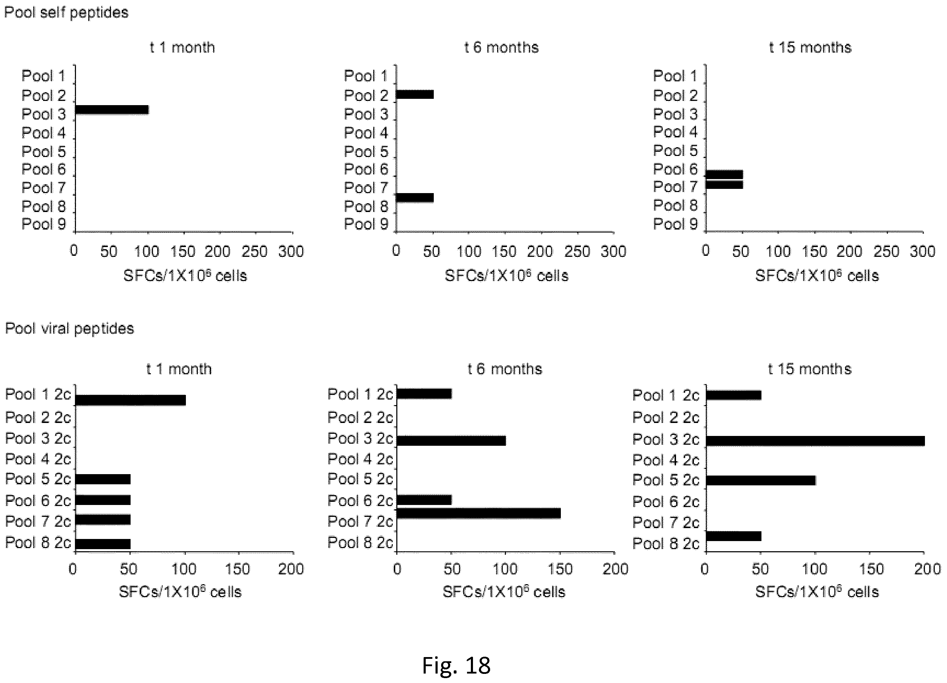

**Figure 2 to 18****: Effector CD8⁺ T cells specific to apoptotic or viral epitopes in patients with acute HCV infection.** Fresh CD8⁺ T cells, purified from PBMCs of patients (Pt.) undergoing chronic (C) or self-limited (S) HCV infection, were able to form IFN-γ spots promptly within 6 h of contact with autologous irradiated CD8-depleted PBMCs, as APCs, plus peptide *ex vivo,* as detected by an ELISPOT assay. Results are expressed as spot-forming cells (SFCs) in 1 x 10⁶ CD8⁺ T cells. The SFC values were subtracted from the background (BG). Because of the limited number of PBMCs obtained from patients, freshly isolated CD8⁺ T cells were tested against 9 pools of apoptotic epitopes (Pool self peptides, see also Supplementary Table 1A-C) and 8 pools of viral peptides (Pool viral peptides, see also Supplementary Table 2A-D). **Fig. 2 to 12** represent responses from PBMCs of patients undergoing chronic HCV infection, **Fig. 13 to 18** represent responses from PBMCs of patients undergoing self-limited HCV infection. Pools of apoptotic self-epitopes or viral epitopes are indicated in **Tables 11-13 and 14-17.**

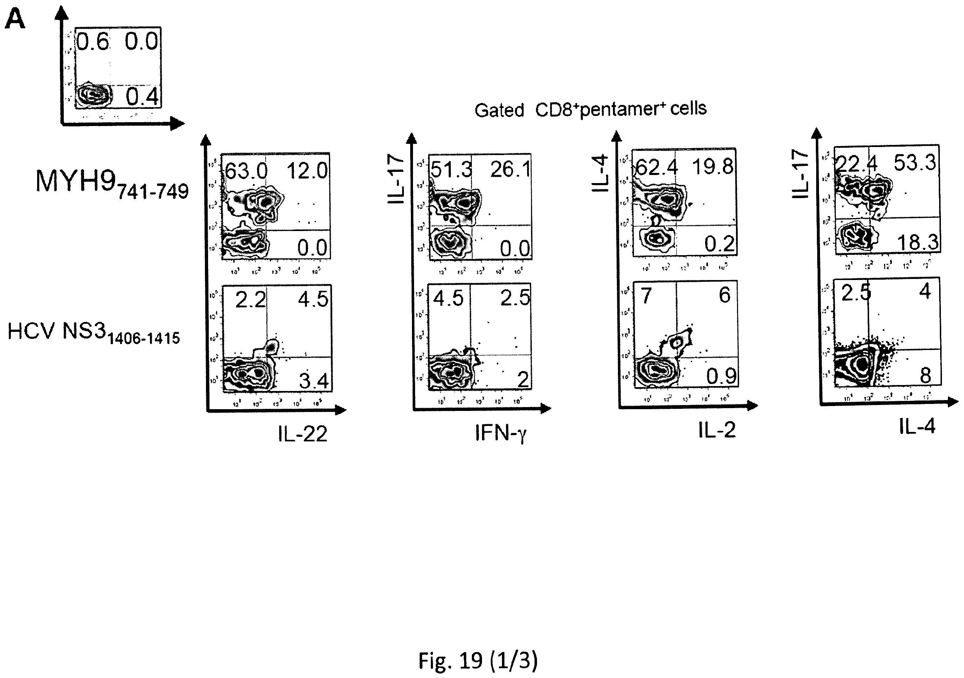

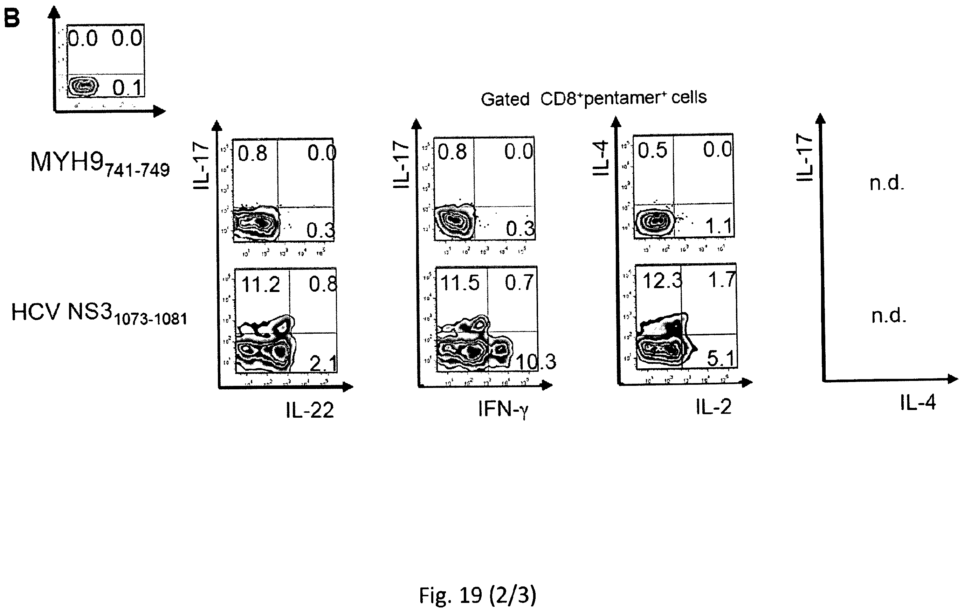

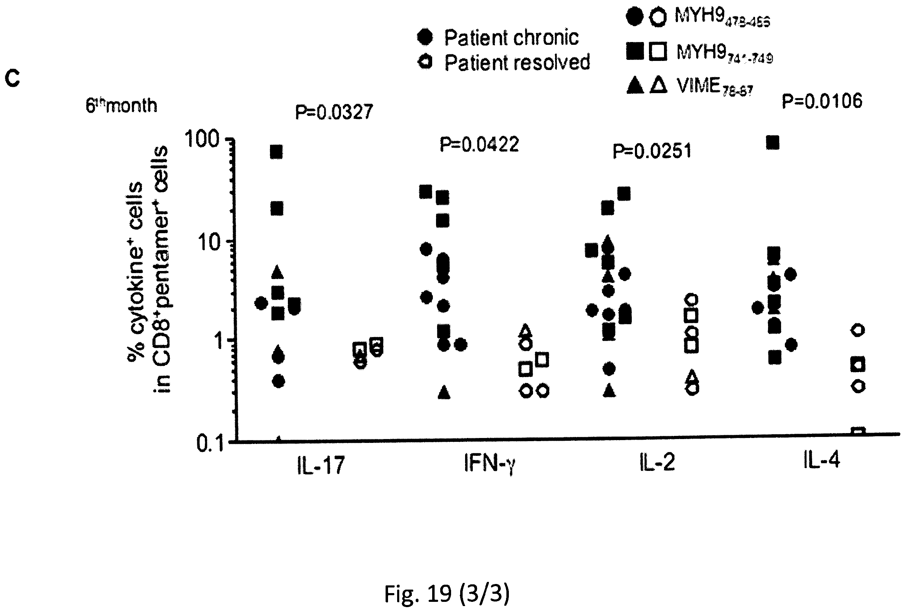

**Figure 19****: Polyfunctional CD8⁺ T_{EM} cells specific to apoptotic epitopes distinguish patients experiencing chronic infection. (A,B)** Representative flow cytometry analyses of PBMCs from patients with acute HCV infection experiencing chronic infection (**A**) or undergoing infection resolution (**B**) that were stained with mAb to CD8 and pentamers complexed to the indicated apoptotic or viral epitopes. Cells were then stimulated with the relevant soluble peptides plus anti-CD28 mAb and processed for the detection of IL-17, IL-22, IFN-γ, IL-4, and IL-2 by ICS assay with the relevant mAbs. Counterplot analyses are gated on CD8⁺pentamer⁺ cells and show percentages of cytokine-producing cells. The percentage of cells is reported in each quadrant. The small histograms show ICS analyses of representative cytokine (IL-17, IL-22, IFN-γ, IL-4, or IL-2) production without antigenic stimulation by gated CD8⁺pentamer⁺ cells. Irrelevant cytokine production (<0.1% cells) was observed when either apoptotic epitope- or viral epitope-specific CD8⁺pentamer⁺ T cells of 20 HLA-A2⁺ healthy individuals were stimulated with this procedure; thus, the author considered 1% cytokine-producing cells in pentamer⁺ cells as the sensitive level of this assay. (**C**) Percentage of cells producing the indicated cytokines in CD8⁺pentamer⁺ cells in response to the indicated apoptotic epitopes (evaluated at the indicated time point by flow cytometry analyses) from patients with acute HCV infection experiencing chronic infection (filled symbols) or undergoing infection resolution (empty symbols). The horizontal dashed line delimits an arbitrary background, which is based on the values of 20 HLA-A2⁺ healthy individuals exhibiting < 0.1% cytokine-producing cells in each test.

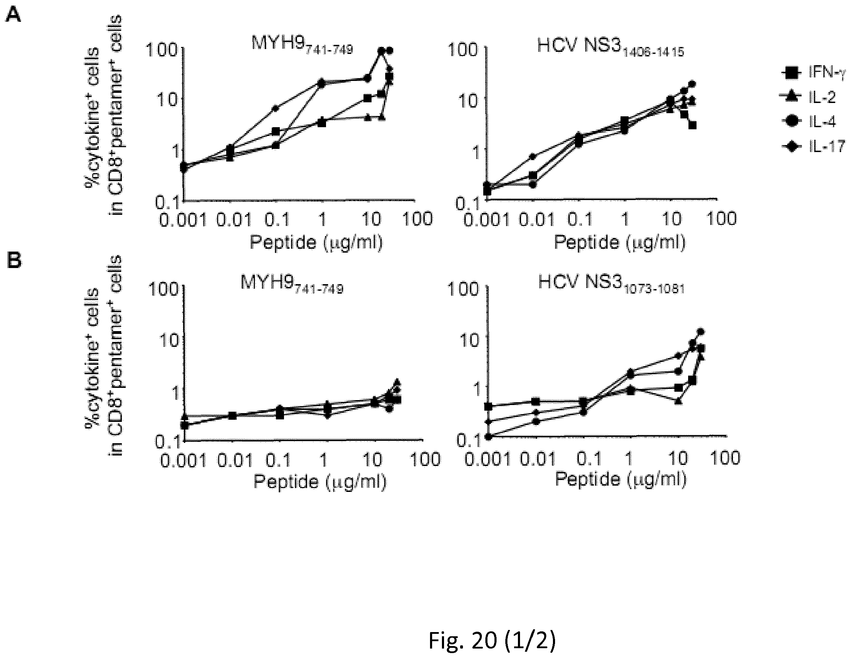

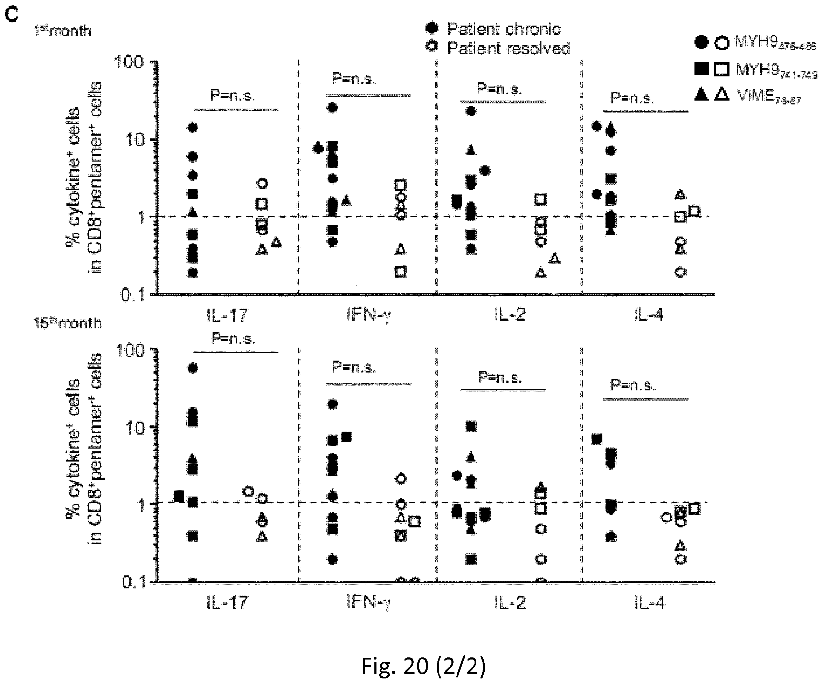

**Figure 20****: Kinetics of cytokine-producing CD8⁺pentamer⁺ cells in patients with acute HCV infection. (A,B)** Peptide dose-response curves of cytokine-producing CD8⁺pentamer⁺ cells from patients with acute HCV infection experiencing chronic infection (**A**) or undergoing infection resolution (**B**). Cells were stained with the pentamers expressing the indicated peptides and stimulated for 6 h with the same soluble peptides. They were then processed for the detection of the different cytokines indicated by ICS assay. Values are shown with the background subtracted. (**C**) Polyfunctional CD8⁺ T_{EM} cells specific to apoptotic epitopes in patients with acute HCV infection. Percentage of cells producing the indicated cytokines in CD8⁺pentamer⁺ cells in response to the indicated apoptotic epitopes (evaluated at the indicated time points by flow cytometry analyses) from patients with acute HCV infection experiencing chronic infection (filled symbols) or undergoing infection resolution (empty symbols). The horizontal dashed line delimits an arbitrary background, which is based on the values of 20 HLA-A2⁺ healthy individuals exhibiting < 0.1% cytokine-producing cells in each test.

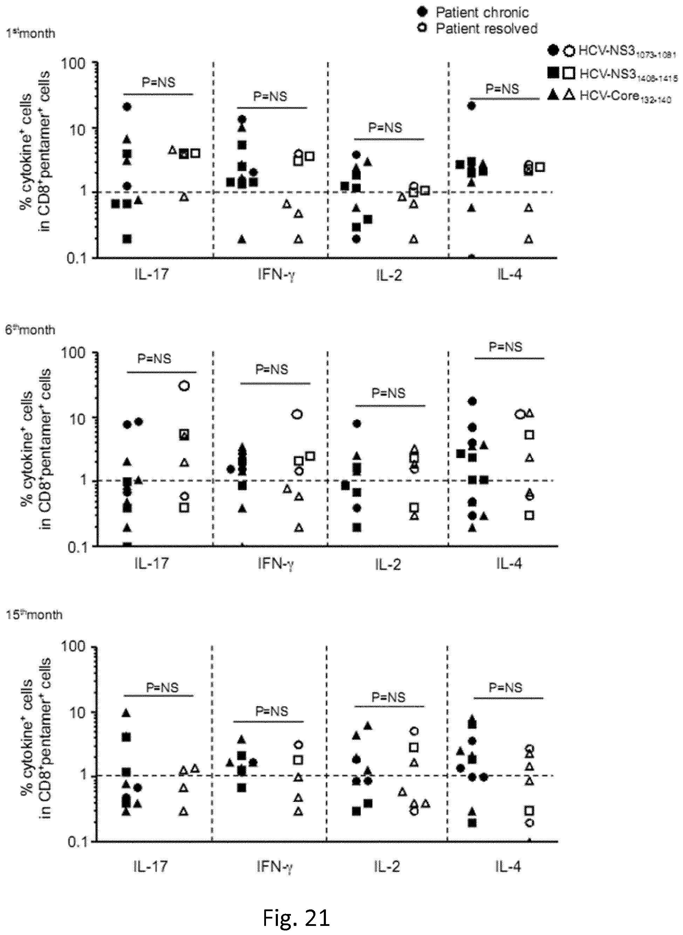

**Figure 21****: Polyfunctional CD8⁺ T_{EM} cells specific to viral epitopes in patients with acute HCV infection.** Percentage of cells producing the indicated cytokines in CD8⁺pentamer⁺ cells in response to the indicated viral epitopes (evaluated at the indicated time points by flow cytometry analyses) from patients with acute HCV infection experiencing chronic infection (filled symbols) or undergoing infection resolution (empty symbols). The horizontal dashed line delimits an arbitrary background, which is based on the values of 20 HLA-A2⁺ healthy individuals exhibiting < 0.1% cytokine-producing cells in each test.

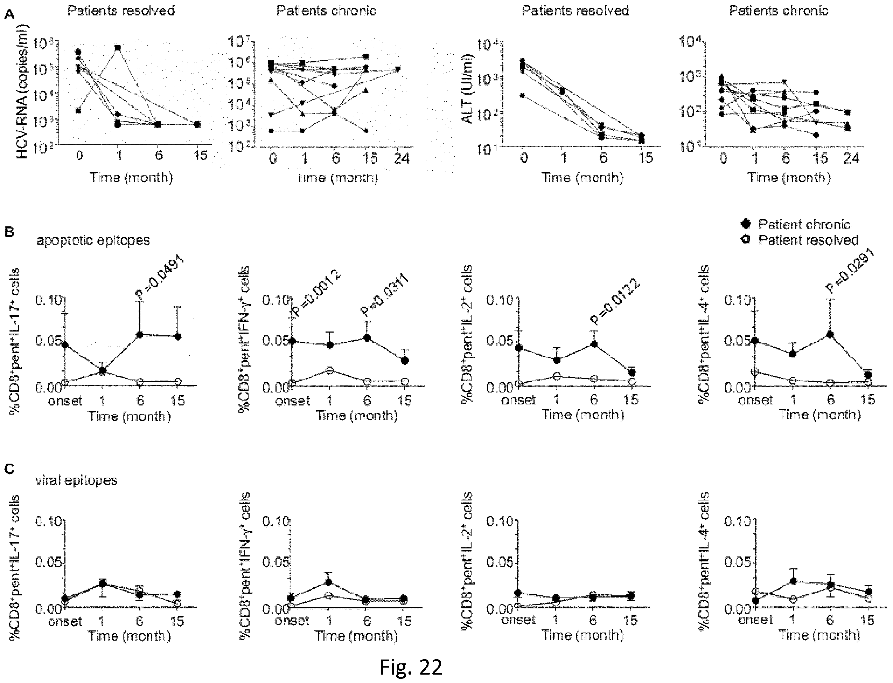

**Figure 22****: Polyfunctional CD8⁺ T_{EM} cells specific to apoptotic epitopes predict the chronic progression of HCV infection.** (**A**) Kinetics of plasma HCV-RNA and alanineaminotransferase (ALT) levels until 15-24 months from clinical onset of infection. Each symbol represents a single patient. (**B,C)** Kinetics of the means of all the CD8⁺pentamer⁺ (pent) cell percentages promptly producing the indicated cytokines in response to the apoptotic (**B**) or viral (**C**) epitopes. The cells were calculated as a percentage of the absolute number of cells expressing CD8, pentamer ligands, and cytokines in the PBMCs. Values are shown with the background subtracted. Filled circles represent patients with acute HCV infection experiencing chronic infection; empty circles represent a HCV-patients undergoing infection resolution.

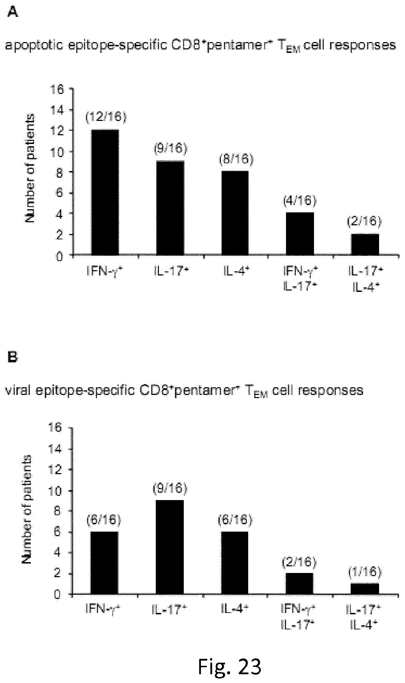

**Figure 23****: (A,B)** Number of patients exhibiting a wide repertoire of polyfunctional CD8⁺ T cells producing one or two cytokines in response to apoptotic (**A**) or viral (**B**) epitopes.

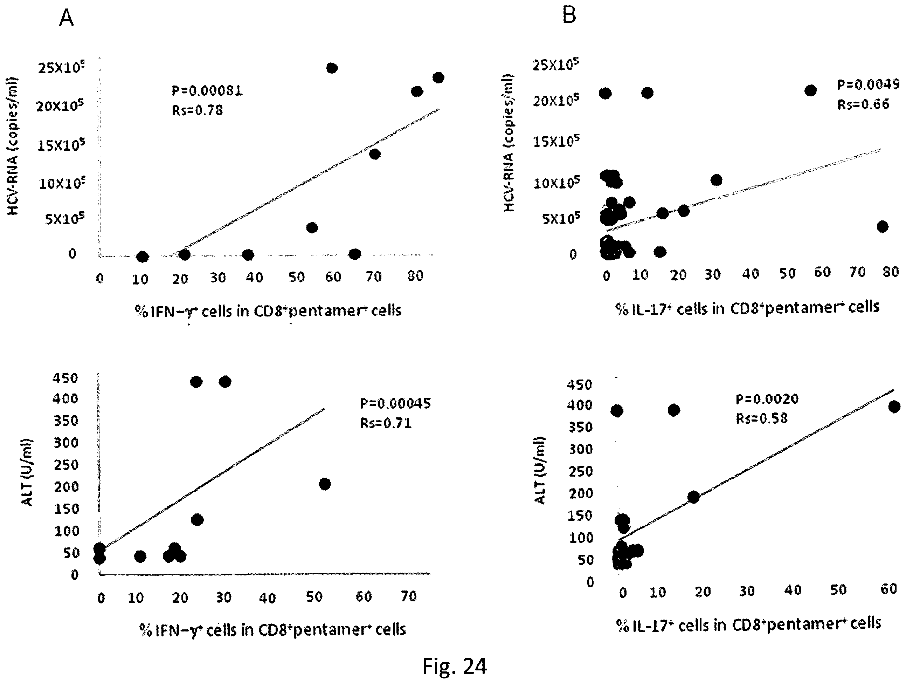

**Figure 24****: (A,B)** Correlation between CD8⁺pentamer⁺ T_{EM} cells producing IFN-γ (**A**) or IL-17 (**B**) in response to apoptotic epitopes and the viral load (HCV-RNA copies) or ALT in patients with acute HCV infection experiencing chronic infection or infection resolution. Statistical analysis was performed using non-parametric Spearman's test.

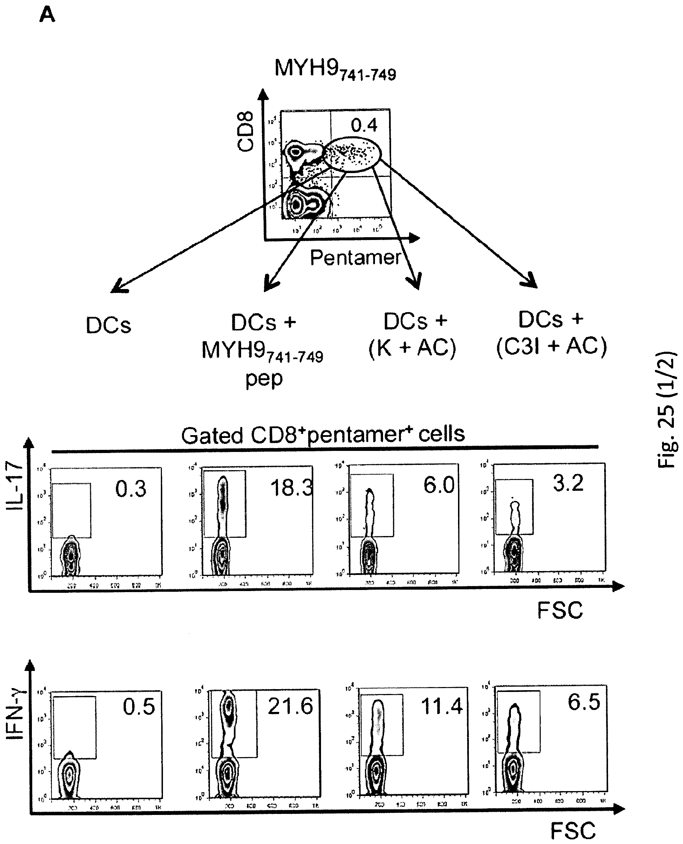

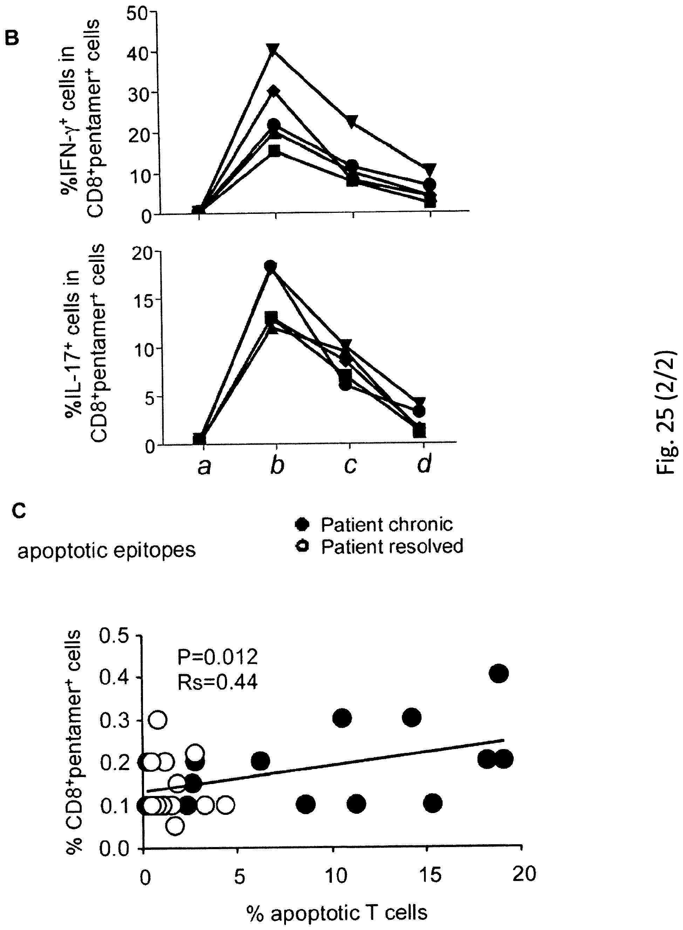

**Figure 25****: CD8⁺ T_{EM} cells specific to apoptotic epitopes are activated by apoptotic epitopes naturally processed and cross-presented by dendritic cells (DCs). (A**) One representative of five experiments in which PBMCs from one patient with acute HCV infection were double-stained with mAb to CD8 and pentamers complexed with the indicated apoptotic epitope, and cultured with autologous DCs that had been pulsed or not with the relevant soluble peptide, apoptotic cloned T cells (ACs = apoptotic cells), ACs previously treated with a negative caspase control (K = Z-FA-FMK), or ACs previously treated with the caspase 3 inhibitor (C3I = Z-DEVD-FMK). After 6 h, CD8⁺pentamer⁺ cells were tested for their capacity to produce the different cytokines indicated by the ICS assay. Counterplots are gated on CD8⁺pentamer⁺ cells and show percentages of the different cytokine-producing cells in each quadrant. (**B**) Cumulative experiments in 5 independent patients, performed as described in (A), showing the percentages of cells producing the indicated cytokines in CD8⁺pentamer⁺ cells in response to the following stimuli: autologous DCs alone (*a*); DCs that had been pulsed with the MYH9₇₄₁₋₇₄₉ peptide *(b);* DCs that had been pulsed with ACs, previously treated with a negative caspase control (Z-FA-FMK) (*c*); or DCs that had been pulsed with ACs previously treated with the caspase 3 inhibitor (Z-DEVD-FMK) (*d*). Each symbol represents a single patient. (**C**) Correlation (analysed at the 15^{th} month after clinical onset) between the percentage of CD8⁺pentamer⁺ cells specific to apoptotic epitopes and the percentage of circulating apoptotic T cells in patients with acute HCV infection experiencing chronic infection (filled circles) or infection resolution (empty circles). Statistical analysis was performed using non-parametric Spearman's test.

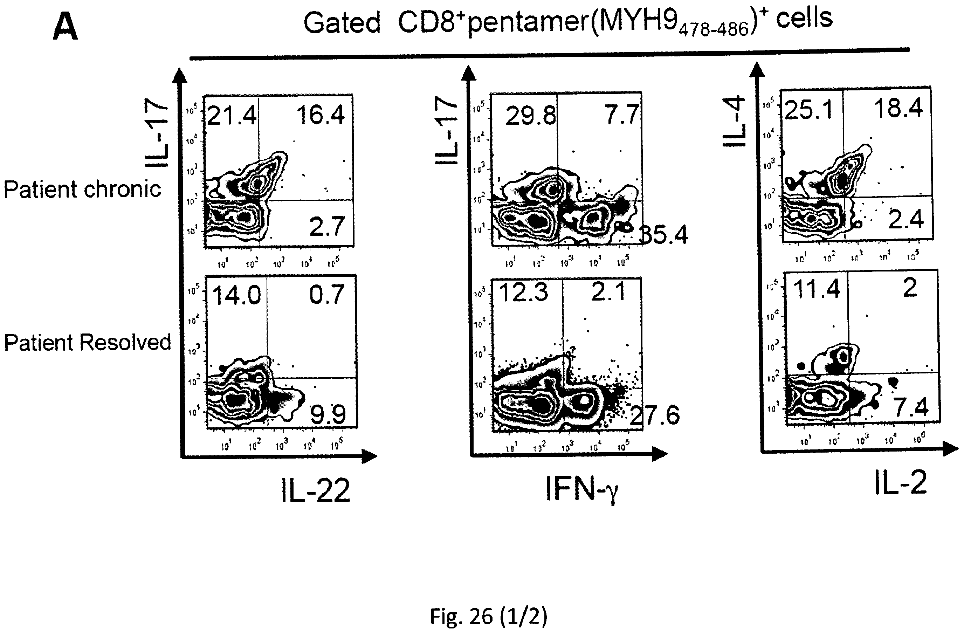

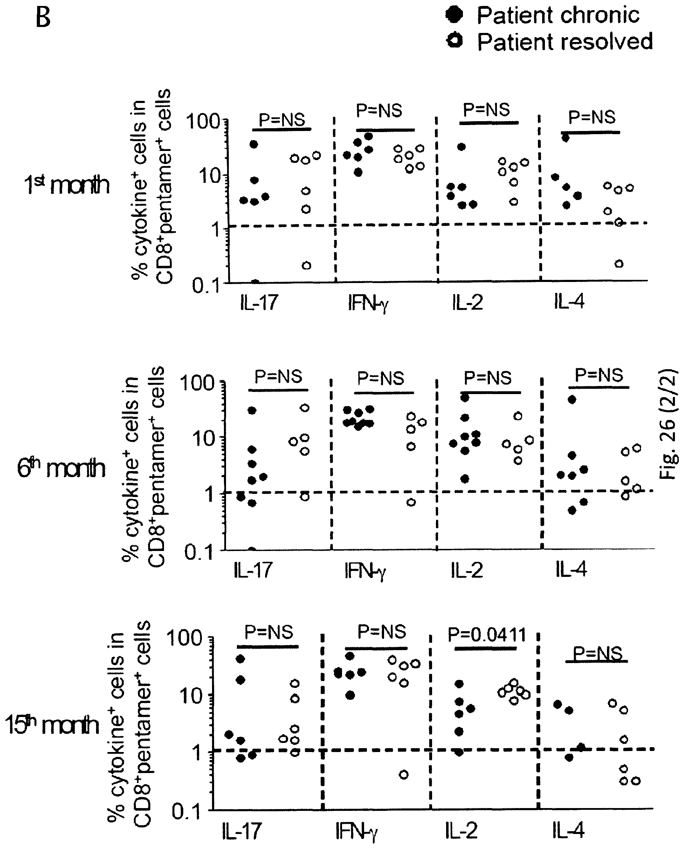

**Figure 26****: No intrinsic defect of effector functions in CD8⁺ T cells from patients with acute HCV infection. (A**) One representative flow cytometry analysis in which PBMCs from patients with acute HCV infection were stained with mAb to CD8 and the indicated pentamer, stimulated with PMA and iono for 6 h, and processed for the detection of the indicated cytokines by ICS assay with the relevant mAbs. Counterplot analyses are gated on CD8⁺pentamer⁺ cells and show percentages of cytokine-producing cells. The percentage of cells is reported in each quadrant. (**B**) Percentage of cells producing the indicated cytokines in CD8⁺pentamer⁺ cells in response to PMA and iono (evaluated at the indicated time points by flow cytometry analyses) from patients with acute HCV infection experiencing chronic infection (filled symbols) or undergoing infection resolution (empty symbols).

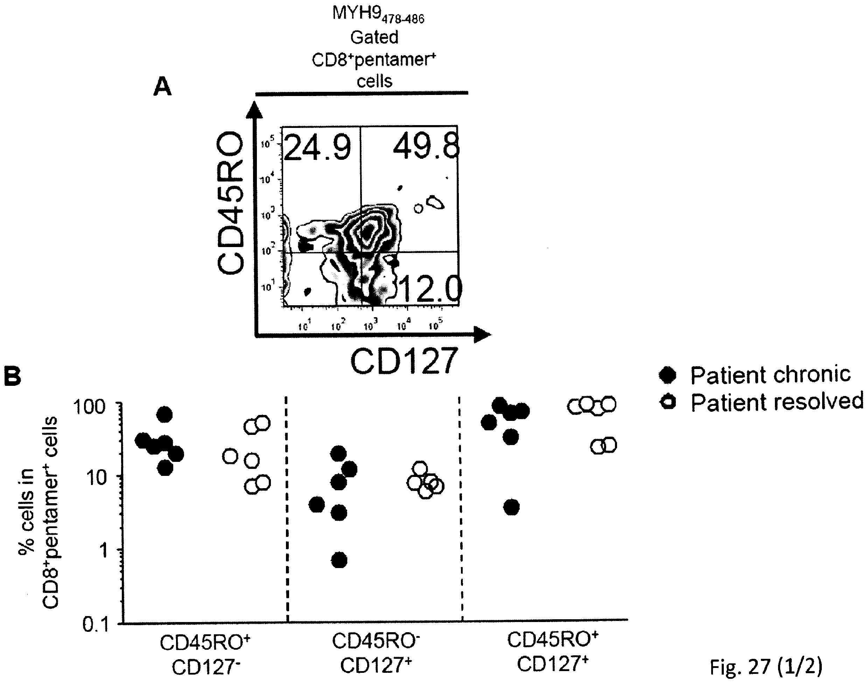

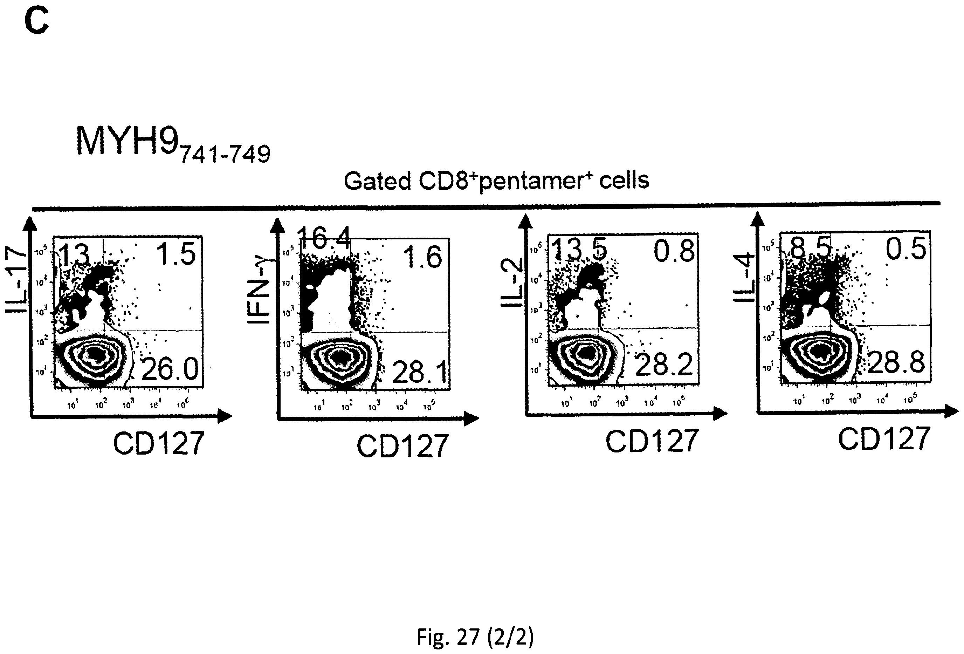

**Figure 27****: naïve, central, and effector memory CD8⁺ T cells specific to apoptotic or viral epitopes. (A**) One representative flow cytometry analysis, the values of the total patients are shown in (**B**), in which PBMCs from a patient with acute HCV infection were stained with mAbs to CD8, CD45RO, and CD127 and with the pentamer complexed with the indicated apoptotic peptide. Counterplot analyses are gated on CD8⁺pentamer cells and show percentages of CD45RO⁺ and/or CD127⁺ cells. The percentage of cells is reported in each quadrant. (**B**) Percentages of CD45RO⁺CD127⁻, CD45RO⁻CD127⁺, or CD45RO⁺CD127⁺ cells in CD8⁺pentamer⁺ cells from HCV patients experiencing chronic infection (filled circles) or infection resolution (empty circles). (**C**) One representative of six flow cytometry analyses in which PBMCs from a patient with acute HCV infection were stained with mAbs to CD8 and CD127 and with the pentamer complexed with the indicated peptide. Cells were stimulated with the same soluble peptide for 6 h and then processed for the detection of the indicated cytokines by ICS assay with the relevant mAbs.

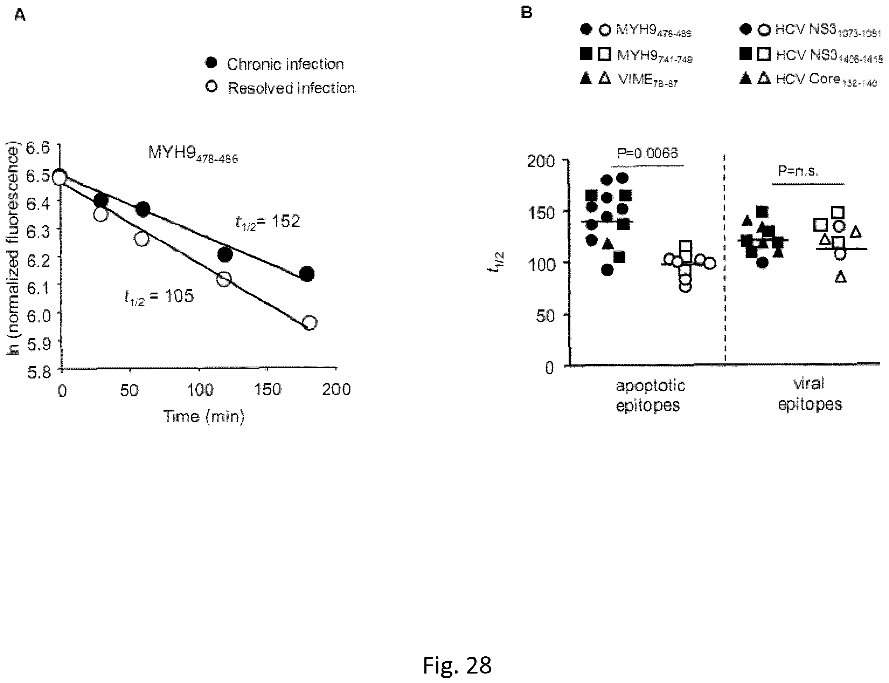

**Figure 28****: Decay kinetics of pentamer staining for CD8⁺ T cells.** (**A**) Representative decay plot of the natural logarithm of the normalized fluorescence versus time after anti-HLA-A2 mAb addition for fresh PBMCs stained with pentamers that were complexed with the indicated apoptotic epitope. The *t*_{1/2} represents the staining half-times for the pentamers to CD8⁺ T cells. Filled or empty circles represent PBMCs from patients with acute HCV infection experiencing chronic infection or undergoing infection resolution, respectively. (**B**) The *t*_{1/2} for the pentamers (complexed with the indicated apoptotic or viral epitopes) binding CD8⁺ T cells from patients with acute HCV infection experiencing chronic infection (filled symbols) or undergoing infection resolution (empty symbols).

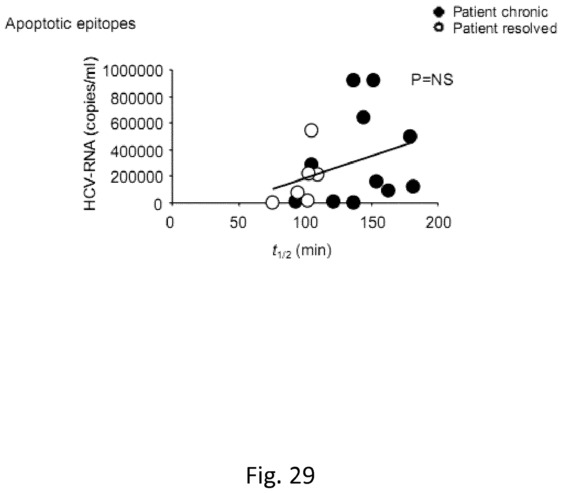

**Figure 29****: Link between viral load and the decay kinetics of pentamer staining for CD8⁺ T cells.** Correlation between plasma HCV-RNA copies and the staining half-times (*t*_{1/2}) for the pentamers to CD8⁺ T cells from patients with acute HCV infection experiencing chronic infection (filled circles) or infection resolution (empty circles). NS = not significant.

### Detailed description of the invention

### MATERIALS AND METHODS

### Study population

The study cohort included 16 patients with acute HCV infection (4 women, 12 men, median age 31 years, range 22-57 years). Diagnosis of acute HCV infection was based on (1) high levels of serum ALT; (2) sero-conversion assessed by third generation enzyme linked immune-sorbent assay, or anti-HCV positivity at the time of the diagnosis with an anti-HCV negative test in the previous 12 months; (3) presence of HCV-RNA in at least the first serum sample, and (4) sudden onset of liver disease symptoms. Alternative causes of acute hepatitis, such as other viral infection, autoimmunity, alcohol, drugs, and toxins were excluded. Patients with concomitant immunological disorders or with HIV co-infection were also excluded from the study.

### Cell preparations

PBMCs were isolated and T cell clones were generated as previously described (48). CD8⁺ T cells were purified from PBMCs by positive selection coupled to magnetic beads (MiltenyiBiotec) as previously described (38). Flow cytometry analysis demonstrated >99% CD8⁺ cells in the positively purified population and <5% in the CD8-depleted population. To purify antigen-specific type-17 CD8⁺ cells, PBMCs were stimulated with the relevant peptide plus anti-CD28 (4 µg/ml) (BD Pharmingen). Then, cells were stained with allophycocyanin (APC)-labeled anti-CCR6 (R&D System) and PE-cyanine 7 (PE-Cy7)-labeled anti-CCR4 (BD Pharmingen) and processed with FACSAria (Becton Dickinson) to sort CCR6⁺CCR4⁺ cells: >98% of these cells both produced IL-17 in response to the relevant peptide and were susceptible of staining with the relevant pentamers, as detected below. Spontaneous apoptosis of PBMCs from patients was determined by staining with Annexin-V (ApoAlert Apoptosis Kit, Clontech Laboratories Inc), propidium iodide (PI) (Sigma-Aldrich) and PE-Cy7-labeled anti-CD3 (BD Pharmingen) before and after 18 hours incubation at 37°C. Immature (i)DCs were derived from peripheral monocytes that had been purified by positive selection with anti CD14 mAb coupled to magnetic beads (MiltenyiBiotec). CD14⁺ cells were incubated for 5 days in RPMI 1640 medium containing 5% FCS, 2 mM glutamine, 1% nonessential amino acids, 1% sodium pyruvate, 50 µg/ml kanamycin (Gibco BRL), 50 ng/ml rGM-CFS (Novartis Pharma), and 1000 U/ml rIL-4 (gently provided by A. Lanzavecchia, Bellinzona, CH). Mature DCs were obtained by a 40-h stimulation of iDCs with soluble rCD40L molecules (Alexis Biochemicals, Alexis Corporation). The definition of monocyte-derived DCs was based on their surface phenotype profile by staining with anti CD14, anti-CD86 (Caltag Laboratories), anti CD1a, anti-CD1c, anti-CD11c, anti-CD32, anti-CD80 (BD PharMingen) mAbs, Annexin-V (ApoAlert Apoptosis Kit, Clontech Laboratories Inc), propidium iodide (PI) (Sigma-Aldrich), and the appropriate secondary labeled antibodies (BD PharMingen).

### ELISPOT assay ex vivo

Highly purified CD8⁺ T cells (1 x 10⁵) from PBMCs were stimulated for 4-6 h with eight independent pools of viral-peptides (genotype 1b) and eight independent pools of viral-peptides (genotype 2c) (see Supplemental Table 1A-C, and Supplemental Table 2A-D below) (5 µg/ml per single peptide) and irradiated autologous CD8-depleted PBMCs, used as APCs, and tested by an ELISPOT assay, as described (14).

### Pentamer staining

PBMCs were incubated with APC-labeled-HLA-A*0201 pentamers (complexed to vimentin₇₈₋₈₇ [LLQDSVDFSL], non-muscle myosin₄₇₈₋₄₈₆ [QLFNHTMFI], or nonmuscle myosin₇₄₁₋₇₄₉ [VLMIKALEL] peptide) for apoptotic epitopes and APC-labeled-HLA-A*0201 pentamers (complexed to HCV-NS3₁₀₇₃₋₁₀₈₁ [CINGVCWTV], HCV-NS3₁₄₀₆₋₁₄₁₅ [KLVALGINAV] or HCV-Core₁₃₂₋₁₄₀ [DLMGYIPAV] peptide) for viral epitopes (ProImmune Limited, Oxford, United Kingdom), in FACS buffer (PBS 1×, 2% human AB serum) for 10 min at 37°C, followed by washing and further incubation with APC-Cy7-labeled mAb to CD8 (BD Pharmingen, San Diego, CA), FITC-labeled anti-PD-1 (BD Pharmingen), PE-labeled anti-CD127 (BD Pharmingen), FITC-labeled anti-CD45RO (Caltag Laboratories, Burlingame, CA) for 20 min at 4°C. Negative controls were obtained by staining cells with an irrelevant isotype-matched mAb. Cells were washed, acquired with a FACSCanto flow cytometer and FACSDiva analysis software (Becton Dickinson) or FlowJo software version 7.5.5 (Tree star, Inc. San Carlos, CA).

### Intracellular cytokine staining

PBMCs were stained with pentamers and mAb to CD8, and then stimulated with or without different concentrations of the corresponding uncomplexed peptide (ProImmune Limited) plus anti-CD28 mAb (4 µg/ml) (BD Pharmingen), or with PMA (50ng/ml) plus ionomycin (1 µg/ml) (Sigma Aldrich, Milan, Italy), for 6 h at 37°C. At the 2nd h, 10 µg/ml Brefeldin A (Sigma-Aldrich) was added. Cells were washed, fixed and permeabilized using Cytofix/Cytoperm solution (BD Pharmingen) at 4°C for 20 min, re-washed with Perm Wash Buffer (BD Pharmingen), and intracellularly stained with different combinations of Alexa Fluor 488-labeled anti-IL17A (eBioscience San Diego, CA), PE- labeled anti-IFN-γ, PE-labeled anti-IL-2, FITC-labeled anti-IL-4 (BD Pharmingen), PE-labeled anti-RORC (eBioscience) or purified anti-T-bet (Santa Cruz Biotechnology Santa Cruz, California) for 30 min at 4°C. When stained with unlabeled specific antibody to detect T-bet, cells were washed and stained with the appropriate secondary FITC-labeled antibody. Cells were washed, acquired with a FACSCanto flow cytometer and FACSDiva analysis software (Becton Dickinson) or FlowJo software (Tree star).

### Cross-presentation of apoptotic cells

Cloned CD8⁺CD95⁺ T cells (10-100 x 10⁶) were incubated in the presence or absence of 14 mg/ml C3I (Z-DEVD-FMK), or a negative caspase control (K, Z-FA-FMK) (BD Pharmingen) for 1 h at 37°C in a 24-well plate. Then, cells were induced to apoptosis by incubation with 500 ng/ml anti-Fas (anti-CD95 mAb [clone CH11], Upstate Biotechnology) for at least 6 h. Apoptotic cells were determined by staining with Annexin- V (ApoAlert Apoptosis Kit, Clontech Laboratories Inc), PI (Sigma-Aldrich) and flow-cytometry analysis. PBMCs were double-stained with pentamers and mAb to CD8 and cultured with iDCs that had been pulsed or not with apoptotic cloned T cells. After 6-8 h, cells were tested for their capacity to produce IL-17 and IFN-γ by intracellular cytokine staining (ICS) as described above. Cells were washed, acquired with a FACSCanto flow cytometer and analyzed with FACSDiva analysis software (Becton Dickinson) or FlowJo software (Treestar).

### Pentamer staining decay (dissociation kinetics)

PBMCs were stained with saturating amounts of APC-labeled-HLA-A*0201 pentamers and APC-Cy7-labeled-CD8 (BD Pharmingen) for 45 min at room temperature (28). Then, cells were washed three times with buffer (2% FCS, 0.01 sodium azide in PBS) and resuspended in 500 µl of buffer with saturanting amounts of mAb to HLA-A2 (BB7.2, ATCC). At various time points (0, 30', 1h, 2h and 3h), an aliquot cells was washed and the fluorescence intesity was determined by flow cytometry analysis. Double staining using an anti-human TCRα/β (BD Pharmingen) and pentamers was performed in parallel to normalize pentamer fluorescence against the expressed TCR. The values were then normalized to percent of the total fluorescence at the initial time point and plotted on a logarithmic scale. *t*_{1/2} are determined by calculating the (ln2)/mean slope value of plots of the natural logarithm (ln) of the pentamer fluorescence normalized for the TCR fluorescence. The slope is equivalent to ln(Fa/Fb)/t, where Fa is the normalized fluorescence at the start of the interval, Fb is the normalized fluorescence at the end of the interval, and t is the length of the interval (minutes).

### Blockade of PD-1/PD-L1 interaction

PBMCs were incubated for 10 days a 37°C with specific peptides (apoptotic peptides or viral peptides) and anti-PD-L1 (10 µg/ml) (R&D systems Minneapolis, MN) or isotype control antibody (10 µg/ml). Recombinant IL-2 (50 U/ml) (CHIRON Emeryville, California) was added on day 4 of culture. On day 10, cells were stained with surface antibodies, pentamers, anti-IL-17A, anti-IFN-γ, anti-IL-2 and anti-IL-4 mAbs. Cells were washed, acquired with a FACSCanto flow cytometer and analyzed with FACSDiva analysis software (Becton Dickinson) or FlowJo software (Tree star).

### T cell polarization

PBMCs were incubated for 10 days at 37°C with specific peptides (apoptotic or viral peptides), human rIL-6 (50 ng/ml), rIL-1β (10 ng/ml), rIL-23 (50 ng/ml) and rTGF-β (10 ng/ml) (R&D Systems) for the Th17 cell polarization. For the Th1 cell polarizing condition, PBMCs were antigen-stimulated in the presence of recombinant human rIL-12 (10 ng/ml) and rIFN-γ (100 U/ml) (R&D Systems). Recombinant IL-2 was added on day 4 of culture (50 U/ml). On day 10, cells were stained with surface antibodies, pentamers, anti IL-17A, anti-IFN-γ, anti-IL-2 and anti-IL-4 mAbs. Cells were washed, acquired with a FACSCanto flow cytometer and analyzed with FACSDiva analysis software (Becton Dickinson) or FlowJo software (Tree star).

### Statistical analysis

All statistical analyses were performed with Prism 4 (GraphPad) software using nonparametric Spearman's correlation test, nonparametric Mann-Whitney U-test for unpaired data and Wilcoxon test for paired data. The differences were considered significant at P<0.05.

### RESULTS

### Proteomic analysis of apoptotic or live cells

As reported in (14), the author identified proteins differentially expressed in apoptotic cells. The results are reported in Table 1 below.

**Table 1 Relative abundance of spots identified in apoptotic or control T cell clones by 2DE and MALDI-TOF analyses**

| **Spot no.2** | **AN** | **ID** | **Description** | **Relative abundance** | | **M/pl Theoretical Found** | | **Matching peptides** | **Percentage sequence coverage (AA)** | **Note** |
|---|---|---|---|---|---|---|---|---|---|---|
| | | | | **CCs** | **ACs** | | | | | |
| 23 | P08670 | VIME | Vimentin | ++ | + | 53/5 | 53/4.8 | 25 | 60(33-465) | Whole |
| 3 | P08670 | VIME | Vimentin | - | + | 53/5 | 25/4.5 | 13 | 39(196-423) | Fragment |
| 4 | P08670 | VIME | Vimentin | - | + | 53/5 | 40/4.5 | 9 | 22(100-389) | Fragment |
| 6 | P08670 | VIME | Vimentin | - | + | 53/5 | 30/5.2 | 7 | 20(13-183) | Fragment |
| 13 | P08670 | VIME | Vimentin | - | + | 53/5 | 20/4.5 | 6 | 14(100-183) | Fragment |
| 1 | P35579 | MYH9 | Nonmuscle myosin | + | ++ | 226/5.5 | 90/5.2 | 40 | 6(126-1930) | Fragment |
| 19 | P20700 | LAM1 | Lamin B1 | ++ | + | 67/5.1 | 70/5 | 8 | 15(79-386) | Whole |
| 2 | P20700 | LAM1 | Lamin B1 | - | + | 67/5.1 | 30/4.7 | 13 | 21(14-220) | Fragment |
| 17 | P52566 | GDIS | Rho GDP dissociation inhibitor 2 | ++ | + | 23/5.1 | 23/4.8 | 8 | 49(22-196) | Whole |
| 8 | P52566 | GDIS | Rho GDP dissociation inhibitor 2 | - | + | 23/5.1 | 21/6.6 | 12 | 52(22-164) | Fragment |
| 9 | P52566 | GDIS | Rho GDP dissociation inhibitor 2 | - | + | 23/5.1 | 21/6.9 | 5 | 30(34-131) | Fragment |
| 5 | Q07244 | ROK | Heterogeneous nuclear ribonucleoprotein K | + | ++ | 51/5.4 | 45/6.2 | 15 | 24(22-326) | Fragment |
| 30 | Q07244 | ROK | Heterogeneous nuclear ribonucleoprotein K | ++ | + | 51/5.4 | 65/5 | 5 | 15(70-33) | Whole |
| 7 | P02570 | ACTB | Actin cytoplasmatic 1 | - | + | 42/5.3 | 30/5.1 | 8 | 23(12-278) | Fragment |
| 10 | P02570 | ACTB | Actin cytoplasmatic 1 | - | + | 42/5.3 | 12/5.8 | 5 | 17(12-106) | Fragment |
| 16 | P02570 | ACTB | Actin cytoplasmatic 1 | - | + | 42/5.3 | 40/5 | 8 | 28(19-312) | Fragment |
| 27 | P02570 | ACTB | Actin cytoplasmatic 1 | + | ++ | 42/5.3 | 32/4.7 | 4 | 14(29-206) | Fragment |
| 18 | P18669 | PMG1 | Phosphoglycerate mutase 1 | ++ | + | 29/6.7 | 28/7 | 4 | 24(90-239) | Whole |
| 20 | Q9NYL9 | TMO3 | Ubiquitous tropomodulin | ++ | + | 40/5.1 | 40/5 | 6 | 29(12328) | Whole |
| 21 | Q9Y3F4 | UNRI | UNR interacting protein | ++ | + | 39/4.9 | 38/4.7 | 4 | 18(45-290) | Whole |
| 24 | P25786 | PSA1 | Proteasome subunit a type 1 | + | ++ | 30/6.5 | 30/6.2 | 7 | 31(1-214) | Whole |
| 25 | P05387 | RLA2 | 60S acidic ribosomal protein P2 | + | ++ | 12/4.4 | 12/4.3 | 6 | 72(1-94) | Whole |
| 28 | P08758 | ANX5 | annexin V | ++ | + | 36/4.9 | 36/4.6 | 7 | 22(6-259) | Whole |
| 33 | P31146 | CO1A | Coronin like protein p57 | ++ | + | 52/6.2 | 70/6.7 | 4 | 9(11-393) | Whole |
| 34 | P78371 | TCBP | Tcomplex protein 1, b subunit | ++ | + | 58/6 | 60/6.5 | 7 | 24(26-516) | Whole |
| 35 | n.i. | | | ++ | + | | 36/5.7 | | | |
| 36 | n.i. | | | ++ | + | | 38/5.6 | | | |
| 37 | n.i. | | | ++ | + | | 39/5.5 | | | |
| 38 | n.i. | | | ++ | + | | 37/5.4 | | | |
| 39 | n.i. | | | ++ | + | | 55/5.6 | | | |
| 40 | n.i. | | | ++ | + | | 55/5.9 | | | |
| 41 | n.i. | | | ++ | + | | 65/6 | | | |
| 42 | n.i. | | | ++ | + | | 43/6.7 | | | |
| 43 | n.i. | | | ++ | + | | 65/7 | | | |
| 44 | n.i. | | | ++ | + | | 90/6.9 | | | |
| 45 | n.i. | | | ++ | + | | 29/6.4 | | | |
| 11 | n.i. | | | - | + | | 12/5.9 | | | |
| 12 | n.i. | | | - | ++ | | 20/4.5 | | | |
| 14 | n.i. | | | + | + | | 10/4.4 | | | |
| 15 | n.i. | | | - | + | | 15/3.5 | | | |
| 31 | n.i. | | | ++ | + | | 80/4.5 | | | |
| 32 | n.i. | | | ++ | + | | 55/5 | | | |
| 22 | n.i. | | | ++ | + | | 15/4.6 | | | |
| 26 | n.i. | | | + | ++ | | 36/6.7 | | | |
| 29 | n.i. | | | ++ | + | | 17/5.4 | | | |

| | | | | | | | | | | |
|---|---|---|---|---|---|---|---|---|---|---|
| *Spot number corresponding to those shown m 2DE analysis in Figure1; n.i., spot not identified; -, spot absent; +, spot present; ++, spot whose relative abundance is at least twice than in the corresponding + spot; AN, access nuntber in Swiss-Prot database; ID, identity; CCs, control cells; ACs, apoptotic cells; Mr, relative molecular mass; AA, amino acids; RI, isoelectric point; | | | | | | | | | | |

### HLA-A2 binding affinity of synthetic peptides derived from fragmented proteins

The author tested the HLA-A2 binding affinity of 9 to 10 amino acid long fragments of the proteins identified and reported in Table 1. The results are reported in Tables 2-9.

**Table 2. HLA-A2 binding affinity of synthetic peptides derived from fragmented proteins.**

| | | | | | | **Binding capacity (IC50§ nM)** | | | | | **Alleles bound** |
|---|---|---|---|---|---|---|---|---|---|---|---|
| **SEQ ID No.** | **Organism** | **Protein** | **1^{th} Pos°** | **Sequence** | **Length** | **A*0201** | **A*0202** | **A*0203** | **A*0206** | **A*6802** | |
| 1 | Human | ACTB" | 131 | AMYVAIQAV | 9 | 2,8 | 1,5 | 1,6 | 7.1 | 190 | 5 |
| 2 | Human | ACTB | 319 | ALAPSTMKI | 9 | 4,3 | 2,9 | 2,5 | 40 | 5,5 | 5 |
| 3 | Human | ACTB | 170 | ALPHAILRL | 9 | 128 | 98 | 7,9 | 186 | 34091 | 4 |
| 4 | Human | ACTB | 266 | FLGMESCGI | 9 | 2,7 | 71 | 11 | 16 | 3979 | 4 |
| 5 | Human | ACTB | 312 | RMQKEITAL | 9 | 14 | 0,29 | 6,5 | 28 | 6667 | 4 |
| 6 | Human | ACTB | 66 | TLKYPIEHGI | 10 | 369 | 202 | 138 | 26442 | 290 | 4 |
| 7 | Human | ACTB | 131 | AMYVAIQAVL | 10 | 351 | 21 | 12195 | 363 | 234 | 4 |
| 8 | Human | ACTB | 348 | SLSTFQQMWI | 10 | 5,6 | 4,5 | 7,7 | 791 | 299 | 4 |
| 9 | Human | ACTB | 46 | GMGQKDSYV | 9 | 196 | 17 | 156 | 13541 | 17632 | 3 |
| 10 | Human | ACTB | 241 | ELPDGQVITI | 10 | 974 | 140 | 1953 | 3728 | 100 | 2 |
| 11 | Human | ACTB | 184 | DLTDYLMKI | 9 | 2029 | 218 | 1542 | 2065 | 4464 | 1 |
| 12 | Human | ACTB | 177 | RLDLAGRDL | 9 | 838 | 2263 | 5373 | - | - | 0 |
| 13 | Human | ACTB | 184 | DLTDYLMKIL | 10 | 1031 | 5668 | 10229 | 1980 | 8682 | 0 |
| 14 | Human | ROK^ | 154 | SIAGGIIGV | 9 | 0,56 | 1,0 | 1,1 | 0,14 | 49 | 5 |
| 15 | Human | ROK | 41 | EMVELRILL | 9 | 92 | 211 | 870 | 152 | 80 | 4 |
| 16 | Human | ROK | 67 | ALRTDYNASV | 10 | 8,9 | 5,0 | 11 | 349 | 1354 | 4 |
| 17 | Human | ROK | 100 | ILKKIIPTL | 9 | 121 | 15 | 5,8 | 914 | 24029 | 3 |
| 18 | Human | ROK | 193 | VLIGGKPDRV | 10 | 14 | 8,5 | 12 | 1282 | 7417 | 3 |
| 19 | Human | ROK | 209 | ILDLISESPI | 10 | 360 | 65 | 1272 | 2598 | 3083 | 2 |
| 20 | Human | ROK | 122 | QLPLESDAV | 9 | 246 | 601 | 1270 | 925 | 1999 | 1 |
| 21 | Human | ROK | 48 | LLQSKNAGAV | 10 | 5026 | 534 | 31 | 11017 | - | 1 |
| 22 | Human | ROK | 146 | ELRLLIHQSL | 10 | 1506 | 135 | 1581 | 1191 | 1130 | 1 |
| 23 | Human | ROK | 426 | PIEGSEDRI | 9 | 16751 | 3509 | 26921 | - | 15762 | 0 |
| 24 | Human | ROK | 124 | PLESDAVECL | 10 | - | 4230 | 11892 | - | - | 0 |
| 25 | Human | ROK | 169 | ELRENTQTTI | 10 | 3392 | 2513 | 1828 | - | 2532 | 0 |
| 26 | Human | ROK | 426 | PLEGSEDRII | 10 | - | - | - | - | - | 0 |
| 27 | Human | LAM1* | 496 | TIWAANAGV | 9 | 8,9 | 263 | 308 | 22 | 20 | 5 |
| 28 | Human | LAM1 | 41 | RLAVYIDKV | 9 | 0,16 | 0,84 | 0,18 | 14 | 1004 | 4 |
| 29 | Human | LAM1 | 301 | SLSSQLSNL | 9 | 1,6 | 0,57 | 2,4 | 104 | 1377 | 4 |
| 30 | Human | LAM1 | 361 | QLLDVKLAL | 9 | 0,060 | 98 | 288 | 4.0 | 5848 | 4 |
| 31 | Human | LAM1 | 182 | QLADETLLKV | 10 | 39 | 14 | 82 | 61 | 14354 | 4 |
| 32 | Human | LAM1 | 133 | KLREYEAAL | 9 | 80 | 28 | 22 | 4326 | 33708 | 3 |
| 33 | Human | LAM1 | 140 | ALNSKDAAL | 9 | 34 | 1,9 | 5,9 | 3625 | - | 3 |

| | | | | | | | | | | | |
|---|---|---|---|---|---|---|---|---|---|---|---|
| #=identity, "=actin cytoplasmic I; ^=eterogeneous nuclear ribonucleoprotein K; *=lamin B1°=1st amino acid position; §=IC50 indicates binding affinity and it is expressed as 50% inhibitory nanomolar concentration, a dash ("-") indicates an IC50 >50000 nM; peptides with IC50 <50nM are classified as high affinity peptides, peptides with IC50>50 and <500nM | | | | | | | | | | | |

**Table 3. HLA-A2 binding affinity of synthetic peptides derived from fragmented proteins.**

| | | | | | | **Binding capacity (IC50§ nM)** | | | | | **Alleles bound** |
|---|---|---|---|---|---|---|---|---|---|---|---|
| **SEQ ID No.** | **Organism** | **Protein** | **1^{th} Pos°** | **Sequence** | **Length** | **A*0201** | **A*0202** | **A*0203** | **A*0206** | **A*6802** | |
| 34 | Human | LAM1* | 291 | ELMESRMRI | 9 | 246 | 1226 | 284 | 25188 | 45 | 3 |
| 35 | Human | LAM1 | 355 | QLNDYEQLL | 9 | 2,0 | 0,23 | 18 | 1493 | 4808 | 3 |
| 36 | Human | LAM1 | 388 | KLSPSPSSRV | 10 | 50 | 18 | 45 | 563 | 20979 | 3 |
| 37 | Human | LAM1 | 488 | VLKAGQTVTI | 10 | 263 | 37 | 23 | 4060 | 15429 | 3 |
| 38 | Human | LAM1 | 37 | ELNDRLAVYI | 10 | 1161 | 134 | 1224 | 30389 | 61 | 2 |
| 39 | Human | LAM1 | 60 | QLQYIEREEV | 10 | 16688 | 1297 | 197 | 237 | - | 2 |
| 40 | Human | LAM1 | 378 | KLLEGEEERL | 10 | 30 | 29 | 3395 | 1944 | 20515 | 2 |
| 41 | Human | LAM1 | 529 | ILKNSQGEEV | 10 | 1647 | 58 | 371 | - | 2705 | 2 |
| 42 | Human | LAM1 | 51 | SLETENSAL | 9 | 695 | 124 | 5974 | 8911 | 30303 | 1 |
| 43 | Human | LAM1 | 84 | ELADARRAL | 9 | - | 1902 | 7437 | - | 97 | 1 |
| 44 | Human | LAM1 | 157 | SLEGDLEDL | 9 | 1068 | 202 | 6246 | 3738 | - | 1 |
| 45 | Human | LAM1 | 164 | DLKDQIAQL | 9 | 15715 | 2616 | 114 | - | 991 | 1 |
| 46 | Human | LAM1 | 175 | SLAAAKKQL | 9 | 23555 | 172 | 612 | 23009 | 36585 | 1 |
| 47 | Human | LAM1 | 379 | LLEGEEERL | 9 | 537 | 61 | 616 | 20505 | - | 1 |
| 48 | Human | LAM1 | 20 | PLSPTRLSRL | 10 | 4761 | 227 | 3342 | - | 22244 | 1 |
| 49 | Human | LAM1 | 76 | GLKALYETEL | 10 | 1582 | 15 | 785 | - | - | 1 |
| 50 | Human | LAM1 | 34 | ELRELNDRL | 9 | 2510 | 571 | 22553 | 37575 | 2837 | 0 |
| 51 | Human | LAM1 | 126 | DLNGAQIKL | 9 | - | 2616 | - | - | 3571 | 0 |
| 52 | Human | LAM1 | 161 | DLEDLKDQI | 9 | 24324 | 16376 | 13617 | - | 44118 | 0 |
| 53 | Human | LAM1 | 192 | DLENRCQSL | 9 | 12584 | 12360 | 32752 | - | - | 0 |
| 54 | Human | LAM1 | 247 | EMREQHDAQV | 10 | 8828 | 510 | 3948 | - | 548 | 0 |
| 55 | Human | LAM1 | 262 | ELEQTYHAKL | 10 | 40156 | 2838 | - | - | 11673 | 0 |
| 56 | Human | LAM1 | 308 | NLQKESRACL | 10 | 9872 | 1130 | 4730 | - | 23622 | 0 |
| 57 | Human | LAM1 | 370 | DMEISAYRKL | 10 | - | 11689 | 27629 | - | - | 0 |
| 58 | Human | MYH9** | 9 | YLYVDKNFI | 9 | 9,3 | 0,85 | 4,8 | 45 | 217 | 5 |
| 59 | Human | MYH9 | 108 | GLIYTYSGL | 9 | 22 | 11 | 10 | 197 | 58 | 5 |
| 60 | Human | MYH9 | 111 | YTYSGLFCV | 9 | 0,12 | 2.1 | 3,0 | 0,84 | 3,2 | 5 |
| 61 | Human | MYH9 | 126 | NLPIYSEEI | 9 | 67 | 77 | 390 | 27 | 47 | 5 |
| 62 | Human | MYH9 | 145 | EMPPHIYAI | 9 | 323 | 67 | 110 | 85 | 1,8 | 5 |
| 63 | Human | MYH9 | 186 | KVIQYLAYV | 9 | 0,67 | 0,17 | 1,2 | 0,21 | 1,8 | 5 |
| 64 | Human | MYH9 | 478 | QLFNHTMFI | 9 | 1,4 | 0,71 | 3,2 | 14 | 14 | 5 |
| 65 | Human | MYH9 | 584 | WLMKNMDPL | 9 | 9,7 | 0,36 | 14 | 6,0 | 1,9 | 5 |

| | | | | | | | | | | | |
|---|---|---|---|---|---|---|---|---|---|---|---|
| #=identity; *=lamin B1; **=mn muscle myosin; °=1st aminoacid posttion; §=IC50 indicates biding affinity and it is expressed as 50% inhibitory nanomolar concentration, a dash ("-") indicates an IC50 >50000 nM; peptides with IC50 <50nM are classified as high affinity peptides, peptides with IC50>50 and <300nM are classified as intermediate affinity peptides, peptides with IC50>500nM are classified as low affinity peptides. | | | | | | | | | | | |

**Table 4. HLA-A2 binding affinity of synthetic peptides derived from fragmented proteins.**

| | | | | | | **Binding capacity (IC50§ nM)** | | | | | **Alleles bound** |
|---|---|---|---|---|---|---|---|---|---|---|---|
| **SEQ ID No.** | **Organism** | **Protein** | **1^{th} Pos°** | **Sequence** | **Length** | **A*0201** | **A*0202** | **A*0203** | **A*0206** | **A*6802** | |
| 66 | Human | MYH9** | 653 | QLAKLMATL | 9 | 5,4 | 1,4 | 2,9 | 26 | 18 | 5 |
| 67 | Human | MYH9 | 111 | YTYSGLFCVV | 10 | 0,21 | 0,34 | 0,12 | 4,2 | 0,4 | 5 |
| 68 | Human | MYH9 | 424 | RMFRWLVLRI | 10 | 1,1 | 5,0 | 10 | 71 | 398 | 5 |
| 69 | Human | MYH9 | 478 | QLFNHTMFIL | 10 | 0,97 | 0,42 | 8,2 | 49 | 82 | 5 |
| 70 | Human | MYH9 | 257 | YLLEKSRAI | 9 | 39 | 57 | 16 | 94 | 17045 | 4 |
| 71 | Human | MYH9 | 272 | RTFHIFYYL | 9 | - | 5,8 | 238 | 1,3 | 144 | 4 |
| 72 | Human | MYH9 | 280 | LLSGAGEHL | 9 | 199 | 5,7 | 19 | 361 | - | 4 |
| 73 | Human | MYH9 | 302 | FLSNGHVTI | 9 | 0,11 | 1,0 | 3,8 | 11 | 3050 | 4 |
| 74 | Human | MYH9 | 338 | GLLRVISGV | 9 | 0,21 | 3,0 | 3,1 | 4,3 | 8823 | 4 |
| 75 | Human | MYH9 | 412 | FAIEALAKA | 9 | 26 | 1,7 | 4,0 | 0,43 | - | 4 |
| 76 | Human | MYH9 | 450 | ILDIAGFEJ | 9 | 5,5 | 50 | 257 | 21 | 3064 | 4 |
| 77 | Human | MYH9 | 733 | FMDGKQACV | 9 | 0,18 | 7,8 | 33 | 6,6 | 10345 | 4 |
| 78 | Human | MYH9 | 741 | VLMIKALEL | 9 | 0,15 | 15 | 38 | 78 | 870 | 4 |
| 79 | Human | MYH9 | 829 | RLF7KVKPL | 9 | 205 | 9,7 | 6,2 | 421 | - | 4 |
| 80 | Human | MYH9 | 1084 | ELQAALARV | 9 | 558 | 117 | 57 | 40 | 2,5 | 4 |
| 81 | Human | MYH9 | 1277 | KLQVELDNV | 9 | 4,1 | 8,8 | 30 | 9,2 | - | 4 |
| 82 | Human | MYH9 | 1326 | SLSTKLKQV | 9 | 157 | 54 | 10 | 422 | 28642 | 4 |
| 83 | Human | MYH9 | 1843 | KLKDVLLQV | 9 | 3,3 | 2,5 | 6,7 | 0,20 | 18519 | 4 |
| 84 | Human | MYH9 | 279 | YLLSGAGEHL | 10 | 4,9 | 6,3 | 175 | 11 | - | 4 |
| 85 | Human | MYH9 | 322 | TMEAMRIMGI | 10 | 123 | 137 | 132 | 568 | 26 | 4 |
| 86 | Human | MYH9 | 338 | GLLRVISGVL | 10 | 68 | 304 | 118 | 390 | 736 | 4 |
| 87 | Human | MYH9 | 648 | QLYKEQIAKL | 10 | 78 | 4,6 | 16 | 147 | 9146 | 4 |
| 88 | Human | MYH9 | 733 | FMDGKQACVL | 10 | 21 | 27 | 158 | 683 | 374 | 4 |
| 89 | Human | MYH9 | 1920 | KLRRGDLPFV | 10 | 2,9 | 15 | 9,1 | 5,5 | - | 4 |
| 90 | Human | MYH9 | 210 | QLLQANPIL | 9 | 4,5 | 183 | 1507 | 19 | - | 3 |
| 91 | Human | MYH9 | 847 | MMAKEEELV | 9 | 9,1 | 1,2 | 276 | 1070 | 5639 | 3 |
| 92 | Human | MYH9 | 877 | QLMAEKLQL | 9 | 8,2 | 7,9 | 205 | 657 | 4360 | 3 |
| 93 | Human | MYH9 | 940 | KMQQNIQEL | 9 | 13 | 15 | 82 | 536 | 28571 | 3 |
| 94 | Human | MYH9 | 1270 | ELADKVTKL | 9 | 2513 | 33 | 114 | 38752 | 3,8 | 3 |
| 95 | Human | MYH9 | 1298 | KLTKDFSAL | 9 | 550 | 20 | 34 | 29 | 1592 | 3 |
| 96 | Human | MYH9 | 1372 | KMEDSVGCL | 9 | 284 | 22 | 176 | 2846 | - | 3 |
| 97 | Human | MYH9 | 1389 | KLQKDLEGL | 9 | 29 | 25 | 118 | 619 | 15139 | 3 |

| | | | | | | | | | | | |
|---|---|---|---|---|---|---|---|---|---|---|---|
| #=identity; **=non muscle myosin; °=1st aminoacid position; §=IC50 indicates binding affinity and it is expressed as 50% inhibitory nanomolar concentration, a dash ("-") indicates an IC50 >50000 nM; peptides with IC50 <50nM are classified as high affinity peptides, peptides with IC50>50 and <500nM are classified as intermediate affinity peptides, peptides with | | | | | | | | | | | |

**Table 5. HLA-A2 binding affinity of synthetic peptides derived from fragmented proteins.**

| | | | | | | **Binding capacity (IC50§ nM)** | | | | | **Alleles bound** |
|---|---|---|---|---|---|---|---|---|---|---|---|
| **SEQ ID No.** | **Organism** | **Protein** | **1^{th} Pos°** | **Sequence** | **Length** | **A*0201** | **A*0202** | **A*0203** | **A*0206** | **A*6802** | |
| 98 | Human | MYH9" | 1536 | EMKTQLEEL | 9 | 20769 | 83 | 81 | 22770 | 284 | 3 |
| 99 | Human | MYH9 | 1726 | RLEARIAQL | 9 | 433 | 7,7 | 15 | 2292 | 46881 | 3 |
| 100 | Human | MYH9 | 1793 | KLQEMEGTV | 9 | 23 | 99 | 48 | 867 | 19355 | 3 |
| 101 | Human | MYH9 | 1811 | ALEAKIAQL | 9 | 103 | 15 | 47 | 1100 | 8772 | 3 |
| 102 | Human | MYH9 | 1909 | AMNREVSSL | 9 | 92 | 1,6 | 3,9 | 2994 | 11952 | 3 |
| 103 | Human | MYH9 | 74 | KMNPPKFSKV | 10 | 34 | 2457 | 50 | 154 | 20000 | 3 |
| 104 | Human | MYH9 | 660 | TLRNTNPNFV | 10 | 21 | 107 | 40 | - | 593 | 3 |
| 105 | Human | MYH9 | 688 | VLDQLRCNGV | 10 | 6,6 | 186 | 61 | 868 | - | 3 |
| 106 | Human | MYH9 | 752 | NLYRIGQSKV | 10 | 322 | 87 | 76 | 17848 | 1024 | 3 |
| 107 | Human | MYH9 | 811 | VLQRNCAAYL | 10 | 96 | 18 | 95 | 2180 | - | 3 |
| 108 | Human | MYH9 | 829 | RLFTKVKPLL | 10 | 53 | 78 | 96 | 4039 | 16393 | 3 |
| 109 | Human | MYH9 | 1027 | AMITDLEERL | 10 | 127 | 10 | 193 | 543 | 941 | 3 |
| 110 | Human | MYH9 | 1228 | ELANEVKVLL | 10 | 66 | 17 | 1255 | 24371 | 2,9 | 3 |
| 111 | Human | MYH9 | 248 | YIVGANIET | 9 | 422 | 622 | 9009 | 81 | 626 | 2 |
| 112 | Human | MYH9 | 591 | PLNDNIATL | 9 | 1374 | 137 | 294 | - | 21277 | 2 |
| 113 | Human | MYH9 | 641 | GMFRTVGQL | 9 | 867 | 74 | 89 | 14107 | - | 2 |
| 114 | Human | MYH9 | 975 | KLEEEQIIL | 9 | 72 | 74 | 2552 | 940 | 21583 | 2 |
| 115 | Human | MYH9 | 982 | ILEDQNCKL | 9 | 90 | 41 | 997 | 6043 | - | 2 |
| 116 | Human | MYH9 | 1309 | QLQDTQELL | 9 | 49 | 5,0 | 2451 | 4684 | 7879 | 2 |
| 117 | Human | MYH9 | 1540 | QLEELEDEL | 9 | 404 | 176 | - | 28866 | 4718 | 2 |
| 118 | Human | MYH9 | 1646 | QMKDCMREL | 9 | 3622 | 155 | 14 | 9819 | - | 2 |
| 119 | Human | MYH9 | 1677 | SMEAEMIQL | 9 | 40 | 70 | 2382 | 1093 | - | 2 |
| 120 | Human | MYH9 | 1681 | EMIQLQEEL | 9 | 3097 | 123 | 35881 | 23126 | 105 | 2 |
| 121 | Human | MYH9 | 1751 | RLKKANLQI | 9 | 1847 | 269 | 151 | 3769 | - | 2 |
| 122 | Human | MYH9 | 100 | NLKERYYSGL | 10 | 7214 | 1,6 | 214 | - | 5455 | 2 |
| 123 | Human | MYH9 | 161 | MMQDREDQSI | 10 | 2,1 | 25 | 542 | 1245 | 4005 | 2 |
| 124 | Human | MYH9 | 428 | WLVLRINKAL | 10 | 976 | 23 | 257 | 556 | 5329 | 2 |
| 125 | Human | MYH9 | 599 | LLHQSSDKFV | 10 | 808 | 14 | 78 | 17504 | 3571 | 2 |
| 126 | Human | MYH9 | 821 | KLANWQW WRL | 10 | 56 | 54 | 1037 | 5442 | - | 2 |
| 127 | Human | MYH9 | 846 | EMMAKEEELV | 10 | 33 | 9,6 | 958 | 2456 | 2809 | 2 |
| 128 | Human | MYH9 | 1143 | ALKTELEDTL | 10 | 652 | 27 | 431 | 17831 | - | 2 |
| 129 | Human | MYH9 | 1316 | LLQEENRQKL | 10 | 245 | 120 | 1903 | 3764 | - | 2 |

| | | | | | | | | | | | |
|---|---|---|---|---|---|---|---|---|---|---|---|
| #=identity; **=non muscle myosin °=1st aminoacid position; §=IC50 indicates binding affinity and it is expressed as 50% inhibitory nanomolar concentration, a dash ("-") indicates an IC50 >50000 nM; peptides with IC50 <50nM are classified as high affinity peptides, peptides with IC50>50 and <500nM are classified as intermediate affinity peptides, peptides with | | | | | | | | | | | |

**Table 6. HLA-A2 binding affinity of synthetic peptides derived from fragmented proteins.**

| | | | | | | **Binding capacity (IC50§ nM)** | | | | | **Alleles bound** |
|---|---|---|---|---|---|---|---|---|---|---|---|
| **SEQ ID No.** | **Organism** | **Protein** | **1^{th} Pos°** | **Sequence** | **Length** | **A*0201** | **A*0202** | **A*0203** | **A*0206** | **A*6802** | |
| 130 | Human | MYH9** | 1417 | RLQQELDDLL | 10 | 92 | 5,0 | 13611 | 1041 | 17072 | 2 |
| 131 | Human | MYH9 | 1674 | KLKSMEAEMI | 10 | 1465 | 93 | 314 | 7835 | 9009 | 2 |
| 132 | Human | MYH9 | 339 | LLRVISGVL | 9 | 1756 | 1769 | 133 | 1148 | 3191 | 1 |
| 133 | Human | MYH9 | 588 | NMDPLNDNI | 9 | 1928 | 108 | 2492 | 25916 | - | 1 |
| 134 | Human | MYH9 | 748 | ELDSNLYRI | 9 | 353 | 522 | 13587 | 4010 | 1099 | 1 |
| 135 | Human | MYH9 | 846 | EMMAKEEEL | 9 | 3226 | 67 | 16679 | 24202 | 13889 | 1 |
| 136 | Human | MYH9 | 870 | EMETLQSQL | 9 | 6093 | 389 | 48703 | - | 14778 | 1 |
| 137 | Human | MYH9 | 894 | ELCAEAEEL | 9 | 16191 | 194 | 18303 | 24732 | 1274 | 1 |
| 138 | Human | MYH9 | 989 | KLAKEKKLL | 9 | 9793 | 197 | 1293 | 37909 | 17143 | 1 |
| 139 | Human | MYH9 | 1045 | ELEKTRRKL | 9 | 8215 | 275 | 32650 | 45176 | 8403 | 1 |
| 140 | Human | MYH9 | 1052 | KLEGDSTDL | 9 | 2001 | 482 | 25287 | 10828 | 2463 | 1 |
| 141 | Human | MYH9 | 1059 | DLSDQIAEL | 9 | 2217 | 72 | 2924 | 11939 | 583 | 1 |
| 142 | Human | MYH9 | 1066 | ELQAQIAEL | 9 | 1093 | 1022 | 4539 | 29473 | 14 | 1 |
| 143 | Human | MYH9 | 1077 | QLAKKEEEL | 9 | 7887 | 110 | 29889 | - | 15625 | 1 |
| 144 | Human | MYH9 | 1108 | ELESQISEL | 9 | - | 95 | 23119 | 45805 | - | 1 |
| 145 | Human | MYH9 | 1228 | ELANEVKVL | 9 | 7891 | 1008 | 914 | 38221 | 116 | 1 |
| 146 | Human | MYH9 | 1281 | ELDNVTGLL | 9 | 9654 | 22 | 2539 | 49404 | - | 1 |
| 147 | Human | MYH9 | 1354 | NLEKQIATL | 9 | 1280 | 393 | 662 | 31973 | 26470 | 1 |
| 148 | Human | MYH9 | 1410 | KLEKTKTRL | 9 | 19721 | 1346 | 4972 | 106 | - | 1 |
| 149 | Human | MYH9 | 1417 | RLQQELDDL | 9 | 960 | 106 | 1628 | 6747 | - | 1 |
| 150 | Human | MYH9 | 1421 | ELDDLLVDL | 9 | 926 | 180 | 25362 | 20240 | 12533 | 1 |
| 151 | Human | MYH9 | 1508 | DLMSSKDDV | 9 | 4292 | 10433 | 19470 | - | 352 | 1 |
| 152 | Human | MYH9 | 88 | ELTCLNEASV | 10 | 4576 | 699 | 3482 | 3739 | 69 | 1 |
| 153 | Human | MYH9 | 561 | QLKDKADFCI | 10 | 859 | 4,3 | 1235 | 32059 | 16129 | 1 |
| 154 | Human | MYH9 | 591 | PLNDNIATLL | 10 | 15874 | 165 | 1315 | 10005 | 8929 | 1 |
| 155 | Human | MYH9 | 853 | ELVKVREKQL | 10 | 1134 | 120 | 1593 | 9037 | 5190 | 1 |
| 156 | Human | MYH9 | 972 | KLKKIFEEQI | 10 | 4265 | 133 | 1487 | - | - | 1 |
| 157 | Human | MYH9 | 1020 | KLKNKHEAMI | 10 | 4078 | 161 | 566 | - | 10250 | 1 |
| 158 | Human | MYH9 | 1396 | GLSQRHEEKV | 10 | 1016 | 429 | 1754 | - | 12672 | 1 |
| 159 | Human | MYH9 | 1547 | ELQATEDAKL | 10 | 20843 | 246 | 36430 | 8677 | 12970 | 1 |
| 160 | Human | MYH9 | 1718 | ALALEEKRRL | 10 | 12186 | 91 | 1478 | - | - | 1 |
| 161 | Human | MYH9 | 57 | ELVENGKKV | 9 | 5352 | 3186 | 8839 | 9575 | 7634 | 0 |

| | | | | | | | | | | | |
|---|---|---|---|---|---|---|---|---|---|---|---|
| #=identity; **=non muscle myosin;°=1st aminoacid position; §=IC50 indicates binding affinity and it is expressed as 50% inhibitory nanomolar concentration, a dash ("-") indicates an IC50 >50000 nM; peptides with IC50 <50nM are classified as high affinity peptides, peptides with IC50>50 and <500nM are classified as intermediate affinity peptides, peptides with | | | | | | | | | | | |

**Table 7. HLA-A2 binding affinity of synthetic peptides derived from fragmented proteins.**

| | | | | | | **Binding capacity (IC50§ nM)** | | | | | **Alleles bound** |
|---|---|---|---|---|---|---|---|---|---|---|---|
| **SEQ ID No.** | **Organism** | **Protein** | **1^{th} Pos°** | **Sequence** | **Length** | **A*0201** | **A-0202** | **A*0203** | **A*0206** | **A*6802** | |
| 162 | Human | MYH9** | 507 | DLQPCIDLI | 9 | 14684 | 562 | 5210 | 21315 | 1685 | 0 |
| 163 | Human | MYH9 | 610 | ELWKDVDRI | 9 | 1619 | 2655 | 12174 | 11071 | 2254 | 0 |
| 164 | Human | MYH9 | 776 | DLKITDVII | 9 | 1640 | 8882 | 1538 | 1201 | 1813 | 0 |
| 165 | Human | MYH9 | 905 | RLTAKKQI1 | 9 | 19580 | 1134 | 2436 | - | 25862 | 0 |
| 166 | Human | MYH9 | 912 | ELEEICHDL | 9 | 10924 | 1221 | 10150 | - | 36585 | 0 |
| 167 | Human | MYH9 | 1136 | DLGEELEAL | 9 | 26540 | 3367 | 27494 | - | - | 0 |
| 168 | Human | MYH9 | 1140 | ELEALKTEL | 9 | - | 4688 | 14696 | - | 15905 | 0 |
| 169 | Human | MYH9 | 1214 | NLEKAKQTL | 9 | 23964 | 6332 | 46974 | 43929 | 693 | 0 |
| 170 | Human | MYH9 | 1221 | TLENERGEL | 9 | 28583 | 1344 | 18763 | 45571 | 21456 | 0 |
| 171 | Human | MYH9 | 1448 | QLLAEEKTI | 9 | 4386 | 988 | 2603 | 18097 | - | 0 |
| 172 | Human | MYH9 | 1522 | ELEKSKRAL | 9 | 30314 | 19745 | 39690 | - | - | 0 |
| 173 | Human | MYH9 | 1614 | KIEMDLKDL | 9 | 1091 | 3124 | 3816 | 2548 | - | 0 |
| 174 | Human | MYH9 | 1618 | DLKDLEAHI | 9 | - | 14337 | 46029 | - | 21127 | 0 |
| 175 | Human | MYH9 | 1782 | QLERQNKEL | 9 | 10043 | 1401 | 38094 | 22548 | 25210 | 0 |
| 176 | Human | MYH9 | 1871 | RLKQLKRQL | 9 | 3813 | 5498 | 832 | - | 9967 | 0 |
| 177 | Human | MYH9 | 126 | NLPIYSEEIV | 10 | 2778 | 614 | 37384 | 9609 | 1179 | 0 |
| 178 | Human | MYH9 | 460 | DLNSFEQLCI | 10 | 4742 | 1388 | 13212 | - | 3344 | 0 |
| 179 | Human | MYH9 | 505 | GLDLQPCIDL | 10 | 690 | 1202 | 16159 | 18387 | - | 0 |
| 180 | Human | MYH9 | 610 | ELWKDVDRII | 10 | 8866 | 5426 | 24375 | - | 13216 | 0 |
| 181 | Human | MYH9 | 770 | HLEEERDLKI | 10 | 5209 | 1336 | 21844 | - | - | 0 |
| 182 | Human | MYH9 | 1100 | NMALKKIREL | 10 | 16361 | 4234 | 791 | 892 | - | 0 |
| 183 | Human | MYH9 | 1160 | ELRSKREQEV | 10 | 18756 | 5446 | 29769 | - | 3033 | 0 |
| 184 | Human | MYH9 | 1189 | EMRQKHSQAV | 10 | 33936 | 8731 | 7945 | - | 615 | 0 |
| 185 | Human | MYH9 | 1368 | DMKKKMEDSV | 10 | 15027 | 14863 | 38466 | - | 10676 | 0 |
| 186 | Human | MYH9 | 1564 | AMKAQFERDL | 10 | 8294 | 961 | 687 | 9690 | - | 0 |
| 187 | Human | MYH9 | 1606 | SMAVAARKKL | 10 | - | 755 | 572 | - | 926 | 0 |
| 188 | Human | GDIS^^ | 100 | VLKEGSEYRV | 10 | 342 | 10 | 149 | 1003 | 234 | 4 |
| 189 | Human | GDIS | 186 | HLSWEWNLSI | 10 | 6,1 | 13 | 68 | 56 | 1093 | 4 |
| 190 | Human | GDIS | 37 | EMDKDDESL | 9 | 340 | 637 | 1099 | 360 | 42 | 3 |
| 191 | Human | GDIS | 51 | TLLGDGPVV | 9 | 20 | 547 | 675 | 236 | 4354 | 2 |
| 192 | Human | GDIS | 93 | ALKKETIVL | 9 | 834 | 38 | 42 | 25437 | 12508 | 2 |
| 193 | Human | GDIS | 44 | SLIKYKKTL | 9 | 3549 | 281 | 1735 | 3982 | - | 1 |

| | | | | | | | | | | | |
|---|---|---|---|---|---|---|---|---|---|---|---|
| #=identity; **=non muscle myosin; ^^=rho GDP dissociation inhibitor 2; °=1st aminoacid position; §=IC50 indicates binding affinity and it is expressed as 50% inhibitory nanomolar concentration, a dash ("-") idicates an IC50>50000 nM; peptides wih IC50<50nM are classified as hgh affinity peptides, peptides with IC50>50 and <500nM are classified as intermediate affinity peptides, peptides with IC30>500nM are classfied as low afinity peptides | | | | | | | | | | | |

**Table 8. HLA-A2 binding affinity of synthetic peptides derived from fragmented proteins.**

| | | | | | | **Binding capacity (IC50§ nM)** | | | | | **Alleles bound** |
|---|---|---|---|---|---|---|---|---|---|---|---|
| **SEQ ID No.** | **Organism** | **Protein** | **1^{th} Pos°** | **Sequence** | **Length** | **A*0201** | **A*0202** | **A*0203** | **A*0206** | **A*6802** | |
| 194 | Human | GDIS^^ | 86 | DLTGDLEAL | 9 | 16949 | 714 | 24638 | 14995 | 18411 | 0 |
| 195 | Human | GDIS | 37 | EMDKDDESLI | 10 | 25743 | 1900 | 17268 | - | - | 0 |
| 196 | Human | GDIS | 44 | SLIKYKKTLL | 10 | 1057 | 885 | 532 | 4916 | 779 | 0 |
| 197 | Human | GDIS | 90 | DLEALKKETI | 10 | - | 21231 | 23588 | - | - | 0 |
| 198 | Human | VIME% | 176 | NLAEDIMRL | 9 | 1,0 | 0,31 | 4,2 | 136 | 83 | 5 |
| 199 | Human | VIME | 50 | SLYASSPGGV | 10 | 2,2 | 4,3 | 2,4 | 461 | 452 | 5 |
| 200 | Human | VIME | 68 | RLRSSVPGV | 9 | 3,3 | 27 | 1,6 | 30 | 9901 | 4 |
| 201 | Human | VIME | 129 | ILLAELEQL | 9 | 35 | 6,5 | 231 | 7,5 | - | 4 |
| 202 | Human | VIME | 225 | SLQEEIAFL | 9 | 0,99 | 0,37 | 19 | 44 | 4777 | 4 |
| 203 | Human | VIME | 331 | ALKGTNESL | 9 | 108 | 9,5 | 47 | 445 | 771 | 4 |
| 204 | Human | VIME | 78 | LLQDSVDFSL | 10 | 3,6 | 1,1 | 149 | 9,2 | 1931 | 4 |
| 205 | Human | VIME | 411 | SLPLPNFSSL | 10 | 61 | 199 | 16 | 641 | 1,2 | 4 |
| 206 | Human | VIME | 79 | LQDSVDFSL | 9 | 5,9 | 47 | 10155 | 2,3 | 774 | 3 |
| 207 | Human | VIME | 324 | SLTCEVDAL | 9 | 54 | 6,3 | 398 | 1080 | 3119 | 3 |
| 208 | Human | VIME | 378 | HLREYQDLL | 9 | 59 | 4,2 | 171 | 8893 | 1641 | 3 |
| 209 | Human | VIME | 384 | DLLNVKMAL | 9 | 181 | 265 | 109 | 787 | 647 | 3 |
| 210 | Human | VIME | 419 | SLNLRETNL | 9 | 402 | 1,4 | 22 | 16458 | - | 3 |
| 211 | Human | VIME | 122 | FLEQQNKILL | 10 | 20 | 0,43 | 63 | 828 | 10669 | 3 |
| 212 | Human | VIME | 232 | FLKKLHEEEI | 10 | 372 | 9,7 | 53 | 18182 | 11931 | 3 |
| 213 | Human | VIME | 235 | KLHEEEIQEL | 10 | 226 | 18 | 278 | 4828 | - | 3 |
| 214 | Human | VIME | 401 | KLLEGEESR1 | 10 | 127 | 35 | 166 | 1172 | - | 3 |
| 215 | Human | VIME | 122 | FIEQQNKIL | 9 | 496 | 25 | 814 | 12281 | - | 2 |
| 216 | Human | VIME | 155 | ELRRQVDQL | 9 | 434 | 89 | 2151 | 9148 | 1449 | 2 |
| 217 | Human | VIME | 201 | TLQSFRQDV | 9 | 154 | 290 | 1264 | 7375 | 2074 | 2 |
| 218 | Human | VIME | 218 | DLERKVESL | 9 | 121 | 70 | 3058 | 3374 | - | 2 |
| 219 | Human | VIME | 108 | ELNDRFANYI | 10 | 1786 | 38 | 543 | 45376 | 14 | 2 |
| 220 | Human | VIME | 370 | NMKEEMARHL | 10 | 260 | 433 | 703 | 10578 | 9346 | 2 |
| 221 | Human | VIME | 385 | LLNVKMALDI | 10 | 4638 | 222 | 162 | 562 | 1201 | 2 |
| 222 | Human | VIME | 421 | NLRETNLDSL | 10 | 5263 | 156 | 83 | 8250 | 2672 | 2 |
| 223 | Human | VIME | 263 | DLTAALRDV | 9 | 10569 | 735 | 147 | 28139 | 2459 | 1 |
| 224 | Human | VIME | 402 | LLEGEESRI | 9 | 856 | 296 | 921 | 1472 | 877 | 1 |
| 225 | Human | VIME | 42 | ALRPSTSRSL | 10 | 3996 | 2489 | 284 | 42830 | - | 1 |
| 226 | Human | VIME | 147 | DLYEEEMREL | 10 | 14986 | 485 | 827 | 33261 | 16216 | 1 |
| 227 | Human | VIME | 413 | PLPNFSSLNL | 10 | 3769 | 172 | 2927 | - | 17910 | 1 |
| 228 | Human | VIME | 152 | EMRELRRQV | 9 | 30508 | 3455 | 3015 | - | 40541 | 0 |
| 229 | Human | VIME | 162 | QLTNDKARV | 9 | 810 | 567 | 634 | 1689 | 695 | 0 |
| 230 | Human | VIME | 136 | QLKGQGKSRL | 10 | 30885 | 2925 | 3801 | - | - | 0 |

| | | | | | | | | | | | |
|---|---|---|---|---|---|---|---|---|---|---|---|
| #=identity; ^^=rho GDP dissociation inhibitor 2; %=vimentin; °=1st aminoacid position; §=IC50 indicates binding affinity and it is expressed as 50% inhibitory nanomolar concentration : dash ("-") indicates an IC50>50000 nM; peptides wih IC50<50nM are classified as high affinity peptites, peptiles with IC50>50 and <500nM are classified as intermediate affinity peptides, peptides wih IC50>500nM are classified as bw affinity peptides. | | | | | | | | | | | |

**Table 9. HLA-A2 binding affinity of synthetic peptides derived from not fragmented proteins.**

| | | | | | | **Binding capacity (IC50§ nM)** | | | | | **Alleles bound** |
|---|---|---|---|---|---|---|---|---|---|---|---|
| **SEQ ID No.** | **Organism** | **Protein** | **1^{th} Pos°** | **Sequence** | **Length** | **A*0201** | **A-0202** | **A*0203** | **A*0206** | **A*6802** | |
| 231 | Human | RLA2* | 38 | RLNKVISEL | 9 | 15433 | 107 | 157 | - | 14162 | 2 |
| 232 | Human | PSA1** | 179 | FMECNLNEL | 9 | 596 | 27 | 55 | 15067 | 14822 | 2 |
| 233 | Human | PSA1 | 183 | NLNELVKHGL | 10 | 145 | 12 | 11 | 4834 | 3618 | 3 |
| 234 | Human | PSA1 | 175 | HMSEFMECNL | 10 | 59 | 11 | 70 | 109 | 35360 | 4 |
| 235 | Human | PSA1 | 63 | ILHVDNHIGI | 10 | - | - | 16720 | - | - | 0 |
| 236 | Human | PSA1 | 37 | GLKSKTHAV | 9 | - | 4552 | 924 | - | 19982 | 0 |
| 237 | Human | PSA1 | 110 | SLIGSKTQI | 9 | - | 15913 | 511 | 34100 | 17135 | 0 |
| 238 | Human | PSA1 | 179 | FMECNLNELV | 10 | 12715 | 194 | 1 | - | - | 2 |
| 239 | Human | PSA1 | 48 | ALKRAQSEL | 9 | - | - | 18952 | - | 29040 | 0 |
| 240 | Human | PSA1 | 76 | GLTADARLL | 9 | 163 | 4,7 | 1 | 7,8 | 12 | 5 |
| 241 | Human | PSA1 | 102 | PLPVSRLVSL | 10 | 4,5 | 1,2 | 0,21 | 40 | 1659 | 4 |
| 242 | Human | PSA1 | 204 | DLTTKNVSI | 9 | 465 | 3,6 | 54 | 4278 | 7386 | 3 |
| 243 | Human | PSA1 | 45 | ELNGKNIEDV | 10 | 104 | 1,2 | 0,21 | 1776 | 14417 | 3 |
| 244 | Human | PSA1 | 55 | ELAAHQKKI | 9 | 38 | 2 | 1,1 | 100 | 9790 | 4 |
| 245 | Human | PSA1 | 186 | ELVKHGLRAL | 10 | 296 | 15 | 1,6 | 8411 | - | 3 |
| 246 | Human | PSA1 | 37 | GLKSKTHAVL | 10 | 2366 | 116 | 24 | 10609 | 21672 | 2 |
| 247 | Human | PSA1 | 191 | GLRALRETL | 9 | 41760 | 45 | 23 | - | - | 2 |
| 248 | Human | PSA1 | 55 | ELAAHQKKIL | 10 | 8856 | 146 | 362 | 16700 | - | 2 |
| 249 | Human | PSA1 | 97 | FVFDRPLPV | 9 | - | 8417 | 2175 | 9732 | 44603 | 0 |
| 250 | Human | RLA2 | 3 | YVASYLLAA | 9 | - | 6236 | 2649 | - | 1908 | 0 |
| 251 | Human | RLA2 | 26 | ILDSVGIEA | 9 | 1271 | 235 | 112 | 1756 | 1598 | 2 |
| 252 | Human | RLA2 | 3 | YVASYLLAAL | 10 | 1 | 0,88 | 0,5 | 2,4 | 1,5 | 5 |

| | | | | | | | | | | | |
|---|---|---|---|---|---|---|---|---|---|---|---|
| #=identity;*RLA2=60S acidic ribosomal protein P2;**PSA1= Proteasome component C2; °=1st aminoacid position; §=IC50 indicates binding affinity and it is expressed as 50% inhibitory nanomolar concentration, a dash ("-") indicates an IC50 >50000 nM; peptides with IC50<50nM are classified as high affinity peptides, peptides with IC50>50 and <500nM | | | | | | | | | | | |

### Multispecificity of CD8⁺ T_{EM} cells to apoptotic epitopes correlates with infection chronicity

The author analyzed longitudinally the responses of 16 patients with acute HCV infection. The follow-up ranged from the onset of acute disease (**clinical onset**) to 15 to 24 months (the sixth month being considered the time of conversion from an acute to a chronic infection). Of the 16 patients, 6 patients had a self-limited infection and 10 patients exhibited a chronic evolution of infection (**Table 10**).

**Table 10. Clinical parameters of patients with acute HCV infection**

| **Pt** | **Genotype** | **Outcome** | **ALT (U/ml)** | **HCV-RNA (copies/ml)** |
|---|---|---|---|---|
| 1C | 1b | Chronic | 107 | 556000 |
| 2C | 2a/2c | Chronic | 2700 | 3310 |
| 3C | 3a | Chronic | 1113 | 156000 |
| 4C | 2a/2c | Chronic | 600 | 915000 |
| 5C | 1b | Chronic | 1515 | 600 |
| 6C | 3a | Chronic | 324 | 940000 |
| 7C | 1b | Chronic | 792 | 462000 |
| 8C | 3a | Chronic | 745 | 900000 |
| 9C | 1a | Chronic | 904 | 500000 |
| 10C | 3a | Chronic | 803 | 919000 |
| 1S | 1b | Self-limited | 2710 | 106000 |
| 2S | 3a | Self-limited | 1967 | 2120 |
| 3S | 2a/2c | Self-limited | 5917 | 361000 |
| 4S | 1a | Self-limited | 1359 | 96100 |
| 5S | n.d. | Self-limited | 2860 | 210200 |
| 6S | n.d. | Self-limited | 2650 | 69500 |

Pt, patient; n.d., not determined; ALT. serum alanine aminotransferase (n.v., 0-40 U/ml)

Initially, the effector responses were determined by the capacity of freshly isolated CD8⁺ T cells from either HLA-A2⁺ patients or healthy controls to form IFN-γ spots within 4 to 6 h of contact with nine pools of synthetic apoptotic peptides (**Tables 11**-**13**) or eight pools of HCV peptides (**Tables 14**-**17**) selected for their capacity to bind the HLA-A2 molecule (14, 20), in an ELISPOT assay.

**Table 11. HLA-A2 binding peptides derived from apoptotic cell-associated proteins appearing with their cleavage products in proteomic analysis.**

| | | | | | | **Binding capacity (IC50§ nM)** | | | | | **Alleles bound** |
|---|---|---|---|---|---|---|---|---|---|---|---|
| Pool | **Organism** | **Protein** | **1^{st} Pos°** | **Sequence** | **Length** | **A*0201** | **A*0202** | **A*0203** | **A*0206** | **A*6802** | |
| 1 | Human | ACTB" | 131 | AMYVAIQAV | 9 | 2.8 | 1.5 | 1.6 | 7.1 | 190 | 5 |
| | Human | ACTB | 319 | ALAPSTMKI | 9 | 4.3 | 2.9 | 2.5 | 40 | 5.5 | 5 |
| | Human | ACTB | 266 | FLGMESCGI | 9 | 2.7 | 71 | 11 | 16 | 3979 | 4 |
| | Human | ACTB | 312 | RMQKEITAL | 9 | 14 | 0.29 | 6.5 | 28 | 6667 | 4 |
| | Human | ACTB | 348 | SLSTFQQMWI | 10 | 5.6 | 4.5 | 7.7 | 791 | 299 | 4 |
| | Human | ACTB | 46 | GMGQKDSYV | 9 | 196 | 17 | 156 | 13547 | 17632 | 3 |
| 2 | Human | ROK^ | 154 | SLAGGIIGV | 9 | 0.56 | 1.0 | 1.1 | 0.14 | 49 | 5 |
| | Human | ROK | 67 | ALRTDYNASV | 10 | 8.9 | 5.0 | 11 | 349 | 1354 | 4 |
| | Human | ROK | 193 | VLIGGKPDRV | 10 | 14 | 8.5 | 12 | 1282 | 7417 | 3 |
| | Human | ROK | 209 | ILDLISESPI | 10 | 360 | 65 | 1272 | 2598 | 3083 | 2 |
| | Human | ROK | 122 | QLPLESDAV | 9 | 246 | 601 | 1270 | 925 | 1999 | 1 |
| 3 | Human | LAM1* | 496 | TIWAANAGV | 9 | 8.9 | 263 | 308 | 22 | 20 | 5 |
| | Human | LAM1 | 41 | RLAVYIDKV | 9 | 0.16 | 0.84 | 0.18 | 14 | 1004 | 4 |
| | Human | LAM1 | 301 | SLSSQLSNL | 9 | 1.6 | 0.57 | 2.4 | 104 | 1377 | 4 |
| | Human | LAM1 | 361 | QLLDVKLAL | 9 | 0.060 | 98 | 288 | 4.0 | 5848 | 4 |
| | Human | LAM1 | 291 | ELMESRMRI | 9 | 246 | 1226 | 284 | 25188 | 45 | 3 |
| | Human | LAM1 | 355 | QLNDYEQLL | 9 | 2.0 | 0.23 | 18 | 1493 | 4808 | 3 |
| | Human | LAM1 | 388 | KLSPSPSSRV | 10 | 50 | 18 | 45 | 563 | 20979 | 3 |
| | Human | LAM1 | 488 | VLKAGQTVTI | 10 | 263 | 37 | 23 | 4060 | 15429 | 3 |
| | Human | LAM1 | 378 | KLLEGEEERL | 10 | 30 | 29 | 3395 | 1944 | 20515 | 2 |
| 4 | Human | MYH9" | 9 | YLYVDKNFI | 9 | 9.3 | 0.85 | 4.8 | 45 | 217 | 5 |
| | Human | MYH9 | 108 | GLIYTYSGL | 9 | 22 | 11 | 10 | 197 | 58 | 5 |
| | Human | MYH9 | 111 | YTYSGLFCV | 9 | 0.12 | 2.1 | 3.0 | 0.84 | 3.2 | 5 |
| | Human | MYH9 | 145 | EMPPHIYAl | 9 | 323 | 67 | 110 | 85 | 1.8 | 5 |
| | Human | MYH9 | 186 | KVIQYLAYV | 9 | 0.67 | 0.17 | 1.2 | 0.21 | 1.8 | 5 |
| | Human | MYH9 | 478 | QLFNHTMFI | 9 | 1.4 | 0.71 | 3.2 | 14 | 14 | 5 |
| | Human | MYH9 | 584 | WLMKNMDPL | 9 | 9.7 | 0.36 | 14 | 6.0 | 1.9 | 5 |
| | Human | MYH9 | 653 | QLAKLMATL | 9 | 5.4 | 1.4 | 2.9 | 26 | 18 | 5 |
| | Human | MYH9 | 111 | YTYSGLFCW | 10 | 0.21 | 0.34 | 0.12 | 4.2 | 0.4 | 5 |

| | | | | | | | | | | | |
|---|---|---|---|---|---|---|---|---|---|---|---|
| °=1^{st} amino acid position; §=IC50 indicates binding affinity and it is expressed as 50% inhibitory nanomolar concentration, a dash ("-") indicates an IC50 >50000 nM; peptides with IC50 <50nM are classified as high affinity peptides, peptides with IC50>50 and <500nM are classified as intermediate affinity peptides, peptides with IC50>500nM are classified as low affinity peptides; "=actin cytoplasmatic 1; ^=heterogeneous nuclear ribonucleoprotein K; *=lamin B1; **=non muscle myosin. | | | | | | | | | | | |

**Table 12. HLA-A2 binding peptides derived from apoptotic cell-associated proteins appearing with their cleavage products in proteomic analysis.**

| | | | | | | **Binding capacity (IC50§ nM)** | | | | | **Alleles bound** |
|---|---|---|---|---|---|---|---|---|---|---|---|
| **Pool** | **Organism** | **Protein** | **1^{st} Pos°** | **Sequence** | **Length** | **A*0201** | **A*0202** | **A*0203** | **A*0206** | **A*6802** | |
| 5 | Human | MYH9** | 424 | RMFRWLVLRI | 10 | 1.1 | 5.0 | 10 | 71 | 398 | 5 |
| | Human | MYH9 | 478 | QLFNHTMFIL | 10 | 0.97 | 0.42 | 8.2 | 49 | 82 | 5 |
| | Human | MYH9 | 302 | FLSNGHVTI | 9 | 0.11 | 1.0 | 3.8 | 11 | 3050 | 4 |
| | Human | MYH9 | 338 | GLLRVISGV | 9 | 0.21 | 3.0 | 3.1 | 4.3 | 8823 | 4 |
| | Human | MYH9 | 412 | FAIEALAKA | 9 | 26 | 1.7 | 4.0 | 0.43 | - | 4 |
| | Human | MYH9 | 450 | ILDIAGFEI | 9 | 5.5 | 50 | 257 | 21 | 3064 | 4 |
| | Human | MYH9 | 733 | FMDGKQACV | 9 | 0.18 | 7.8 | 33 | 6.6 | 10345 | 4 |
| | Human | MYH9 | 741 | VLMIKALEL | 9 | 0.15 | 15 | 38 | 78 | 870 | 4 |
| | Human | MYH9 | 1277 | KLQVELDNV | 9 | 4.1 | 8.8 | 30 | 9.2 | - | 4 |
| | Human | MYH9 | 1843 | KLKDVLLQV | 9 | 3.3 | 2.5 | 6.7 | 0.20 | 18519 | 4 |
| | Human | MYH9 | 279 | YLLSGAGEHL | 10 | 4.9 | 6.3 | 175 | 11 | - | 4 |
| | Human | MYH9 | 733 | FMDGKQACVL | 10 | 21 | 27 | 158 | 683 | 374 | 4 |
| 6 | Human | MYH9 | 1920 | KLRRGDLPFV | 10 | 2.9 | 15 | 9.1 | 5.5 | - | 4 |
| | Human | MYH9 | 210 | QLLQANPIL | 9 | 4.5 | 183 | 1507 | 19 | - | 3 |
| | Human | MYH9 | 847 | MMAKEEELV | 9 | 9.1 | 1.2 | 276 | 1070 | 5639 | 3 |
| | Human | MYH9 | 877 | QLMAEKLQL | 9 | 8.2 | 7.9 | 205 | 657 | 4360 | 3 |
| | Human | MYH9 | 1726 | RLEARIAQL | 9 | 433 | 7.7 | 15 | 2292 | 46881 | 3 |
| | Human | MYH9 | 1793 | KLQEMEGTV | 9 | 23 | 99 | 48 | 867 | 19355 | 3 |
| | Human | MYH9 | 660 | TLRNTNPNFV | 10 | 21 | 107 | 40 | - | 593 | 3 |
| | Human | MYH9 | 688 | VLDQLRCNGV | 10 | 6.6 | 186 | 61 | 868 | - | 3 |
| | Human | MYH9 | 752 | NLYRIGQSKV | 10 | 322 | 87 | 76 | 17848 | 1024 | 3 |
| | Human | MYH9 | 248 | YIVGANIET | 9 | 422 | 622 | 9009 | 81 | 626 | 2 |
| | Human | MYH9 | 1540 | QLEELEDEL | 9 | 404 | 176 | - | 28866 | 4718 | 2 |
| | Human | MYH9 | 161 | MMQDREDQSI | 10 | 2.1 | 25 | 542 | 1245 | 4005 | 2 |
| | Human | MYH9 | 821 | KLRNWQWWRL | 10 | 56 | 54 | 1037 | 5442 | - | 2 |
| | Human | MYH9 | 846 | EMMAKEEELV | 10 | 33 | 9.6 | 958 | 2456 | 2809 | 2 |

| | | | | | | | | | | | |
|---|---|---|---|---|---|---|---|---|---|---|---|
| °=1^{st} amino acid position; §=IC50 indicates binding affinity and it is expressed as 50% inhibitory nanomolar concentration, a dash ("-") indicates an IC50 >50000 nM; peptides with IC50 <50nM are classified as high affinity peptides, peptides with IC50>50 and <500nM are classified as intermediate affinity peptides, peptides with IC50>500nM are classified as low affinity peptides; **=non muscle myosin. | | | | | | | | | | | |

**Table 13.HLA-A2 binding peptides derived from apoptotic cell-associated proteins appearing with their cleavage products in proteomic analysis.**

| | | | | | | **Binding capacity (IC50§ nM)** | | | | | **Alleles bound** |
|---|---|---|---|---|---|---|---|---|---|---|---|
| **Pool** | **Organism** | **Protein** | **1^{st} Pos^{o}** | **Sequence** | **Length** | **A*0201** | **A*0202** | **A*0203** | **A*0206** | **A*6802** | |
| 7 | Human | VIME% | 176 | NLAEDIMRL | 9 | 1.0 | 0.31 | 4.2 | 136 | 83 | 5 |
| | Human | VIME | 50 | SLYASSPGGV | 10 | 2.2 | 4.3 | 2.4 | 461 | 452 | 5 |
| | Human | VIME | 68 | RLRSSVPGV | 9 | 3.3 | 27 | 1.6 | 30 | 9901 | 4 |
| | Human | VIME | 129 | ILLAELEQL | 9 | 35 | 6.5 | 231 | 7.5 | - | 4 |
| | Human | VIME | 225 | SLQEEIAFL | 9 | 0.99 | 0.37 | 19 | 44 | 4777 | 4 |
| | Human | VIME | 78 | LLQDSVDFSL | 10 | 3.6 | 1.1 | 149 | 9.2 | 1931 | 4 |
| | Human | VIME | 79 | LQDSVDFSL | 9 | 5.9 | 47 | 10155 | 2.3 | 774 | 3 |
| | Human | VIME | 419 | SLNLRETNL | 9 | 402 | 1.4 | 22 | 16458 | - | 3 |
| | Human | VIME | 122 | FLEQQNKILL | 10 | 20 | 0.43 | 63 | 828 | 10669 | 3 |
| | Human | VIME | 370 | NMKEEMARHL | 10 | 260 | 433 | 703 | 10578 | 9346 | 2 |
| 8 | Human | PSA1*** | 179 | FMECNLNEL | 9 | 596 | 27 | 55 | 15067 | 14822 | 2 |
| | Human | PSA1 | 183 | NLNELVKHGL | 10 | 145 | 12 | 11 | 4834 | 3618 | 3 |
| | Human | PSA1 | 175 | HMSEFMECNL | 10 | 59 | 11 | 70 | 109 | 35360 | 4 |
| | Human | PSA1 | 63 | ILHVDNHIGI | 10 | - | - | 16720 | - | - | 0 |
| | Human | PSA1 | 37 | GLKSKTHAV | 9 | - | 4552 | 924 | - | 19982 | 0 |
| | Human | PSA1 | 110 | SLIGSKTQI | 9 | - | 15913 | 511 | 34100 | 17135 | 0 |
| | Human | PSA1 | 179 | FMECNLNELV | 10 | 12715 | 194 | 1 | - | - | 2 |
| | Human | PSA1 | 48 | ALKRAQSEL | 9 | - | - | 18952 | - | 29040 | 0 |
| | Human | PSA1 | 76 | GLTADARLL | 9 | 163 | 4.7 | 1 | 7.8 | 12 | 5 |
| | Human | PSA1 | 102 | PLPVSRLVSL | 10 | 4.5 | 1.2 | 0.21 | 40 | 1659 | 4 |
| | Human | PSA1 | 204 | DLTTKNVSI | 9 | 465 | 3.6 | 54 | 4278 | 7386 | 3 |
| | Human | PSA1 | 45 | ELNGKNIEDV | 10 | 104 | 1.2 | 0.21 | 1776 | 14417 | 3 |
| | Human | PSA1 | 55 | ELAAHQKKI | 9 | 38 | 2 | 1.1 | 100 | 9790 | 4 |
| 9 | Human | PSA1 | 186 | ELVKHGLRAL | 10 | 296 | 15 | 1.6 | 8411 | - | 3 |
| | Human | PSA1 | 37 | GLKSKTHAVL | 10 | 2366 | 116 | 24 | 10609 | 21672 | 2 |
| | Human | PSA1 | 191 | GLRALRETL | 9 | 41760 | 45 | 23 | - | - | 2 |
| | Human | PSA1 | 55 | ELAAHQKKIL | 10 | 8856 | 146 | 362 | 16700 | - | 2 |
| | Human | PSA1 | 97 | FVFDRPLPV | 9 | - | 8417 | 2175 | 9732 | 44603 | 0 |

| | | | | | | | | | | | |
|---|---|---|---|---|---|---|---|---|---|---|---|
| °=1st amino acid position; §=IC50 indicates binding affinity and it is expressed as 50% inhibitory nanomolar concentration, a dash ("-") indicates an IC50 >50000 nM; peptides with IC50 <50nM are classified as high affinity peptides, peptides with IC50>50 and <500nM are classified as intermediate affinity peptides, peptides with IC50>500nM are classified as low affinity peptides; %=vimentin; ***PSA1= Proteasome component C2. | | | | | | | | | | | |

**Table 14. HLA class I binding capacity of potential HCV epitopes (Genotype 1b)**

| **Pool** | **Organism** | **Protein** | **1st Pos°** | **Sequence** | **SEQ ID No.** | **Length** |
|---|---|---|---|---|---|---|
| 1 | Human | HCV Core protein | 35 | YLLPRRGPRL | 253 | 10 |
| | Human | HCV Core protein | 132 | DLMGYIPLV | 254 | 9 |
| | Human | HCV Core protein | 177 | FLLALLSCL | 255 | 9 |
| | Human | HCV Core protein | 177 | FLLALLSCLT | 256 | 10 |
| | Human | HCV Core protein | 180 | ALLSCLTTPA | 257 | 10 |
| | Human | HCV Core protein | 181 | LLSCLTTPA | 258 | 9 |
| 2 | Human | E1 protein | 322a | MMMNWSPTT | 259 | 9 |
| | Human | E1 protein | 322b | MMMNWSPTTA | 260 | 10 |
| | Human | E1 protein | 362 | YSMAGNWAKV | 261 | 10 |
| | Human | E1 protein | 371 | VLIVMLLFA | 262 | 9 |
| | Human | E1 protein | 372 | LIVMLLFAGW | 263 | 10 |
| | Human | E2 protein | 614 | RLWHYPCTV | 264 | 9 |
| | Human | E2 protein | 665 | LLLSTTEWQV | 265 | 10 |
| | Human | E2 protein | 666 | LLSTTEWQV | 266 | 9 |
| 3 | Human | E2 protein | 701 | YLYGIGSAV | 267 | 9 |
| | Human | E2 protein | 701 | YLYGIGSAVV | 268 | 10 |
| | Human | E2 protein | 718 | YVLLLFLLLA | 269 | 10 |
| | Human | E2 protein | 719 | VLLLFLLLA | 270 | 9 |
| | Human | E2 protein | 723 | FLLLADARV | 271 | 9 |
| | Human | E2 protein | 736 | WMMLLIAQA | 272 | 9 |
| | Human | E2 protein | 738 | MLLIAQAEA | 273 | 9 |
| 4 | Human | NS2 | 838 | FLARLIWWL | 274 | 9 |
| | Human | NS2 | 875 | LLMCAVHPEL | 275 | 10 |
| | Human | NS2 | 890 | KLLIAILGPL | 276 | 10 |
| | Human | NS2 | 891 | LLIAILGPL | 277 | 9 |
| | Human | NS2 | 901 | VLQAGITRV | 278 | 9 |
| | Human | NS2 | 917 | GLIHACMLV | 279 | 9 |
| | Human | NS2 | 940 | KLGALTGYI | 280 | 10 |
| | Human | NS2 | 948 | YIYNHLTPL | 281 | 9 |
| 5 | Human | NS3 | 1038 | GLLGCIITSL | 282 | 10 |
| | Human | NS3 | 1039 | LLGCIITSL | 283 | 9 |
| | Human | NS3 | 1069 | FLATCVNGV | 284 | 9 |
| | Human | NS3 | 1406 | KLSGLGINAV | 285 | 10 |
| | Human | NS3 | 1542 | YLNTPGLPV | 286 | 9 |
| | Human | NS3 | 1585 | YLVAYQATV | 287 | 9 |
| | Human | NS3 | 1606 | QMWKCLIRL | 288 | 9 |

**Table 15. HLA class I binding capacity of potential HCV epitopes (Genotype 1b)**

| **Pool** | **Organism** | **Protein** | **1st Pos°** | **Sequence** | **SEQ ID No.** | **Length** |
|---|---|---|---|---|---|---|
| 6 | Human | NS4A | 1661 | VLVGGVLAAL | 289 | 10 |
| | Human | NS4B | 1764 | HMWNFISGI | 290 | 9 |
| | Human | NS4B | 1768 | FISGIQYLA | 291 | 9 |
| | Human | NS4B | 1789 | SLMAFTASI | 292 | 9 |
| | Human | NS4B | 1807 | LLFNILGGWV | 293 | 10 |
| | Human | NS4B | 1851 | ILAGYGAGV | 294 | 9 |
| 7 | Human | NS5A | 1998 | KLLPQLPGV | 295 | 9 |
| | Human | NS5A | 2077 | RLIVFPDLGV | 296 | 10 |
| | Human | NS5A | 2093 | ALYDVVSTL | 297 | 9 |
| | Human | NS5B | 2726 | KLQDCTMLV | 298 | 9 |
| | Human | NS5B | 3732 | MLVNGDDLVV | 299 | 10 |
| | Human | NS5B | 2827 | WLGNIIMYA | 300 | 9 |
| | Human | NS5B | 2842 | MILMTHFFSI | 301 | 10 |
| 8 | Human | NS5B | 2843 | ILMTHFFSI | 302 | 9 |
| | Human | NS5B | 2843 | ILMTHFFSIL | 303 | 10 |
| | Human | NS5B | 2861 | ALDCQIYGA | 304 | 9 |
| | Human | NS5B | 2884 | RLHGLSAFSL | 305 | 10 |
| | Human | NS5B | 2991 | FMLCLLLLS | 306 | 9 |
| | Human | NS5B | 299 | FMLCLLLLSV | 307 | 10 |
| | Human | NS5B | 2992 | MLCLLLLSV | 308 | 9 |
| | Human | NS5B | 2995 | LLLLSVGVGI | 309 | 10 |
| | Human | NS5B | 2997 | LLSVGVGIYL | 310 | 10 |

**Table 16. HLA class I binding capacity of potential HCV epitopes (Genotype 2c)**

| **Pool** | **Organism** | **Protein** | **1th Pos°** | **Sequence** | **SEQ ID No.** | **Length** |
|---|---|---|---|---|---|---|
| 1 | Human | HCV Core protein | 177 | FLLALLSCI | 311 | 9 |
| | Human | HCV Core protein | 178 | LLALLSCISV | 312 | 10 |
| | Human | HCV Core protein | 180 | ALLSCISVPV | 313 | 10 |
| | Human | HCV Core protein | 181 | LLSCISVPV | 314 | 9 |
| 2 | Human | E₁ protein | 283 | ALMIAAQVVV | 315 | 9 |
| | Human | E₁ protein | 332 | MLLAYLVRI | 316 | 10 |
| | Human | E₁ protein | 355 | VMFGLAYFSM | 317 | 10 |
| | Human | E₁ protein | 362 | FSMQGAWAKV | 318 | 9 |
| | Human | E₂ protein | 397 | HLFTSMFSL | 319 | 10 |
| | Human | E₂ protein | 437 | FLAALFYTS | 320 | 9 |
| | Human | E₂ protein | 705 | YLYGLSPAI | 321 | 10 |
| 3 | Human | p27 | 768 | GLLYFILFFV | 322 | 10 |
| | Human | p27 | 769 | LLYFILFFV | 323 | 9 |
| | Human | p27 | 769 | LLYFILFFVA | 324 | 10 |
| | Human | p27 | 792 | YTLLGCWSFV | 325 | 10 |
| | Human | p27 | 793 | TLLGCWSFV | 326 | 9 |
| | Human | p27 | 793 | TLLGCWSFVL | 327 | 10 |
| | Human | p27 | 803 | LLMALPHQA | 328 | 9 |
| 4 | Human | NS2 | 826 | LLIAITAF | 329 | 9 |
| | Human | NS2 | 841 | ILLSRCLWWT | 330 | 10 |
| | Human | NS2 | 846 | CLWWTCYMLV | 331 | 10 |
| | Human | NS2 | 851 | YMLVLAEALI | 332 | 10 |
| | Human | NS2 | 859 | LIQDWIPPL | 333 | 9 |
| | Human | NS2 | 880 | AMTMFYPGV | 334 | 9 |
| | Human | NS2 | 1001 | ALLRMCALV | 335 | 9 |
| | Human | NS2 | 1027 | RLLAPITAYA | 336 | 10 |
| | Human | NS2 | 1028 | LLAPITAYA | 337 | 9 |
| 5 | Human | NS3 | 1073 | FLGTSISGV | 338 | 9 |
| | Human | NS3 | 1076 | TSISGVLWTV | 339 | 10 |
| | Human | NS3 | 1077 | SISGVLWTV | 340 | 9 |
| | Human | NS3 | 1081 | VLWTVFHGA | 341 | 9 |
| | Human | NS3 | 1135 | YLVTRNADV | 342 | 9 |
| | Human | NS3 | 1610 | VMWKCLIRL | 343 | 9 |

**Table 17. HLA class I binding capacity of potential HCV epitopes (Genotype 2c)**

| **Pool** | **Organism** | **Protein** | **1th Pos°** | **Sequence** | **SEQ ID No.** | **Length** |
|---|---|---|---|---|---|---|
| 6 | Human | NS4A | 1665 | VLAGGVLAAV | 344 | 10 |
| | Human | NS4B | 1793 | SMMSFSAAL | 345 | 9 |
| | Human | NS4B | 1855 | VLAGYGAGI | 346 | 9 |
| | Human | NS4B | 1871 | KIMSGEKPSV | 347 | 10 |
| 7 | Human | NS5A | 2002 | KLFPRLPGI | 348 | 9 |
| | Human | NS5A | 2144 | FMRDEVSFSV | 349 | 10 |
| | Human | NS5A | 2254 | VLMVDSFDPV | 350 | 10 |
| | Human | NS5A | 2255 | LMVDSFDPV | 351 | 9 |
| | Human | NS5B | 2505 | LLDSHYESV | 352 | 9 |
| | Human | NS5B | 2604 | RLIVYPDLGV | 353 | 10 |
| | Human | NS5B | 2734 | SMGNTLTCYV | 354 | 10 |
| | Human | NS5B | 2759 | MLVCGDDLVV | 355 | 10 |
| | Human | NS5B | 2869 | MVLMTHFFSV | 356 | 10 |
| 8 | Human | NS5B | 2870 | VLMTHFFSV | 357 | 9 |
| | Human | NS5B | 2870 | VLMTHFFSVL | 358 | 10 |
| | Human | NS5B | 291 | RLHGLEAFSL | 359 | 10 |
| | Human | NS5B | 2989 | RLLDLSSWFT | 360 | 10 |
| | Human | NS5B | 3016 | RLLLLGLLLL | 361 | 10 |
| | Human | NS5B | 3017 | LLLLGLLLL | 362 | 9 |
| | Human | NS5B | 3018 | LLLGLLLLCV | 363 | 10 |
| | Human | NS5B | 3019 | LLGLLLLCV | 364 | 9 |
| | Human | NS5B | 3021 | GLLLLCVGV | 365 | 9 |

The synthetic apoptotic peptides used were prepared according to the sequence of caspase-cleaved proteins that had been previously identified by the proteomic analyses of apoptotic T cells (i.e., fragments of actin cytoplasmic 1 [ACTB], heterogeneous nuclear ribonucleo protein [ROK], lamin B1 [LAM1], non muscle myosin heavy chain 9 [MYH9], vimentin [VIME], or proteasome component C2 [PSA1]) (14). The author found a significantly larger repertoire of IFN-γ⁺CD8⁺ T_{EM} cells (as detected by ELISPOT) specific to multiple apoptotic epitopes in acutely HCV infected patients undergoing chronic evolution than in patients with self-limited disease (**Figure 1A** **and** **Figures 2 to 18**). By contrast, the repertoire of IFN-γ⁺CD8⁺ T_{EM} cells specific to multiple viral epitopes was wider in patients undergoing recovery (**Figure 1B** **and** **Figures 2****-18**). In addition, the apoptotic epitope repertoire recognized by CD8+ Tcells was significantly larger in patients undergoing chronic infection than in those undergoing recovery (data not shown).

Interestingly, the mean number of IFN-γ spots promptly formed by CD8⁺ T_{EM} cells in response to each pool of apoptotic epitopes (but not viral epitopes) was directly correlated with the viral (plasma HCV-RNA) load, thus supporting the relationship between these responses and chronic evolution (**Figure 1C**). None of the 21 HLA-A2⁺ healthy donors exhibited significant effector responses against any of the apoptotic or viral peptides *ex vivo.* The HLA-restriction of these responses was demonstrated both by blocking responses with an appropriate anti-class I mAb and by determining that no response was observed in HLA-A2- patients.

### Mixed polyfunctional apoptotic epitope-specific CD8⁺ T cells in chronic HCV evolution

The author enumerated specific CD8⁺ T cells directly in the peripheral blood of patients by using pentamers of HLA-A*0201 molecules complexed to either apoptotic (MYH9₄₇₈₋₄₈₆, MYH9₇₄₁₋₇₄₉, VIME₇₈₋₈₇) or viral epitopes (NS3₁₀₇₃₋₁₀₈₁, NS3₁₄₀₆₋₁₄₁₅, Core₁₃₂₋₁₄₀) that had been previously identified as the most immunogenic among all patients tested (**Figure 1D**). The background pentamer values were <0.02% (mean ±s.d. = 0.0173% ± 0.0009% for all pentamers complexed to apoptotic epitopes; 0.0052% ± 0.0035% for those complexed to viral epitopes) in 20 HLA-A2⁺ healthy individuals; thus 0.02% was considered the sensitive level of the assay. Control HLA-A*0201 pentamers complexed to a non-natural irrelevant peptide were unable to stain CD8⁺ T cells in all PBMCs tested. The total frequencies of either apoptotic or viral epitope-specific CD8⁺ T cells, as detected with pentamers, did not differ between patients undergoing chronic or recovery evolution at each time point tested (**Figure 1D-F**), but they were substantially higher than those of IFN-γ⁺CD8⁺T_{EM} cells that had been previously detected by ELISPOT assay. To determine whether additional subsets with different effector functions were present within the total CD8⁺pentamer⁺ + T cells, the author analyzed the frequencies of freshly isolated CD8⁺pentamer⁺ T cells that produced a wide array of cytokines (IL-17, IFN-g IL-4, IL-2 within a few h of contact with the relevant peptides and optimal concentrations of anti-CD28 mAb, which served as a surrogate costimulatory signal. Irrelevant cytokine production (<0.1% cells) was observed when either apoptotic epitope- or viral epitope-specific CD8⁺pentamer⁺ T cells of 20 HLA-A2⁺ healthy individuals were stimulated with this procedure; thus, the author considered 1% cytokine-producing cells in pentamer⁺ cells as the sensitive level of this assay. Importantly, apoptotic epitope-specific CD8⁺pentamer⁺ T_{EM} cells promptly produced notable and sustained amounts of all the cytokines tested within a few hours of contact with the relevant epitopes, much more in patients experiencing chronic infection than in those undergoing infection resolution (**Figure 19A- C**). Peptide dose-response curves of cytokine-producing CD8⁺pentamer⁺ cells emphasized this difference (**Figure 20** **A,B**). The polycytokine production by apoptotic epitope-specific CD8⁺ pentamer⁺ T_{EM} cells was significantly higher in patients experiencing chronic infection at the 6^{th} month after the clinical onset (**Figure 19C**), and tended to be higher also in the other time points tested (**Figure 20C**). Compared with the apoptotic epitope- specific CD8⁺ T_{EM} cells, the virus-specific CD8⁺pentamer⁺ T_{EM} cells produced lower amounts of the same cytokine in both categories of patients without any differences between them (**Figure 19A**,**B**, **Figure 20A****,B** and **Figure 21****).** Time course analyses, performed longitudinally throughout the follow-up, revealed that the frequencies of polyfunctional apoptotic epitope-specific CD8⁺ T_{EM} cells (calculated in terms of their absolute number) were sustained over time in relation to the sustained viral load (HCV- RNA copies, sign of viral persistence) and the progressive decline of alanineaminotransferase (ALT) levels (sign of the termination of acute infection) only in patients who evolved into chronic infection (**Figure 22A,B****).** Then, the majority of these cell frequencies tended to decline considerably later in patients who evolved into chronic infection than in those resolving infection (**Figure 22B**). By contrast, no substantial difference was confirmed in the time course of the virus-specific effector response between the two categories of patients: the virus-specific CD8⁺ T_{EM} cell frequencies were substantially lower than the apoptotic epitope-specific and decreased over time (**Figure 22C**). Notably, the polyfunctional responses in the majority of patients were maintained by the parallel presence of different antigen-specific CD8⁺ T cell subsets, each of which produced a single cytokine in all time-points tested (mixed polyfunctional populations) (**Figure 23A**,**B**). By contrast, 4 out of the 16 patients studied also showed cells simultaneously producing more than one cytokine: in particular, 4 patients showed cells simultaneously producing significant amounts of IFN-γ and IL-17, and two of these 4 patients showed cells simultaneously producing significant amounts of IL-17 and IL-4 (**Figure 19A**,**B** and **Figure 23A**,**B**). Importantly, the frequencies of CD8⁺pentamer⁺ T_{EM} cells promptly producing IFN-γ or IL-17 in response to the relevant apoptotic epitopes (**Figure 24A,B****),** but not to the viral epitopes, tended to be directly correlated with the plasma viral load or the serum ALT levels.

It is for interest the finding that fresh apoptotic epitope-specific CD8⁺pentamer⁺ T_{EM} cells promptly produced IFN-γ or IL-17 *ex vivo* within a few hours of contact with DCs that had been pulsed with apoptotic T cells (i.e., through the cross-presentation mechanism) (**Figure 25A****,****B****).** The cross-presentation resulted in a marked decrease in IFN-γ or IL-17 production when apoptotic cells had been previously treated with a selective caspase-3 inhibitor (C3I) (**Figure 25A****,****B****).** This phenomenon was confirmed in five independent patients (**Figure 25B**). The frequencies of apoptotic epitope-specific CD8⁺ T cells (but not those of viral epitope-specific CD8⁺ T cells [data not shown]) correlated with the number of circulating apoptotic T cells (**Figure 25C**). The percentage of apoptotic T cells in PBMCs was significantly higher in patients than in the 20 healthy donors tested (11.0 ± 7.7 versus 3.9 ± 3.9; *P<* .001). To understand why the self-epitope-specific cells of patients undergoing resolution display significantly lower polyfunctional functions than patients experiencing chronic infection (**Figure 19A-D** and **Figure 20**), despite the similar frequency of total CD8⁺pentamer⁺ cells (**Figure 1E**,**F**), the author performed a series of functional experiments. First, the author ruled out the possibilities that apoptotic epitope-specific CD8⁺ T cells from the recovered patients expressed an intrinsic defect of effector cell functions, or were derived from the naive CD8⁺ T cell repertoire (**Figure 26** **and** **27**).

### Linking TCR avidity of apoptotic epitope-specific CD8⁺ T cells to infection outcome

Finally, the author hypothesized that differences in TCR avidity might account for the different apoptotic epitope-specific CD8⁺ T cell responsiveness between patients experiencing chronic infection and those undergoing infection resolution. To assess TCR avidity, the author evaluated the dissociation kinetics of peptide/HLA-A*0201 pentamer binding to antigen-specific CD8⁺ T cells that were isolated from the two groups of patients (28). Specifically, the author stained fresh CD8⁺ T cells with saturating amounts of HLA A*0201 pentamers that were complexed to either apoptotic or viral epitopes and an anti- CD8 mAb for 45 minutes at room temperature. Cells were then washed and incubated at 4°C with saturating amounts of an anti-HLA-A2 mAb to prevent rebinding of pentamers during the pentamer dissociation assay. The rate of decay was measured by flow cytometry at appropriate time points. The author obtained linear decay plots of the natural logarithm of the normalized fluorescence versus time in all experiments performed, indicating that the pentamer decay was occurring stochastically and that the resulting pentamer staining *t*_{1/2} should be proportional to the *t*_{1/2} of respective pentamer/TCR complexes (**Figure 28A****).**

The *t*_{1/2} for apoptotic epitope-complexed pentamer staining to fresh CD8⁺ T cells from patients experiencing chronic infection was significantly longer than the decay of pentamer staining to CD8⁺ T cells from patients undergoing infection resolution (**Figure 28A,B****).** By contrast, the *t*_{1/2} for viral epitope-complexed pentamers to CD8⁺ T cells did not differ between patients experiencing chronic infection and those undergoing infection resolution (**Figure 28A,B****).** The *t*_{1/2} for apoptotic epitope-complexed pentamer staining to CD8⁺ T cells tended (although not significantly) to be directly correlated with the viral load (**Figure 29**). Finally, control experiments in which HLA-A*0201 pentamers were complexed to a non-natural irrelevant peptide showed that the *t*_{1/2} for staining to CD8⁺ T cells was undetectable (data not shown).

### DISCUSSION

In the present invention, the author demonstrates for the first time that the emergence of mixed polyfunctional (type-1, -2, -17) CD8⁺ T_{EM} cell responses to apoptotic self-epitopes is related to the chronic evolution of an acute infection. Indeed, the multispecificity, magnitude, and polyfunctional strength of CD8⁺ T_{EM} cell responses directed to apoptotic self-epitopes were amazingly wide and robust during the acute phase of HCV infection, particularly in patients experiencing chronic progression compared with those undergoing infection resolution. The responses were then sustained over time in relation to viral persistence, suggesting that they can contribute to the immunopathology and chronic evolution of acute HCV infection. This hypothesis is further supported by the evidence of a direct correlation between the frequencies of CD8⁺pentamer⁺ T_{EM} cells promptly producing IFN-γ or IL-17 in response to the relevant apoptotic epitopes, and the plasma viral load or the serum ALT levels (which represent the most important clinical sign of liver damage). Recently, several models of chronic viral infection demonstrated that virus-specific CD4⁺ or CD8⁺ T cells producing elevated levels of IL-17 correlated with either viral persistence or a wasting syndrome with a multiple organ neutrophil infiltration (7, 29, 30). Currently, the author's data suggest that the emergence of high frequencies of mixed autoreactive CD8⁺ T cells producing a broad array of cytokines (including IL-17) rather than the dysfunction or altered quality of virus-specific CD8⁺ T cells is related to the progression toward chronic disease in the human model of acute HCV infection (31-35). Indeed, the author found that the frequencies of virus-specific effector cells (producing the different cytokines analyzed) were extremely low as compared with the apoptotic epitope-specific.

This is consistent with the majority of studies revealing that the magnitude of HCV-specific CD8 T cell effector responses does not correlate with the clinical or viral outcome in acute HCV infection (Refs in 35). In particular, HCV-specific CD8 T cells have been reported to express a dysfunctional phenotype (weak proliferation, IFN-γ production, and cytotoxicity) and increased levels of PD-1, known to be associated with the exhausted phenotype, irrespective of infection progression (Refs in 35). Polycytokine (IL-17, IFN-γ, IL-2, and IL-4) production by autoreactive CD8⁺ T_{EM} cells may have crucial implications in HCV-related immunopathology, which stresses the importance of infections in inducing and maintaining autoimmunity (36). IL-17 production can account for the transient intra-hepatic infiltration of neutrophils found only in the early phase of acute HCV infection (37). Then, the timely decline in hepatocyte damage and the consequent evolution toward a state of chronic low-level liver disease, in patients developing viral persistence, may be achieved through the simultaneous presence of autoreactive type-1,-2, and -17 CD8⁺ TE_{M} cells, which may regulate each other, and even the capacity of type-17 CD8⁺ T_{EM} cells to express a certain degree of plasticity (2, 21-24), and to convert to CD8⁺ T_{EM} cells with combined features of type-17 and -1 cells. Therefore, the environmental setting during an acute inflammatory disease seems to be addressed to guarantee the coexisting polarization of type-1, -2, and -17 CD8⁺ T_{EM} cells.

This event is likely required to limit excessive host tissue injury by fine-polarized type-1 or type-17 autoreactive CD8⁺ T_{EM} cells, despite the polyfunctional responses override those virus-specific ones, and can provide chronic immunopathology in a state of viral persistence. In this context, it is intriguing the observation that PD-1 expression (27) is not correlated with the exhaustion of the autoreactive CD8⁺ T cells, since they continue to produce a vast array of cytokines *ex vivo,* and they are limited only partially by the inhibitory PD-1 capacity *in vitro.* A possible explanation of this is that these autoreactive CD8⁺ T cells might be less susceptible of the exhaustion, likely because they are primed later than the virus-specific, that in contrast show a profound exhaustion phenotype in patients with acute or chronic hepatitis (27, 33, 35). The PD-1-dependent tuning of autoreactive CD8⁺ T_{EM} cell functions, as well as the high frequencies of apoptotic epitope-specific CD8⁺ T_{EM} cells producing IL-4, may play a pivotal role in limiting excessive functional responses, thus allowing the evolution toward chronic low-level inflammatory liver disease (6, 38-40). In support of this hypothesis, our parallel study in patients with long-term chronic HCV infection demonstrates that CD8⁺ T_{EM} cells specific to apoptotic self-epitopes are compartmentalized in the inflamed liver, where they produce levels of IFN-γ and IL-2 that are significantly lower than in patients with acute hepatitis, as well as very low or undetectable levels of IL-17. In the light of this evidence, the author can reasonably assume that the mixed polyfunctional autoreactive CD8⁺ T_{EM} cells should provoke a robust auto-inflammatory response in the liver through the by-stander effect of the pro-inflammatory cytokines in patients with acute HCV infection, despite difficulty to readily obtain access to liver-infiltrating T cells from patients with acute hepatitis for obvious ethical reasons. This acute response does not seem to threaten the host survival, likely because of the various controlling mechanisms mentioned above, and it progressively undergoes partial exhaustion/contraction in patients with long-term chronic hepatitis.

Several forms of inflammatory diseases (14, 41), including HCV infection (42) correlate with tremendous polyclonal hyper-reactivity of T and B cells (systemic immune activation), which results in a continuous turnover of apoptotic T and B cells (42). In previous studies, the author provided evidence that caspase cleavage permits the release of self-antigenic fragments from apoptotic cells and the subsequent cross-presentation of the relevant processed peptides to a huge number of autoreactive CD8⁺ T cells (14). The observation that cross-presentation of apoptotic T cells by DCs promptly activates CD8⁺ T_{EM} cells specific for caspase-cleaved apoptotic self-epitopes *ex vivo* indicates that this mechanism might be operative in the induction of the resulting polyfunctional autoreactive responses in patients with acute HCV infection who are experiencing chronic progression. This form of systemic autoimmune activation ultimately correlates with the evolution of infection from acute to chronic and can participate in the liver immunopathology through the by-stander effect of the pro-inflammatory cytokines. An important facet of the present findings is that they demonstrate a link between the TCR avidity of autoreactive CD8⁺ T cells and the difference in the responsiveness of apoptotic epitope-specific CD8⁺ T cells exhibited by patients experiencing chronic infection and those undergoing infection resolution. The dissociation kinetics of peptide/HLA-A*0201 pentamer binding to antigen-specific CD8⁺ T cells clearly demonstrated that the *t*_{1/2} for apoptotic epitope-complexed pentamer staining to CD8⁺ T cells from patients experiencing chronic infection was significantly longer than the decay of pentamer staining from patients undergoing infection resolution. The *t*_{1/2} for pentamer staining was detected on freshly isolated CD8⁺ T cells, suggesting that TCR avidity measured by this system likely reflects what occurs *in vivo.* In the original study, this methodological approach made it possible to postulate that T cells with TCRs that bind peptide/MHC complexes for a longer duration are selectively preserved in comparison to T cells that express TCRs with lower avidity (28). Recent studies suggested that in response to different microbial infections, initially naive T cells with a wide range of avidity are efficiently recruited and expanded (43, 44); subsequently, those with lower avidity undergo premature contraction, whereas those with higher avidity are selected because of a more prolonged expansion and correlate with protection (43, 44). The author's results provide an additional challenge to this model, and suggest that autoreactive CD8⁺ T cells with higher avidity for apoptotic self-epitopes are sustained over time and correlate with the progression toward chronic infection. The selection of the autoreactive CD8⁺T cells with higher avidity likely occurs because of a stronger stimulation by apoptotic antigens (14). By contrast, lower avidity CD8⁺ T cells in the presence of weaker stimuli would undergo rapid contraction, as seen in the peripheral blood of patients with self-limited HCV infection. The level of the initial viral load, which the author found to be linked with the level of TCR avidity, may provide the conditions that influence the availability of strong apoptotic antigenic stimuli. Moreover, our model does not exclude the possibility that cross-reactive CD8⁺ T cells, even expressing dual TCR (45), may intervene in this process.

In conclusion, the author's data demonstrate that autoreactive CD8 T cell responses against apoptotic T cell-associated epitopes are strictly dependent on the viral persistence and correlate with immunopathology via the exuberant production of inflammatory cytokines. This model represents a remarkable example of the importance of infections in inducing and particularly maintaining autoimmunity. In addition, our study provides a possible explanation for why the enormous expansion of activated T cells, during persisting viral infections, is only minimally attributable to virus-specific T cells (14). The author can envisage the following multistep model. In the conditions of HCV immune evasion, continuous waves of dysfunctional virus-specific T cells are generated, and can deliver the initial apoptotic antigenic stimuli for priming apoptotic antigen-specific T cells.

Then, the latter can perform their pro-inflammatory functions through the production of a vast array of cytokines, undergo apoptosis, become themselves a new substrate for the selection of apoptotic antigen-specific T cells with high TCR avidity, and so on. In the long run, this vicious spiral may contribute to the irreversible outcome toward the chronic liver disease. By contrast, it would not take place in patients resolving infection, because the virus is efficiently cleared by the protective immune responses, and the dramatic contraction of the latter would not allow the establishment of the pre-requisites (previously illustrated) required to initiate and to sustain apoptotic antigen-specific high avidity T cell responses. To investigate if these responses are however capable to auto-maintain themselves irrespective of the viral presence in particular circumstances, additional studies are in progress to verify if they disappear in HCV-infected patients responding to the antiviral therapy, or persist in that minority of patients continuing to show liver inflammation, despite the viral clearance.

Finally, the author's results have clinical implications. They provide an important platform for the design of innovative therapeutic strategies to re-engineer protective immune responses in persisting infections. Polyfunctional CD8⁺ T cells that are specific to apoptotic epitopes could predict chronic infection in other acute (i.e., HBV or HIV) infections that develop viral persistence. As a consequence, antiviral therapies can be administered earlier so as to improve viral clearance rates of infected patients who are expected to enter a chronic infection state (46, 47). The detection of these autoreactive CD8⁺ T cells may also be relevant in determining whether the contraction or the quality variation of the polyfunctional responses can be used as biomarkers to verify the protective effects of conventional or innovative antiviral therapies.

### BIBLIOGRAPHIC REFERENCES

1. Bettelli E, et al., Nature. 2008;453(7198):1051-1057.
2. Zhou L, Chong MM, and Littman DR. Immunity. 2009;30(5):646-655.
3. Zhou L, and Littman DR. Curr Opin Immunol. 2009;21(2):146-152.
4. Aujla SJ, et al. Nat Med. 2008;14(3):275-281.
5. Lin Y, et al. Immunity. 2009;31(5):799-810.
6. Prezzi C, et al., Eur J Immunol. 2001;31(3):894-906.
7. Intlekofer AM, et al., Science. 2008;321(5887):408-411.
8. Yoo JK, et al., J Exp Med. 2010;207(7):1435-1451.
9. Sallusto F, et al., Nature. 1999;401(6754):708-712.
10. Sallusto F, et al., Annu Rev Immunol. 2000;18593-620.
11. Ahmed R, et al., Nat Rev Immunol. 2009;9(9):662-668.
12. Green DR, et al., Nat Rev Immunol. 2009;9(5):353-363.
13. Ravichandran KS. J Exp Med. 2010;207(9):1807-1817.
14. Rawson PM, et al. Nat Med. 2007;13(12):1431-1439.
15. Pang B, et al., J Immunol. 2009;183(2):1083-1090.
16. Lopez D, et al., J Immunol. 2010;184(9):5193-5199.
17. Rock KL, et al., J Immunol. 2010;184(1):9-15.
18. Propato A, et al., Nat Med. 2001;7(7):807-813.
19. Gurung P, et al., J Immunol. 2009;183(10):6114-6123.
20. Wentworth PA, et al., Int Immunol. 1996;8(5):651-659.
21. Bending D, et al., J Clin Invest. 2009.
22. Curtis MM, et al., J Immunol. 2009;183(1):381-387.
23. Mukasa R, et al., Immunity. 2010;32(5):616-627.
24. Yen HR, et al. J Immunol. 2009;183(11):7161-7168.
25. Acosta-Rodriguez EV, et al., Nat Immunol. 2007;8(6):639-646.
26. Acosta-Rodriguez EV, et al., Nat Immunol. 2007;8(9):942-949.
27. Sharpe AH et al., Nat Immunol. 2007;8(3):239-245.
28. Savage PA et al., Immunity. 1999;10(4):485-492.
29. Hou W et al., J Exp Med. 2009;206(2):313-328.
30. Zhang JY et al., Hepatology. 2010;51(1):81-91.
31. Francavilla V et al., Eur J Immunol. 2004;34(2):427-437.
32. Urbani S et al., Hepatology. 2006;44(1):126-139.
33. Urbani S et al., J Virol. 2006;80(22):11398-11403.
34. Kaplan DE, et al., Gastroenterology. 2007;132(2):654-666.
35. Rehermann B. J Clin Invest. 2009;119(7):1745-1754.
36. Wu HJ et al., Immunity. 2010;32(6):815-827.
37. Johnson K et al., Am J Surg Pathol. 2007;31(11):1754-1758.
38. Accapezzato D et al., J Clin Invest. 2004;113(7):963-972.
39. Barnaba V. J Hepatol. 2010;53(4):752-761.
40. Franceschini D et al., J Clin Invest. 2009; 119(3):551-564.
41. Bangs SC et al., Trends Immunol. 2006;27(11):518-524.
42. Nuti S et al., Eur J Immunol. 1998;28(11):3448-3455.
43. Zehn D et al. Nature. 2009;458(7235):211-214.
44. van Heijst JW et al., Science. 2009;325(5945):1265-1269.
45. Ji Q et al., Nat Immunol. 2010;11(7):628-634.
46. Kitahata MM, et al., N Engl J Med. 2009;360(18):1815-1826.
47. Dore GJ et al., Gastroenterology. 2010;138(1):123-135 e121-122.
48. Barnaba V Nature. 1990;345(6272):258-260.

## Claims

1. A method to monitor the progress of a viral infection and/or to monitor the efficacy of a therapy for a viral infection in a biological sample comprising T cell obtained from a subject affected by said viral infection comprising the steps of:
a) incubating the isolated biological sample with at least one peptide consisting of a sequence selected from SEQ ID No.1 to SEQ ID No. 252 under appropriate conditions to generate a T cell response;
b) measuring the T cell response;
c) comparing the measured T cell response to the measured T cell response of a suitable control sample,
wherein said viral infection is caused by a virus selected from the group of HCV, HBV, thereby discriminating patients experiencing chronic infection and those undergoing infection resolution.

2. The method according to claim 1 wherein the biological sample is a sample of PBMCs.

3. The method according to any one of previous claims wherein the at least one peptide of step a) consists of the sequence selected from LLQDSVDFSL (SEQ ID No. 204), QLFNHTMFI (SEQ ID No. 64) or VLMIKALEL (SEQ ID No. 78).

4. The method according to any one of previous claim wherein step b) comprises incubating the biological sample with a pool of peptides selected among the following pools:
- pool 1: SEQ ID Nos. 1, 2, 4, 5, 8 and 9;
- pool 2: SEQ ID Nos. 14, 16, 18, 19 and 20;
- pool 3: SEQ ID Nos.27, 28, 29, 30, 34, 35, 36, 37 and 40;
- pool 4: SEQ ID Nos. 58, 59, 60, 62, 63, 64, 65, 66 and 67;
- pool 5: SEQ ID Nos. 68, 69, 73, 74, 75, 76, 77, 78, 81, 83, 84 and 88;
- pool 6: SEQ ID Nos. 89, 90, 91, 92, 99, 100, 104, 105, 106, 111, 117, 123, 126 and 127;
- pool 7: SEQ ID Nos. 198, 199, 200, 201, 202, 204, 206, 210, 211 and 220;
- pool 8: SEQ ID Nos. 232, 233, 234, 235, 236, 237, 238, 239, 240, 241, 242, 243 and 244 or
- pool 9: SEQ ID Nos. 245, 246, 247, 248 and 249.

5. The method according to any one of previous claims wherein the T cell response is a CD8⁺ T cell response.

6. The method according to any one of previous claims wherein the T cell response is measured by means of an Elispot assay.

7. The method according to claim 6 wherein the Elispot assay is an interferon-gamma, IL-17 or IL-4 Elispot assay.

8. A method to monitor the progress of a viral infection and/or to monitor the efficacy of a therapy for a viral infection in a subject affected by said viral infection comprising the step of detecting and/or quantifying the amount of auto-antibodies directed against at least one peptide as defined in any one of claim 1, 3 or 4, wherein said viral infection is caused by a virus selected from the group of HCV, HBV, thereby discriminating patients experiencing chronic infection and those undergoing infection resolution.

## Patentansprüche

1. Verfahren zur Überwachung des Voranschreitens einer Virusinfektion und/oder zur Überwachung der Wirksamkeit einer Therapie für eine Virusinfektion in einer biologischen Probe, die T-Zellen umfasst, die von einem Subjekt erhalten wurden, das an der Virusinfektion leidet, wobei das Verfahren folgende Schritte umfasst:
a) Inkubieren der isolierten biologischen Probe mit mindestens einem Peptid, das aus einer Sequenz ausgewählt aus SEQ ID NR. 1 bis SEQ ID NR. 252 besteht, unter geeigneten Bedingungen zum Erzeugen einer T-Zell-Antwort;
b) Messen der T-Zell-Antwort;
c) Vergleichen der gemessenen T-Zell-Antwort mit der gemessenen T-Zell-Antwort einer geeigneten Kontrollprobe,
wobei die Virusinfektion durch ein Virus ausgewählt aus der Gruppe von HCV, HBV verursacht wird, wodurch Patienten, die an einer chronischen Infektion leiden, und diejenigen, bei denen ein Rückgang der Infektion festzustellen ist, unterschieden werden.

2. Verfahren nach Anspruch 1, wobei die biologische Probe eine Probe von PBMCs ist.

3. Verfahren nach einem der vorhergehenden Ansprüche, wobei das mindestens eine Peptid von Schritt a) aus der Sequenz ausgewählt aus LLQDSVDFSL (SEQ ID NR. 204), QLFNHTMFI (SEQ ID NR. 64) oder VLMIKALEL (SEQ ID NR. 78) besteht.

4. Verfahren nach einem der vorhergehenden Ansprüche, wobei Schritt b) das Inkubieren der biologischen Probe mit einem Pool von Peptiden ausgewählt aus den folgenden Pools umfasst:
- Pool 1: SEQ ID NR. 1, 2, 4, 5, 8 und 9;
- Pool 2: SEQ ID NR. 14, 16, 18, 19 und 20;
- Pool 3: SEQ ID NR. 27, 28, 29, 30, 34, 35, 36, 37 und 40;
- Pool 4: SEQ ID NR. 58, 59, 60, 62, 63, 64, 65, 66 und 67;
- Pool 5: SEQ ID NR. 68, 69, 73, 74, 75, 76, 77, 78, 81, 83, 84 und 88;
- Pool 6: SEQ ID NR. 89, 90, 91, 92, 99, 100, 104, 105, 106, 111, 117, 123, 126 und 127;
- Pool 7: SEQ ID NR. 198, 199, 200, 201, 202, 204, 206, 210, 211 und 220;
- Pool 8: SEQ ID NR. 232, 233, 234, 235, 236, 237, 238, 239, 240, 241, 242, 243 und 244; oder
- Pool 9: SEQ ID NR. 245, 246, 247, 248 und 249.

5. Verfahren nach einem der vorhergehenden Ansprüche, wobei die T-Zell-Antwort eine CD8⁺ T-Zell-Antwort ist.

6. Verfahren nach einem der vorhergehenden Ansprüche, wobei die T-Zell-Antwort mit Hilfe eines ELISpot-Assays gemessen wird.

7. Verfahren nach Anspruch 6, wobei der ELISpot-Assay ein Interferon-Gamma-, IL-17- oder IL-4-ELISpot-Assay ist.

8. Verfahren zur Überwachung des Voranschreitens einer Virusinfektion und/oder zur Überwachung der Wirksamkeit einer Therapie für eine Virusinfektion bei einem Subjekt, das an der Virusinfektion leidet, welches den Schritt des Nachweisens und/oder Quantifizierens der Menge an Autoantikörpern, die gegen mindestens ein Peptid wie in einem der Ansprüche 1, 3 oder 4 definiert gerichtet sind, umfasst, wobei die Virusinfektion durch ein Virus ausgewählt aus der Gruppe von HCV, HBV verursacht wird, wodurch Patienten, die an einer chronischen Infektion leiden, und diejenigen, bei denen ein Rückgang der Infektion festzustellen ist, unterschieden werden.

## Revendications

1. Procédé de surveillance de l'évolution d'une infection virale et/ou de surveillance de l'efficacité d'une thérapie contre une infection virale dans un échantillon biologique comprenant des lymphocytes T prélevés sur un sujet affecté par ladite infection virale, comprenant les étapes de :
a) incubation de l'échantillon biologique isolé avec au moins un peptide constitué d'une séquence sélectionnée parmi SEQ ID NO : 1 à SEQ ID NO : 252 dans des conditions appropriées pour générer une réponse des lymphocytes T ;
b) mesure de la réponse des lymphocytes T ;
c) comparaison de la réponse des lymphocytes T mesurée à la réponse des lymphocytes T mesurée d'un échantillon témoin approprié,
dans lequel ladite infection virale est provoquée par un virus sélectionné dans le groupe du HCV, du HBV, permettant ainsi de différencier les patients subissant une infection chronique de ceux chez qui l'infection est en phase de résolution.

2. Procédé selon la revendication 1, dans lequel l'échantillon biologique est un échantillon de PBMC.

3. Procédé selon l'une quelconque des revendications précédentes, dans lequel l'au moins un peptide de l'étape a) est constitué de la séquence sélectionnée parmi LLQDSVDFSL (SEQ ID NO : 204), QLFNHTMFI (SEQ ID NO : 64) ou VLMIKALEL (SEQ ID NO : 78).

4. Procédé selon l'une quelconque des revendications précédentes, dans lequel l'étape b) comprend l'incubation de l'échantillon biologique avec un ensemble de peptides sélectionné parmi les ensembles suivants :
- ensemble 1 : SEQ ID NO : 1, 2, 4, 5, 8 et 9 ;
- ensemble 2 : SEQ ID NO : 14, 16, 18, 19 et 20 ;
- ensemble 3 : SEQ ID NO : 27, 28, 29, 30, 34, 35, 36, 37 et 40 ;
- ensemble 4 : SEQ ID NO : 58, 59, 60, 62, 63, 64, 65, 66 et 67 ;
- ensemble 5 : SEQ ID NO : 68, 69, 73, 74, 75, 76, 77, 78, 81, 83, 84 et 88 ;
- ensemble 6 : SEQ ID NO : 89, 90, 91, 92, 99, 100, 104, 105, 106, 111, 117, 123, 126 et 127 ;
- ensemble 7 : SEQ ID NO : 198, 199, 200, 201, 202, 204, 206, 210, 211 et 220 ;
- ensemble 8 : SEQ ID NO : 232, 233, 234, 235, 236, 237, 238, 239, 240, 241, 242, 243 et 244 ou
- ensemble 9 : SEQ ID NO : 245, 246, 247, 248 et 249.

5. Procédé selon l'une quelconque des revendications précédentes, dans lequel la réponse des lymphocytes T est une réponse des lymphocytes T CD8+.

6. Procédé selon l'une quelconque des revendications précédentes, dans lequel la réponse des lymphocytes T est mesurée au moyen d'un test Elispot.

7. Procédé selon la revendication 6, dans lequel le test Elispot est un test Elispot interféron-gamma, IL-17 ou IL-4.

8. Procédé de surveillance de l'évolution d'une infection virale et/ou de surveillance de l'efficacité d'une thérapie contre une infection virale chez un sujet affecté par ladite infection virale, comprenant l'étape de détection et/ou de quantification de la quantité d'auto-anticorps dirigés contre au moins un peptide comme défini dans l'une quelconque des revendications 1, 3 et 4, dans lequel ladite infection virale est provoquée par un virus sélectionné dans le groupe du HCV, du HBV, permettant ainsi de différencier les patients subissant une infection chronique de ceux chez qui l'infection est en phase de résolution.
